# EUROPEAN PATENT APPLICATION

(11) **EP 4 834 890 A1**
(43) Date of publication of application: **04.11.2026**
(21) Application number: 24911510.6
(22) Date of filing: 27.12.2024
(51) Int. Cl.: C07D 295/13, C07D 295/15, A61K 9/51, A61K 47/24, A61K 47/28

(54) **ASYMMETRIC CATIONIC LIPID CONTAINING MULTIPLE TERTIARY AMINES**

(30) Priority: 29.12.2023 CN 202311855275; 30.04.2024 CN 202410539893
(71) Applicant: Xiamen Sinopeg Biotech Co., Ltd., Xiamen, Fujian 361100 (CN)
(72) Inventor: LIN, Minggui, Xiamen, Fujian 361100 (CN); ZHU, Qi, Xiamen, Fujian 361100 (CN); WANG, Ailan, Xiamen, Fujian 361100 (CN); WANG, Linlin, Xiamen, Fujian 361100 (CN); WEI, Guohua, Xiamen, Fujian 361100 (CN); WENG, Wengui, Xiamen, Fujian 361100 (CN); LIU, Chao, Xiamen, Fujian 361100 (CN); LIN, Sheng, Xiamen, Fujian 361100 (CN)
(74) Representative: Weickmann & Weickmann PartmbB
(86) International application number: PCT/CN2024/143282
(87) International publication number: WO 2025/140594

(57) **Abstract**

The present application relates to the field of drug delivery, and provides a tertiary amine-containing, asymmetric cationic lipid having a structure as shown in Formula (1), wherein the definitions of the symbols are consistent with those described herein. The cationic lipid of the present application incorporates biodegradable groups at appropriate positions, wherein the presence of degradable groups enables the LNP pharmaceutical compositions prepared from the cationic lipids to degrade timely within endosomes and exhibit low cytotoxicity, which addresses the issue in the prior art where LNP pharmaceutical compositions prepared from non-degradable or poorly degradable cationic lipids accumulate in endosomes, acidify the endosomal environment, and hinder the endosomal escape of drugs (e.g., mRNA), thereby preventing the delivered drugs from exerting their full effects. The piperazine-containing cationic lipid is prepared using multifunctional piperazine-based raw materials, resulting in a simple preparation process, lower cost, and greener production. The LNP-mRNA compositions prepared from it exhibit low toxicity, high biocompatibility, and high cellular transfection efficiency.

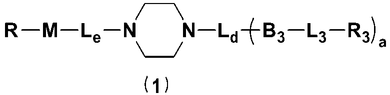

## Description

### TECHNICAL FIELD

The present application relates to the field of drug delivery, specifically relates to a multilevel tertiary amine-containing, asymmetric cationic lipid as a pharmaceutical carrier, particularly a piperazine ring-branched asymmetric cationic lipid, as well as a lipid composition comprising the cationic lipid, a lipid pharmaceutical composition, and formulations and applications thereof.

### BACKGROUND

Nucleic acid drugs are collectively referred to as oligoribonucleotides (RNA) or oligodeoxyribonucleotides (DNA) with different functions, which play a role at the gene level, including but are not limited to: siRNA, miRNA, antisense nucleic acids, aptamers, DNA, messenger RNA (mRNA), etc. Wherein, mRNA is a type of single-stranded ribonucleic acid that is transcribed from one strand of DNA as a template, carries genetic information, and can guide protein synthesis. In recent years, due to the advantages of mRNA as a drug or vaccine, such as translation in the cytoplasm, simple production process, rapid synthesis, low cost, and suitability for large-scale production, it has been applied to the prevention and treatment research of different types of diseases, especially in the vaccines field. However, mRNA has some drawbacks, such as being easily degradable, unstable, and difficult to enter cells. These defects can be overcome by optimizing its own chemical structure and selecting appropriate delivery systems. At present, mRNA delivery systems are one of the key technologies that limit the development of mRNA as a drug.

mRNA delivery systems mainly include viral vectors and non-viral vectors. Viral vectors carry the risk of integrating into the host genome and may cause varying degrees of humoral or cellular immune responses, thus limiting their clinical use. Non-viral vectors mainly include liposomes, lipid nanoparticles (LNPs), inorganic nanoparticles, polymers, and proteins. Lipid nanoparticles are currently the most widely studied and highly focused delivery systems.

Lipid nanoparticles are usually composed of cationic lipids, neutral lipids, sterol lipids, and PEGylated lipids under appropriate conditions. Wherein, cationic lipids are connected to nucleic acids through electrostatic interactions. The cationic lipid component has a significant impact on the delivery performance of lipid nanoparticles. Some cationic lipids that are currently widely used include MC3, ALC-0315, SM-102, etc. However, mRNA lipid nanoparticle delivery technology still faces many challenges. Different research systems may have different optimal cationic lipids and/or lipid formulation ratios. Therefore, there is still a need in this field for improved cationic lipids suitable for routine therapeutic use.

### SUMMARY

The present application provides a piperazine ring-branched asymmetric cationic lipid and preparation methods thereof, a lipid composition containing the cationic lipid, a lipid pharmaceutical composition containing the lipid composition and formulation thereof, and a liposome or LNP containing the lipid composition. Especially, the LNP-nucleic acid pharmaceutical composition containing the lipid composition and its formulations have the advantages of high delivery efficiency, safety, low toxicity, and high biocompatibility, which can improve the therapeutic and/or preventive effects of drugs.

The above-described purposes of this application can be realized via the embodiments below:
In one embodiment of the present application, provided herein is a cationic lipid:
A cationic lipid, wherein the structure is represented by the general formula (1): 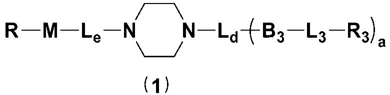
or a salt, tautomer, stereoisomer, deuterated derivative, or solvate thereof,
wherein, L_{d} is -(CH₂)ₜₐN<, and the left end is connected to the piperazine ring; ta is an integer from 1 to 6, a is 2;
Lₑ is a C₁₋₁₂ alkylene group or -CH₂CH(OH)-(CH₂)_{tb}-; wherein, tb is an integer from 1 to 10, and the left of -CH₂CH(OH)-(CH₂)_{tb}- is connected to the piperazine ring;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 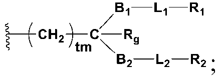 wherein, tm is an integer from 0 to 6; B₁, B₂, and B₃ are each independently a linking bond or a C₁₋₂₀ alkylene group at each occurrence; L₁, L₂, and L₃ are each independently a linking bond or L_{c}; the L_{c} is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O- at each occurrence; R₁, R₂, and R₃ are each independently a linear or branched C₅₋₃₀ hydrocarbon group at each occurrence; when B₁, B₂, L₁, and L₂ are all linking bonds, Lₑ is -CH₂CH(OH)-(CH₂)_{tb}-; s is 1, 2 or 3; R_{g} is H or C₁₋₆ alkyl.

The present application also provides a lipid composition, embodied as follows:
A lipid composition containing a cationic lipid with the structure represented by the formula (1).

The present application also provides a lipid pharmaceutical composition, embodied as follows:
A lipid pharmaceutical composition comprising a lipid composition and a drug; wherein, the lipid composition comprises a cationic lipid with the structure represented by the formula (1); the drug is selected from the group consisting of a nucleic acid drug, a gene vaccine, an anti-tumor drug, a small molecule drug, a peptide drug, and a protein drug.

The present application also provides a formulation of lipid pharmaceutical composition, embodied as follows:
A formulation of lipid pharmaceutical composition comprising the aforementioned lipid pharmaceutical composition and a pharmaceutically acceptable diluent or excipient.

The present application also provides a liposome or LNP, embodied as follows:
A liposome or LNP comprising a lipid composition, wherein the lipid composition contains a cationic lipid with the structure represented by formula (1).

**Compared with the prior art, the present application brings the following beneficial** effects:
The present application provides a novel cationic lipid compound, which enriches the variety of cationic lipid compounds and offers more options for the delivery of nucleic acid drugs, gene vaccines, small molecule drugs, peptides, and protein drugs.

The cationic lipid of the present application introduces a piperazine ring structure into the hydrophilic head group and contains multiple ionizable tertiary amines. These features can enhance its interaction with nucleic acids, thereby enabling it to carry a larger amount of nucleic acids.

The cationic lipid of the present application, containing a piperazine ring, introduces biodegradable groups into the hydrophobic alkyl tail. The presence of these biodegradable groups allows the LNP-drug compositions prepared from it to degrade in a timely manner within endosomes. This addresses the issue of non-biodegradable lipids causing endosomal accumulation and acidification, which hinders the endosomal escape of drugs (e.g., mRNA) and prevents the delivered drugs from functioning effectively. Additionally, the biodegradable groups degrade the cationic lipid into non-toxic metabolites under the action of intracellular enzymes, offering the advantages of high safety and low cytotoxicity.

The cationic lipid containing a piperazine ring has three ionizable tertiary amines that are positively charged in acidic environments and almost neutral in neutral environments. In acidic buffer systems, it binds to negatively charged nucleic acid drugs through electrostatic interactions. Once in the neutral environment of the body, the lipid-nucleic acid drug composition (e.g., lipid nanoparticles) becomes electrically neutral. This effectively prevents the adsorption of plasma proteins, thereby achieving higher delivery efficiency and safety. The piperazine-containing cationic lipid is prepared using multifunctional piperazine-based raw materials, resulting in a simple preparation process, lower cost, and greener production. The LNP-mRNA compositions prepared from it exhibit low toxicity, high biocompatibility, and high cellular transfection efficiency.

### BRIEF DESCRIPTION OF THE DRAWINGS

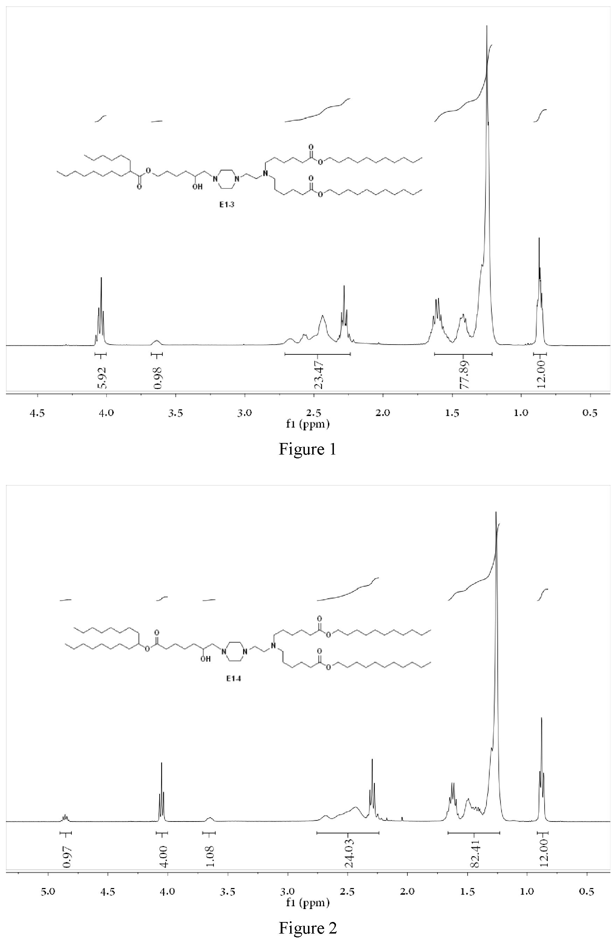

Figure 1 is the ¹H NMR spectrum of cationic lipid E1-3 prepared in Example 1.3.
Figure 2 is the ¹H NMR spectrum of cationic lipid E1-4 prepared in Example 1.4.

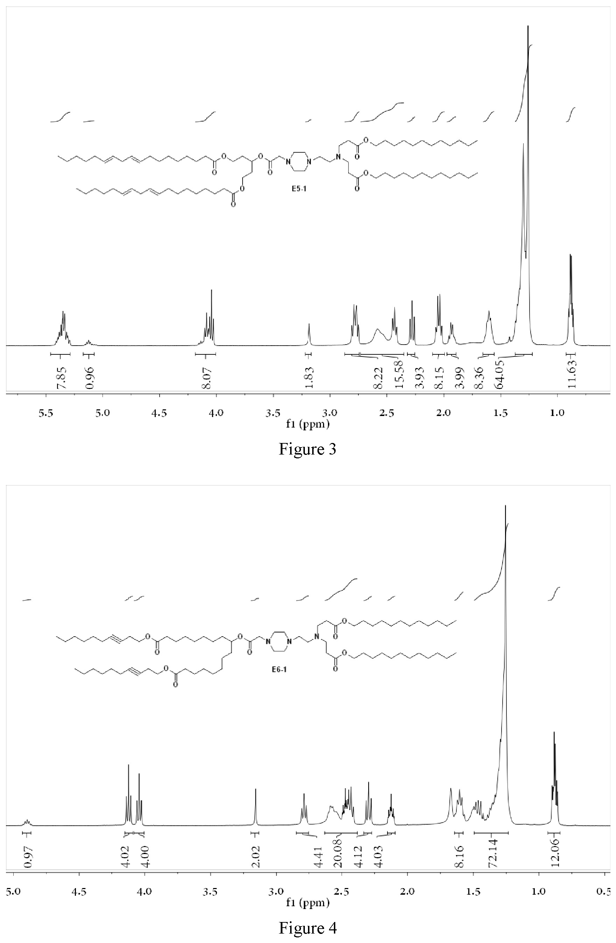

Figure 3 is the ¹H NMR spectrum of cationic lipid E5-1 prepared in Example 5.
Figure 4 is the ¹H NMR spectrum of cationic lipid E6-1 prepared in Example 6.

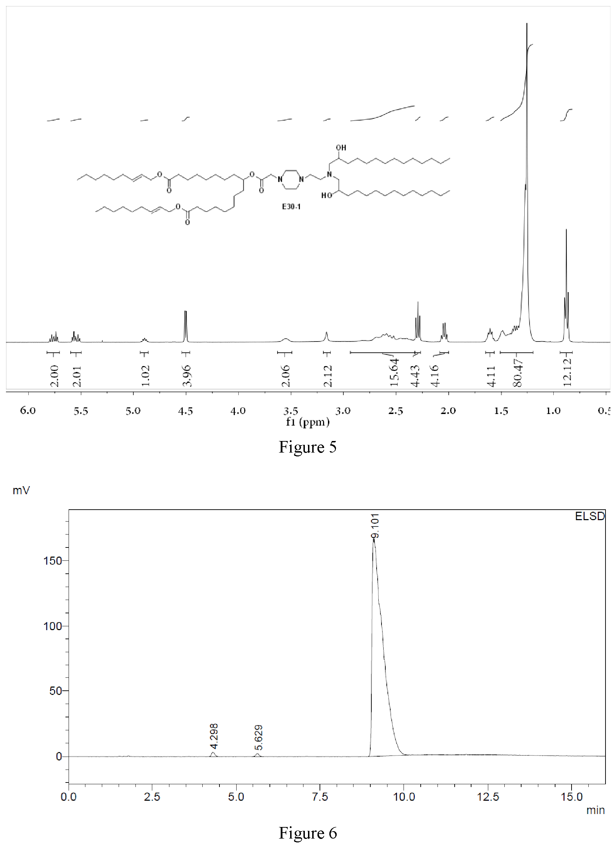

Figure 5 is the ¹H NMR spectrum of cationic lipid E30-1 prepared in Example 30.
Figure 6 is the high-performance liquid chromatography (HPLC) test results of cationic lipid E1-3 prepared in Example 1.3.
Figure 7 is the mass spectrometry (MS) of cationic lipid E1-3 prepared in Example 1.3.
Figure 8 is the cytotoxicity test results of LNP-mRNA pharmaceutical composition L-1-3 prepared in Example 34.
Figure 9 is the imaging results after mouse injection of LNP-mRNA pharmaceutical composition L-1-3 prepared in Example 34.

### DETAILED DESCRIPTION

### Description of Terms

In the present application, the terms have the following meanings, unless otherwise specified.

In the present application, unless otherwise specified, when the structure involved has isomers, it can refer to any one of the isomers. For example, when cis- and trans- isomers are present, it can refer to either a cis isomer or a trans isomer; when E/Z isomers exist, the structure can be either an (E)-structure or a (Z)-structure; and when optical activity is present, it can be either a levorotatory form or a dextrorotatory form.

In the present application, a numerical interval may be indicated by a hyphen (e.g., 0-6), a tilde (e.g., 0~6), or "to" (e.g., 0 to 6, 1 to 6). In the present application, an integer interval represents a group consisting of all integers within the range, including both endpoints, unless otherwise specified. For example, the integer interval 0-12 represents the group consisting of 1, 2, 3, 4, 5, and 6. The numerical interval in the present application includes, but is not limited to, ranges represented by integers, non-integers, percentages, and fractions, and includes both endpoints, unless otherwise specified.

In the present application, "about" and "approximately" generally indicate a range of ±10% for numerical values, and in some cases may be expanded to ±15%, but not more than ±20%, based on the preset values. For example, when the molar percentage of steroid lipid in the total lipids is about 40%, it is generally assumed to include cases where the molar percentage of steroid lipids ranges from 30% to 50%.

In the present application, "pharmaceutically acceptable salt" refers to a salt of this application compounds that is recognized for use in animals, and more specifically in humans. This includes salts formed from the compounds represented by formula (1) with inorganic or organic acids. Such as disclosed in S. M. Berge et al., "Pharmaceutical Salts," J. Pharm. Sci. 1977, 66, 1-19. Wherein, inorganic acids include hydrochloric acid, hydrobromic acid, hydriodic acid, sulfuric acid, phosphoric acid, or nitric acid, and the like; organic acids include formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)-benzoic acid, camphoric acid, cinnamic acid, cyclopentanepropionic acid, d-glucoheptonic acid, 3-hydroxy-2-naphthoic acid, nicotinic acid, pamoic acid, pectinic acid, 3-phenylpropionic acid, picric acid, pivalic acid, 2-hydroxyethanesulfonic acid, itaconic acid, aminosulfonic acid, trifluoromethanesulfonic acid, dodecyl sulfate, ethanesulfonic acid, benzenesulfonic acid, p-toluenesulfonic acid, methanesulfonic acid, 2-naphthalenesulfonic acid, naphthalene-1,5-disulfonic acid, camphorsulfonic acid, citric acid, tartaric acid, stearic acid, lactic acid, oxalic acid, malonic acid, succinic acid, malic acid, adipic acid, alginate, maleic acid, fumaric acid, d-glucuronic acid, mandelic acid, ascorbic acid, gluconic acid, glycerophosphoric acid, aspartic acid, sulfosalicylic acid, and the like. For example, HCl (or hydrochloric acid), HBr (or hydrobromic acid solution), methanesulfonic acid, sulfuric acid, tartaric acid or fumaric acid may be used to form a pharmaceutically acceptable salt with the compounds represented by the formula (1).

In the present application, the "solvate" refers to a composition formed by the compound of formula (1) or its pharmaceutically acceptable salt with a solvent (e.g., ethanol or water). It should be understood that any solvate of the compound of formula (1) used in the treatment of diseases or conditions may provide different properties (including pharmacokinetic properties), but once absorbed into the subject, the compound of formula (1) is obtained, thereby covering the use of the compound of formula (1) and any solvate thereof. It should be further understood that the compound of formula (1) or its pharmaceutically acceptable salt may be isolated in the form of a solvate, and therefore any of the said solvates are included within the scope of this invention. For example, the compound of formula (1) or its pharmaceutically acceptable salt may exist in both non-solvated form and solvated form with a pharmaceutically acceptable solvent (such as water, ethanol, etc.).

In the present application, unless otherwise specified, the term "include", "contain", "comprise", and similar expressions shall be interpreted as "including but not limited to" in the specification and claims, in an open and inclusive manner.

In the present application, when two or more objects are "each independently preferably" selected from multiple levels of preferable options, the objects are not necessarily selected from preferable options of the same level. It is allowed that one is selected from a wider range of options while another one is selected from a narrower range of options. It is also allowed that one is selected from the maximum range of options while another is selected from any allowable options. It is also allowed that the objects are selected from preferable options of the same level.

In the present application and unless otherwise specified, a divalent linking group, e.g., a hydrocarbylene group, an alkylene group, an arylene group, an amide bond, and the like, can use either one of the two linking ends to connect to another group. For example, when an amide bond serves as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present application, when distinguishing the terminus from the substituents of a linking group becomes questionable, " 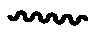 " is used to indicate the connection location between the linking group and the other groups. For example, in structural formulas 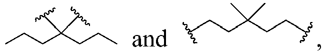 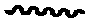 is used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" indicates the number of carbon atoms of a group. For example, C₁₋₁₂ indicates "having 1 to 12 carbon atoms"; C₁₋₃₀ indicates "having 1 to 30 carbon atoms". The "substituted C₁₋₁₂ alkyl group" refers to a C₁₋₁₂ alkyl group with one or more hydrogen atoms being replaced. The "C₁₋₁₂ substituted alkyl group" refers to an alkyl group with one or more hydrogen atoms being replaced, with 1 to 12 carbon atoms remaining. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, the term "group" may be referred to as "bond" without changing the meaning. For example, an ether group (-O-) can also be termed an ether bond, and an ester group (-OC(=O)- or -C(=O)O-) can also be termed an ester bond.

In the present application, the "carbon chain length" between two groups refers to the minimum number of carbon atoms involved in the pathway from one group to another, excluding the groups themselves. For example, in the structures -OC(=O)CH₂CH₂OC(=O)-and -OC(=O)CH(CH₃)CH₂OC(=O)-, the carbon chain lengths between the two ester bonds are both 2 (also referred to as C2); the carbon chain length between the two ester bonds of -OC(=O)CH₂CH₂OCH₂CH₂OC(=O)- is C4 (also referred to as C4).

In the present application, heteroatoms are not particularly limited, including but not limited to O, S, N, P, Si, F, Cl, Br, I, B, etc.

In the present application, the heteroatom used for substitution is referred to as a "substituent atom", and the group used for substitution is referred to as a "substituent group".

In the present application, the term "substituted" indicates that at least one hydrogen atom of the described group (e.g., aliphatic hydrocarbon, hydrocarbon, alkyl, or alkylene group) is replaced by a bond connected to a non-hydrogen atom. The non-hydrogen atom include but are not limited to halogen atoms such as F, Cl, Br, and I, oxo group (=O), hydroxyl group (-OH), hydrocarbonoxy groups (-OR_{d}; wherein, R_{d} is a C₁₋₁₂ alkyl group), carboxyl group (-COOH), amine groups (-NR_{c}R_{c}; wherein, both R_{c} are each independently H or a C₁₋₁₂ alkyl group), C₁₋₁₂ alkyl groups, and cycloalkyl groups. In some embodiments, the substituent is a C₁₋₁₂ alkyl group. In other embodiments, the substituent is a cycloalkyl group. In other embodiments, the substituent is a halogen atom, such as fluorine atom. In other embodiments, the substituent is an oxo group. In other embodiments, the substituent is a hydroxyl group. In other embodiments, the substituent is an alkoxy group. In other embodiments, the substituent is a carboxyl group. In other embodiments, the substituent is an amine group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbon groups" means that the hydrocarbon groups may or may not be substituted, and the description includes both substituted and unsubstituted hydrocarbon groups.

In the present application, a compound or a group can be substituted and heteroatom-incorporated simultaneously, e.g., by replacing a hydrogen atom with a nitrophenyl group, or by replacing -CH₂-CH₂-CH₂- with -CH₂-S-CH(CH₃)-. In the present application, a "linking bond" is only for connecting and does not contain any atoms. When a group is defined as including a linking bond, the group is optionally absent.

In the present application, "each independently at each occurrence" not only means that different groups can be each independently selected from the definitions, but also means that the same group at different positions can be independently selected from the definitions. For example, in -Z-L⁶-Z-, "Z is independently selected form the group consisting of -C(=O)-, -OC(=O)-, -C(=O)O-, -OC(=O)O-, -O-, -S-, -C(=O)S-, -SC(=O)-, -NR^{c}C(=O)-, -C(=O)NR^{c}-, -NR^{c}C(=O)NR^{c}-, -OC(=O)NR^{c}-, -NR^{c}C(=O)O-, -SC(=O)NR^{c}-, and-NR^{c}C(=O)S- at each occurrence; wherein, R_{c} is each independently a hydrogen atom or a C₁₋₁₂ alkyl group at each occurrence", in the group "-Z-L⁶-Z-", both Z can be the same or different, which are each independently a hydrogen atom or a C₁₋₁₂ alkyl group.

In the present application, a "group" contains at least one atom, referring to the radical formed by a compound losing one or more atoms. Relative to a compound, the remaining group formed by the removal of other groups is also referred to as the "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. A linking group may also contain only one atom, such as ether group or thioether group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons that do not contain phenyl rings or hydrocarbyl-substituted phenyl rings are defined as aliphatic hydrocarbons. Hydrocarbons that contain at least one phenyl ring or hydrocarbyl-substituted phenyl ring are defined as aromatic hydrocarbons. An aromatic hydrocarbon may contain aliphatic hydrocarbon structures, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also referred to as alkanes. There is no specific limit to the degree of unsaturation of unsaturated aliphatic hydrocarbons. For example, unsaturated aliphatic hydrocarbons may include, but are not limited to, alkenes (containing carbon-carbon double bonds), alkynes (containing carbon-carbon triple bonds), and dienes (containing conjugated carbon-carbon double bonds), etc. When the aliphatic moieties of aromatic hydrocarbons are saturated, the aromatic hydrocarbons are also termed aralkanes, such as toluene.

In the present application, the structures of hydrocarbons are not particularly limited and may include, for example, linear structures without pendant groups, branched structures with pendant groups, ring-containing structures, dendritic structures, comb-like structures, and hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons. Branched hydrocarbons are also referred to as branched aliphatic hydrocarbons.

In the present application, compounds in which any carbon atom in a hydrocarbon is substituted by a heteroatom are collectively referred to as heterohydrocarbons.

In the present application, "hydrocarbon group" refers to a residue formed by removing at least one hydrogen atom from a hydrocarbon molecule. Depending on the number of hydrogen atoms removed, hydrocarbon groups can be classified into monovalent hydrocarbon groups (with one hydrogen atom removed), divalent hydrocarbon groups (with two hydrogen atoms removed, also referred to as hydrocarbylene groups), trivalent hydrocarbon groups (with three hydrogen atoms removed), and the like. Accordingly, when n hydrogen atoms are lost, the valence of the formed hydrocarbon group is n. Unless otherwise specified, hydrocarbon groups in the present application specifically refer to monovalent hydrocarbon groups. Unless otherwise expressly stated in this specification, a hydrocarbon group is optionally substituted.

In the present application, the source of hydrocarbon group is not particularly limited and may include, for example, from aliphatic hydrocarbons or aromatic hydrocarbons, from saturated hydrocarbons or unsaturated hydrocarbons, from linear hydrocarbons, branched hydrocarbons or cyclic hydrocarbons, and from hydrocarbons or heterohydrocarbons, etc. According to the degree of saturation, hydrocarbon groups can be derived from alkanes, alkenes, alkynes, dienes, etc. With respect to cyclic hydrocarbons, hydrocarbon groups can be derived from alicyclic hydrocarbons or aromatic hydrocarbons, or from monocyclic hydrocarbons or polycyclic hydrocarbons. With respect to heterocyclic hydrocarbons, hydrocarbon groups can be derived from aliphatic heterocyclic hydrocarbons or aromatic heterocyclic hydrocarbons.

In the present application, "aliphatic hydrocarbon group" refers to the residue formed by removing at least one hydrogen atom from an aliphatic hydrocarbon molecule. Unless otherwise specified, aliphatic hydrocarbon groups refer to monovalent hydrocarbon groups in the present application. Aliphatic hydrocarbon groups include both saturated and unsaturated aliphatic hydrocarbon groups. Unless otherwise expressly stated in this specification, aliphatic hydrocarbon groups are optionally substituted.

In the present application, "alkyl group" refers to a hydrocarbon group obtained from an alkane by removing a hydrogen atom at any position. Unless otherwise specified, the alkyl group can be linear or branched, and substituted or unsubstituted. Specifically, a propyl group refers to either a 1-propyl group or an isopropyl group, and a propylene group refers to a 1,3-propylene group, a 1,2-propylene group, or an isopropylidene group. Unless otherwise specified in this specification, alkyl groups are optionally substituted.

In the present application, "unsaturated hydrocarbon group" refers to the hydrocarbon group obtained from an unsaturated hydrocarbon by removing hydrogen atoms. The unsaturated hydrocarbon obtained by removing hydrogen atoms from unsaturated carbon atoms can be classified into alkenyl groups, alkynyl groups, dienyl groups, and the like.

In the present application, "alkenyl group" refers to a linear or branched-chain hydrocarbon group containing at least one carbon-carbon double bond, formed by removing hydrogen atoms from any position of an alkene. For example, "C₂₋₁₅ alkenyl group" refers to a linear or branched alkenyl group containing 2 to 15 carbon atoms and at least one carbon-carbon double bond, i.e., one, two, three, four, or more carbon-carbon double bonds. Unless otherwise specified, the alkenyl groups include substituted and unsubstituted alkenyl groups in the present application. Unless otherwise expressly stated in this specification, alkenyl groups are optionally substituted.

In the present application, "alkynyl group" refers to a linear or branched-chain hydrocarbon group containing at least one carbon-carbon triple bond, formed by losing hydrogen atoms from any position of an alkyne. For example, "C₂₋₁₅ alkynyl group" refers to a linear or branched alkynyl group containing 2 to 15 carbon atoms and at least one carbon-carbon triple bond. An alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to free radicals, consisting only of carbon and hydrogen, which is saturated or unsaturated. Examples include, a hydrocarbylene group with one to twenty-four carbon atoms (a C₁₋₂₄ hydrocarbylene group), a hydrocarbylene group with one to twelve carbon atoms (a C₁₋₁₂ hydrocarbylene group), and specifically, a methylene group, an ethylene group, a propylene group, an *n*-butylene group, a vinylidene group, a propenylene group, an *n*-butenylene group, a propynylene group, an *n*-butynylene group, etc. Unless otherwise explicitly stated in this specification, hydrocarbylene groups are optionally substituted.

In the present application, an "alkylene group" is also a divalent alkyl group, including open-chain alkylene groups and divalent cycloalkyl groups. The open-chain alkylene group refers to a divalent alkyl group without a cyclic structure, and the divalent cycloalkyl group refers to a divalent alkyl group containing a cyclic structure. Unless otherwise expressly stated in this specification, alkylene groups are optionally substituted.

In the present application, "molecular weight" represents the mass of a compound, and "average molecular weight" represents the mass of a compound component of a general formula in macroscopic matter. Unless otherwise specified, the molecular weight of polymers refers to the "average molecular weight", which generally refers to the "number average molecular weight" (Mₙ). The number average molecular weight can be used to describe the molecular weight of either polydisperse blocks or substances, or monodisperse blocks or substances. Unless otherwise specified, the measuring unit of "molecular weight" and "average molecular weight" is Dalton (Da). Polymers can be characterized by the "degree of polymerization", which indicates the number of repeating units (e.g., oxyethylene units of PEG, EO-units) in a molecule. Accordingly, "average degree of polymerization", "number average degree of polymerization" or "number of EO units" represents the average value or the number average value of the number of repeating units, and unless otherwise specified, each refers to the number average degree of polymerization.

In the present application, with respect to percentages, "about" generally refers to a range of ±0.5%.

In the present application, the terms "stable" and "degradable" are a pair of opposing concepts. Detailed examples of stable groups and degradable groups are given in paragraphs [0134] to [0145] of CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a t-butyldimethylsilyl group, an allyl group, an acetal group, and a ketal group. Removal of the acetyl group is generally carried out under basic conditions, most commonly by the ammonolysis with NH₃/MeOH or by the methanol anion-catalyzed methanolysis. The benzyl group can be easily removed via palladium-catalyzed hydrogenolysis in a neutral solution at room temperature, or via a reduction reaction by metallic sodium in ethanol or liquid ammonia. The triphenylmethyl group is generally removed via catalytic hydrogenolysis. The trimethylsilyl group is generally removed using reagents containing fluoride ions (e.g., tetrabutylammonium fluoride/anhydrous THF, etc.). The t-butyldimethylsilyl ether is relatively stable and can withstand ester hydrolysis conditions with alcoholic potassium hydroxide and mild reduction conditions (e.g., Zn/CH₃OH and the like), which can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysis or the deprotection reaction of a carboxyl protecting group itself. A carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me) and an ethyl group (Et).

In the present application, "protected carboxyl group" refers to the group protected by an appropriate carboxyl protecting group, preferably selected from the group consisting of a methoxycarbonyl group, an ethoxycarbonyl group, t-butoxycarbonyl group, and a benzyloxycarbonyl group. The carboxyl protecting groups can be removed through hydrolysis catalyzed by acids or bases, or through pyrolysis reactions occasionally. For example, t-butyl groups can be removed under mild acidic conditions, and benzyl groups can be removed by hydrogenolysis. The reagent used for the removal of carboxyl protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. A protected carboxyl group can undergo deprotection to produce the corresponding free acid, wherein the deprotection is conducted in the presence of a base, and the base forms a pharmaceutically acceptable salt with the free acid produced via the deprotection.

In the present application, "amino protecting group" includes all the groups which are used as amino protecting groups generally, such as an aryl C₁₋₆ alkyl group, a C₁₋₆ alkoxy group, a C₁₋₆ alkyl group, a C₁₋₆ alkoxycarbonyl group, an aryloxycarbonyl group, a C₁₋₆ alkylsulfonyl group, an arylsulfonyl group, a methylsilyl group, etc. An amino protecting group is preferably selected from the group consisting of a t-butoxycarbonyl group (Boc), a p-methoxybenzyloxycarbonyl group (Moz), and a 9-fluorenylmethyloxycarbonyl (Fmoc). The reagent used for the removal of amino protecting groups is selected from the group consisting of TFA, H₂O, LiOH, MeOH, EtOH, and combinations thereof, preferably selected from the group consisting of the combination of TFA and H₂O, the combination of LiOH and MeOH, and the combination of LiOH and EtOH. The Boc protecting group can be removed using TFA or HCl/EA, preferably TFA. The Fmoc protecting group can be removed using the N,N-dimethylformamide (DMF) solution containing 20% piperidine.

In the present application, "cationic lipid" refers to a lipid having a positive charge or being ionizable. Wherein, "cation" refers to the corresponding structure bearing a positive charge, either permanently, or non-permanently but in response to certain conditions (such as pH). Therefore, the cations include permanent cations and cationizable substances. Permanent cations refer to the corresponding compounds, groups, or atoms that bear positive charges under conditions of any pH value or hydrogen ion activity of their environment. Typically, a positive charge is generated by the presence of quaternary nitrogen atom. When a compound carries multiple such positive charges, it can be termed a permanent cation. A cationizable substance refers to a compound, group, or atom that is positively charged at a lower pH and uncharged at a higher pH of its environment. Moreover, in non-aqueous environments where pH cannot be determined, a cationizable compound, group, or atom is positively charged at high hydrogen ion concentration and uncharged at low concentration or activity of hydrogen ions. It depends on the individual properties of the cationizable or polycationizable compound, in particular the pKa of the corresponding cationizable group or atom, at which pH or hydrogen ion concentration said compound is charged or uncharged. In the diluted aqueous environment, the Henderson-Hasselbalch equation can be used to estimate the fraction of positively charged cationizable compounds, groups, or atoms, which is well-known to those skilled in the art. For example, in some embodiments, if a compound or moiety is cationizable, then, preferably, it is positively charged at a pH of about 1 to 9, preferably 4 to 9, 5 to 8, or even 6 to 8, more preferably equal to or below 9, equal to or below 8, equal to or below 7, and most preferably at physiological pH (e.g., about 7.3 to 7.4), i.e., under physiological conditions, especially under physiological conditions of cells in vivo. In other embodiments, it is preferable that the cationizable compound or moiety is predominantly neutral at physiological pH (e.g., about 7.0 to 7.4), but becomes positively charged at lower pH values. In some embodiments, pKa of the cationizable compound or moiety is preferably about 5 to about 7.

In the present application, LNPs, cationic peptides, proteins, polysaccharides, lipids and polymers are uncharged, having a neutral charge or being respectively electrically neutral under physiological conditions, especially under the physiological conditions of cells in vivo. Cationic peptides or proteins preferably contain a larger amount of cationic amino acids, such as a greater amount of Arg, His, Lys, or Orn compared to other amino acid residues (especially more cationic amino acids than anionic amino acid residues such as Asp or Glu), or contain components formed primarily by cationic amino acid residues. The term "cationic" can also refer to "polycationic" components/compounds, and can also refer to cationic lipids that are capable of being positively charged. Examples include, cationic lipids contain one or more amine groups with positive charges, and preferably cationic lipids that are ionizable to exist in either a positively charged or neutral form depending on pH. The ionization of cationic lipids affects the surface charge of LNPs at different pH conditions. This charge state can affect plasma protein absorption, blood clearance, and tissue distribution, as well as the ability to form non-bilayer structures that are essential for the intracellular delivery of nucleic acids.

In the present application, "PEGylated lipid" refers to the molecules containing both lipid and PEG moieties.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, a variant form refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a reactive form that retains its reactivity (remains a reactive group) after undergoing at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc.; alternatively, it may refer to a non-reactive form of protected reactive group.

In the present application, "micro-modification" refers to a chemical modification process that can be completed through simple chemical reactions. The simple chemical reactions mainly include deprotection, salt complexation and decomplexation, ionization, protonation, deprotonation, and transformation of leaving groups. The "micro-modified form" corresponds to "micro-modification" and refers to the structural form that can form the target reactive group after undergoing simple chemical reactions such as deprotection, salt complexation and decomplexation, ionization, protonation, deprotonation, and transformation of leaving groups. For example, the transformation of a leaving group can involve the conversion from an ester form to an acyl chloride form.

In the present application, "N/P ratio" refers to molar ratio of nitrogen atoms in cationic lipids to phosphates in nucleic acids.

In the present application, "nucleic acid" refers to DNA, RNA, or their modified form.

In the present application, "RNA" refers to ribonucleic acid that may be naturally or non-naturally occurring. For example, RNA may include modified and/or non-naturally occurring components such as one or more nucleobases, nucleosides, nucleotides, or linkers. An RNA may include a cap structure, a chain-terminating nucleoside, a stem-loop, a polyA sequence, and/or a polyadenylation signal. An RNA can be a nucleotide sequence encoding a specific polypeptide, or can be a messenger RNA (mRNA). Translation of mRNA encoding a particular polypeptide, for example, in vivo translation of an mRNA inside a mammalian cell, may produce the encoded polypeptide. RNAs may be selected from the group consisting of small interfering RNA (siRNA), asymmetrical interfering RNA (aiRNA), microRNA (miRNA), dicer-substrate RNA (dsRNA), small hairpin RNA (shRNA), messenger RNA (mRNA), single guide RNA (sgRNA), self-amplifying RNA (saRNA), circular RNA (circRNA), Cas9 mRNA, and mixtures thereof.

In the present application, FLuc mRNA can express luciferase protein, which emits bioluminescence in the presence of luciferin substrate, so FLuc is commonly used in mammalian cell culture to measure gene expression and cell viability.

In the present application, methods for determining the expression level of target genes include but are not limited to dot blots, northern blots, in situ hybridization, ELISA, immunoprecipitation, enzymatic function, and phenotypic assays.

In the present application, "transfection" refers to the introduction of a species (e.g., an RNA) into cells. Transfection may occur, such as, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also understood as antigens.

In the present application, "delivery" refers to delivering an entity to the target, for example, delivering drugs and/or therapeutic agents and/or prophylactic agents to subjects, wherein the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including oils from petroleum, animals, vegetables, or synthesis, e.g., peanut oil, soybean oil, mineral oil, sesame oil, etc. When the pharmaceutical composition is administered intravenously, water is an exemplary carrier. Physiological brine, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and water for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, the pharmaceutical compositions can act systemically and/or locally. For this purpose, they can be administered via appropriate routes, such as by injection (e.g., intravenous, intra-arterial, subcutaneous, intraperitoneal, intramuscular, including infusion) or transdermal administration; or be administered by oral, buccal, nasal, transmucosal, topical, ophthalmic formulations, or inhalation. For these routes of administration, the pharmaceutical compositions of this invention can be provided in suitable dosage forms. These dosage forms include, but are not limited to, tablets, capsules, lozenges, troches, powders, sprays, creams, ointments, suppositories, gels, pastes, lotions, soft ointments, aqueous suspensions, injectable solutions, elixirs, and syrups.

In the present application, "vaccines" are preventive or therapeutic materials that provide at least one antigen or antigenic function. Antigen or antigenic function can stimulate the body's adaptive immune system to provide an adaptive immune response.

In the present application, "treatment" refers to the management and care of patients to combat diseases, obstacles, or symptoms, intended to delay the progression of diseases, obstacles, or conditions, reduce or alleviate symptoms and complications, and/or cure or eliminate diseases, disorders, or conditions. The patients to be treated are preferably mammals, particularly humans.

### Detailed Description of the Application

### 1. Cationic Lipids

An embodiment of the present application:
A cationic lipid, wherein the structure is represented by the general formula (1): 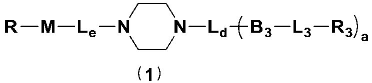
or a salt, tautomer, stereoisomer, deuterated derivative, or solvate thereof;
wherein, L_{d} is -(CH₂)ₜₐN<, and the left end is connected to the piperazine ring; ta is an integer from 1 to 6, a is 2;
Lₑ is a C₁₋₁₂ alkylene group or -CH₂CH(OH)-(CH₂)_{tb}-; wherein, tb is an integer from 1 to 10, and the left of -CH₂CH(OH)-(CH₂)_{tb}- is connected to the piperazine ring;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 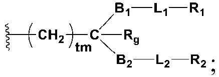 wherein, tm is an integer from 0 to 6; B₁, B₂, and B₃ are each independently a linking bond or a C₁₋₂₀ alkylene group at each occurrence; L₁, L₂, and L₃ are each independently a linking bond or L_{c}; the L_{c} is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O- at each occurrence; R₁, R₂, and R₃ are each independently a linear or branched C₅₋₃₀ hydrocarbon group at each occurrence; when B₁, B₂, L₁, and L₂ are all linking bonds, Lₑ is -CH₂CH(OH)-(CH₂)_{tb}-; s is 1, 2 or 3; R_{g} is H or C₁₋₆ alkyl.

### 1.1. B₁, B₂, B₃

In the present application, B₁ B₂, and B₃ are each independently a linking bond or a C₁₋₂₀ alkylene group.

In a specific embodiment of the present application, the B₁, B₂, and B₃ are selected from any of the following cases:
Case (1): B₁, B₂, and B₃ are each independently a C₁₋₂₀ alkylene group;
Case (2): both B₁ and B₂ are linking bonds, and B₃ is independently a C₁₋₂₀ alkylene group at each occurrence;
Case (3): B₁ and B₂ are each independently a C₁₋₂₀ alkylene, and B₃ is a linking bond;
Case (4): B₁, B₂, and B₃ are all linking bonds;
the aforementioned C₁₋₂₀ alkylene group is preferably a C₁₋₁₀ alkylene group, more preferably selected from the group consisting of a methylene group, an ethylene group, a propylene group, a butylene group, a pentylene group, a hexylene group, and a heptylene group.

### 1.2. L₁, L₂, L₃

In the present application, L₁, L₂, and L₃ are each independently a linking bond or L_{c}, wherein the L_{c} is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-.

In a specific embodiment of the present application, L₁, L₂, and L₃ are preferably selected from any of the following cases:
Case (1): L₁, L₂, and L₃ are each independently L_{c};
Case (2): one of L₁, L₂, and L₃ is a linking bond, and the other two are each independently L_{c}; preferably, L₃ is a linking bond, L₁ and L₂ are each independently L_{c};
Case (3): two of L₁, L₂, and L₃ are linking bonds, and the other is L_{c}; preferably, L₁ and L₂ are linking bonds, and L₃ is L_{c};
Case (4): L₁, L₂, and L₃ are all linking bonds;
more preferably, L₁, L₂, and L₃ are selected from any of the following cases:
   Case (a): L₁, L₂, and L₃ are each independently selected from the group consisting of -OC(=O)-, -C(=O)O-, -C(=O)NH-, -NHC(=O)-, -OC(=O)O-, -NHC(=O)O-, and -OC(=O)NH-;
   Case (b): L₃ is a linking bond, and L₁ and L₂ are each independently selected from the group consisting of -OC(=O)-, -C(=O)O-, -C(=O)NH-, -NHC(=O)-, -OC(=O)O-, -NHC(=O)O-, and -OC(=O)NH-;
   Case (c): L₁ and L₂ are linking bonds, and L₃ is selected from the group consisting of -OC(=O)-, -C(=O)O-, -C(=O)NH-, -NHC(=O)-, -OC(=O)O-, -NHC(=O)O-, and -OC(=O)NH-.

### 1.3. R₁, R₂, R₃

In the present application, R₁, R₂, and R₃ are each independently a linear or branched C₅₋₃₀ hydrocarbon group, preferably an alkyl, alkenyl, or alkynyl group.

In a specific embodiment of the present application, the linear C₅₋₃₀ hydrocarbon group is selected from the group consisting of a linear C₅₋₃₀ alkyl, a linear C₅₋₃₀ alkenyl group, and a linear C₅₋₃₀ alkynyl group; preferably each independently a linear C₅₋₂₅ alkyl group, a linear C₅₋₂₅ alkenyl group, or a linear C₅₋₂₅ alkynyl group; more preferably, the linear C₅₋₃₀ hydrocarbon group is selected from the group consisting of the following structures: 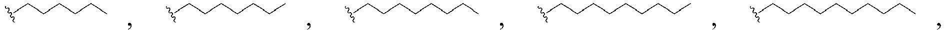 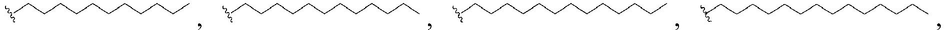 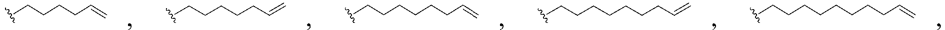 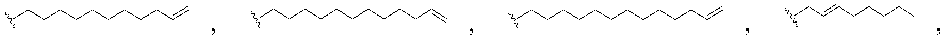 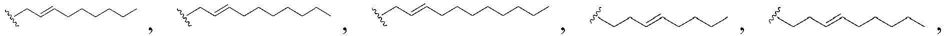 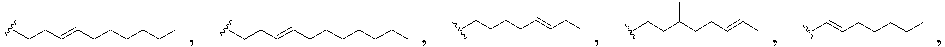 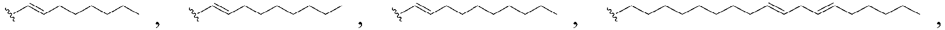 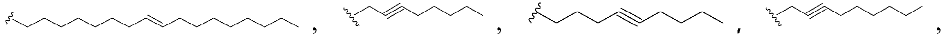 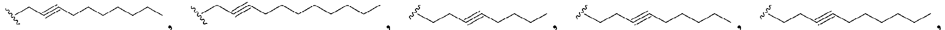 and

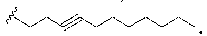

In a specific embodiment of the present application, the branched C₅₋₃₀ hydrocarbon group is selected from a branched C₅₋₃₀ alkyl group, a branched C₅₋₃₀ alkenyl group, and a branched C₅₋₃₀ alkynyl group, each independently represented by 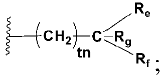 wherein, tn is an integer from 0 to 12; Rₑ and R_{f} are each independently selected from the group consisting of a C₁₋₁₅ alkyl group, a C₂₋₁₅ alkenyl group, and a C₂₋₁₅ alkynyl group; preferably, Rₑ and R_{f} are each independently selected from a butyl group, a pentyl group, a hexyl group, a heptyl group, a octyl group, a nonyl group, a decyl group, a undecyl group, a dodecyl group, a pentenyl group, a hexenyl group, a heptenyl group, a octenyl group, a nonenyl group, and a decenyl group; the branched C₅₋₃₀ hydrocarbon group is most preferably selected from the consisting of the following structures:

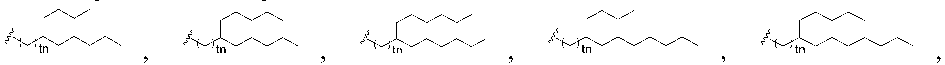

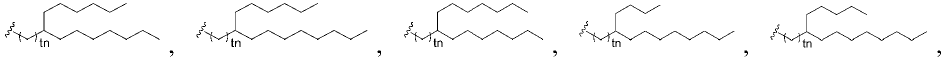

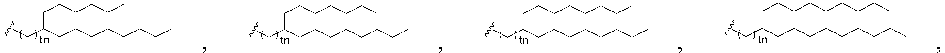

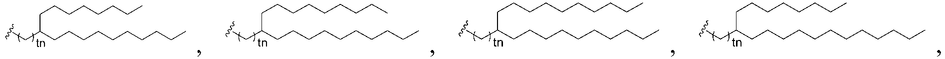

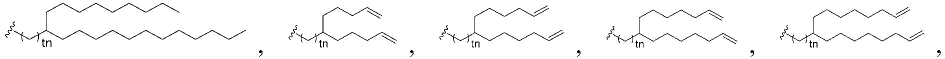

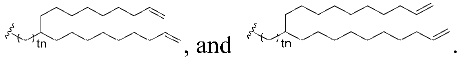

In a specific embodiment of the present application, the R₁, R₂, and R₃ are preferably selected from any of the following cases:
Case (1): the R₁, R₂, and R₃ are all linear C₅₋₃₀ hydrocarbon groups;
Case (2): the R₁, R₂, and R₃ are all branched C₅₋₃₀ hydrocarbon groups;
Case (3): one of the R₁, R₂, and R₃ is a linear C₅₋₃₀ hydrocarbon group, and the other two are branched C₅₋₃₀ hydrocarbon groups;
Case (4): any two of the R₁, R₂, and R₃ are linear C₅₋₃₀ hydrocarbon groups, and the other is a branched C₅₋₃₀ hydrocarbon group;
Preferably, R₁, R₂, and R₃ are all linear C₅₋₃₀ hydrocarbon groups.

### 1.4. R

In a specific embodiment of the present application, the R is selected from the group consisting of the following structures: 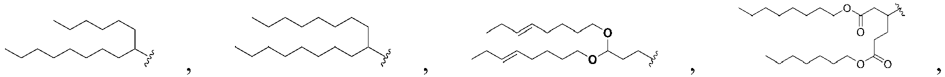 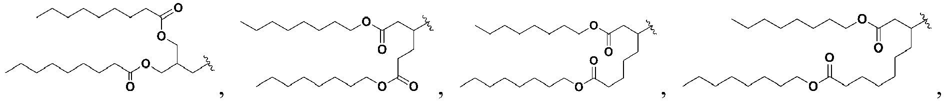 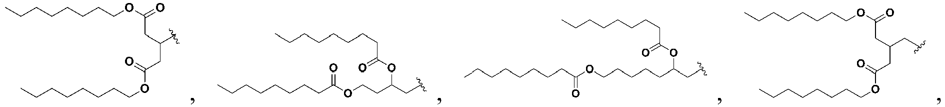 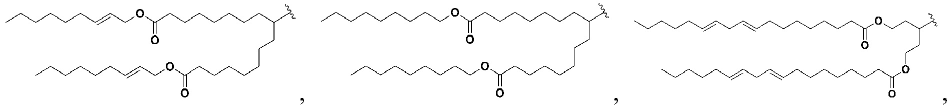 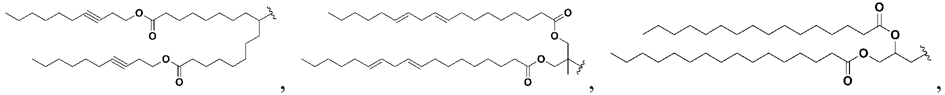 and

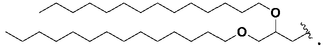

### 1.5. the fragments -B₃-L₃-R₃

In a specific embodiment of the present application, the fragments -B₃-L₃-R₃ is independently selected from the group consisting of the following structures: 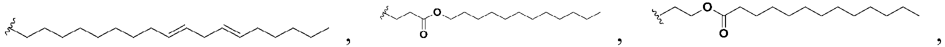 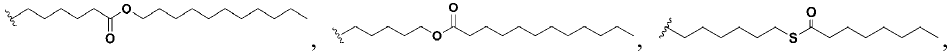 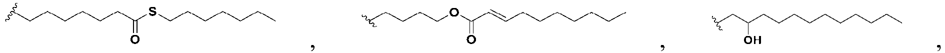 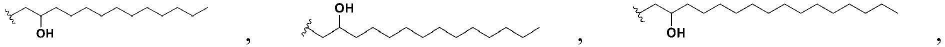 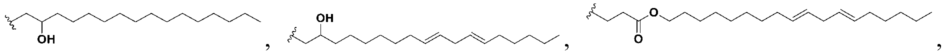 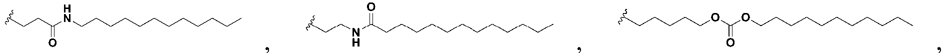 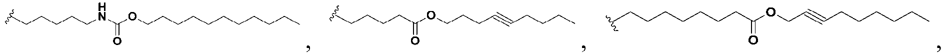 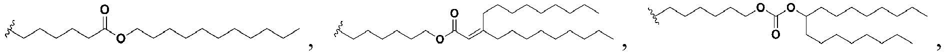 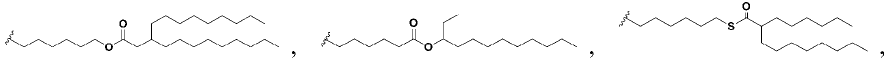 and

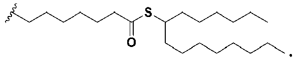

### 1.6. Examples of general formulas of structures

In a specific embodiment of the present application, the cationic lipid satisfies any one of the following general formulas: 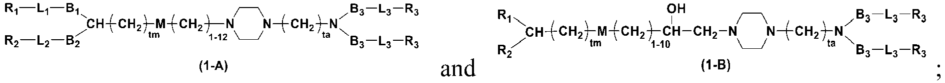 wherein, L₁ and L₂ in formula (1-A) are divalent linking bonds; wherein, M is -OC(=O)- or -C(=O)O-; the definitions of other symbols are the same as those in formula (1).

In a specific embodiment of the present application, the aforementioned cationic lipid is preferably satisfies any one of the following general formulas: 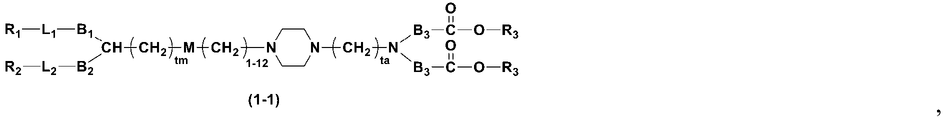 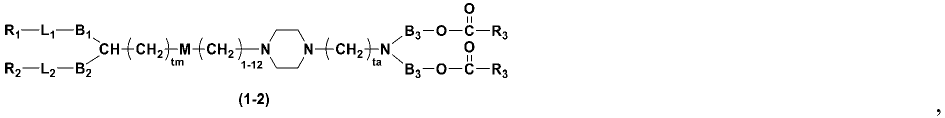 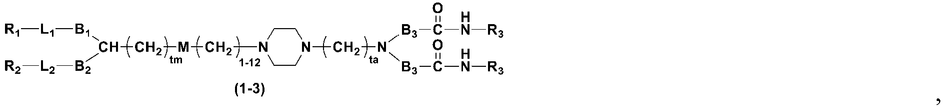 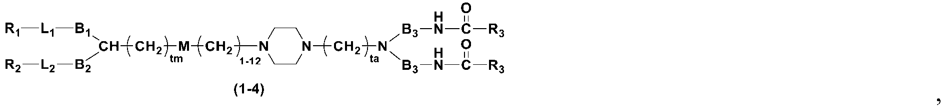 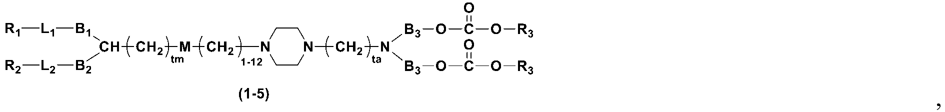 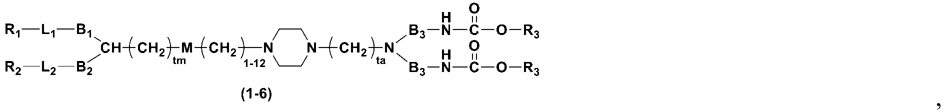 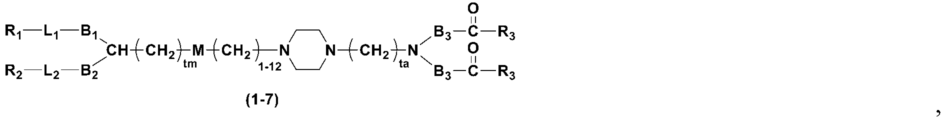 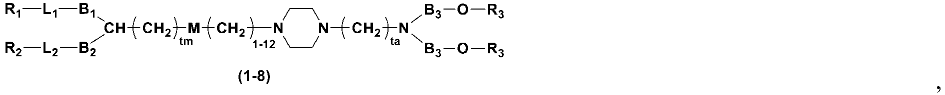 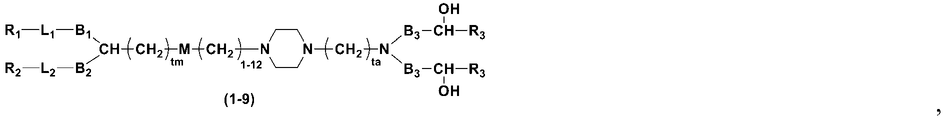 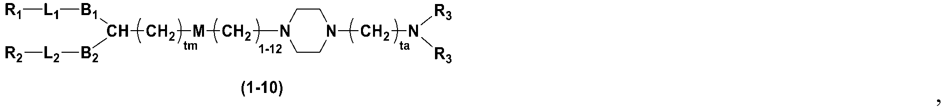 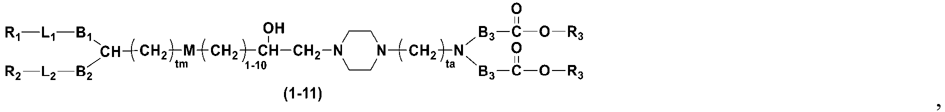 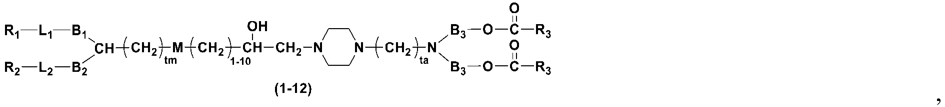 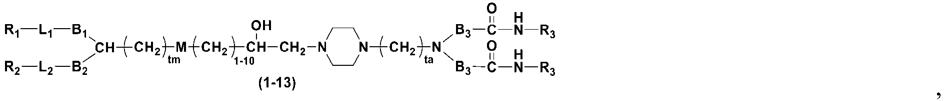 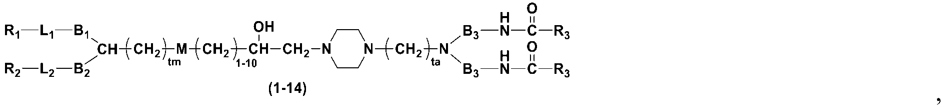 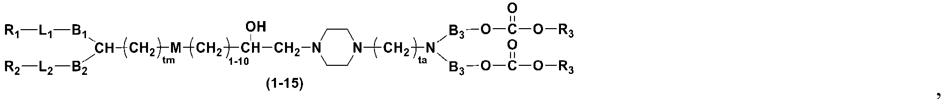 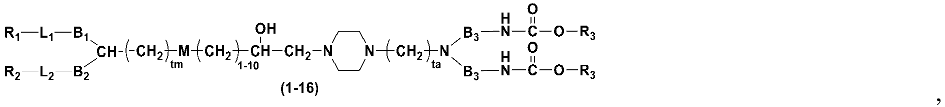 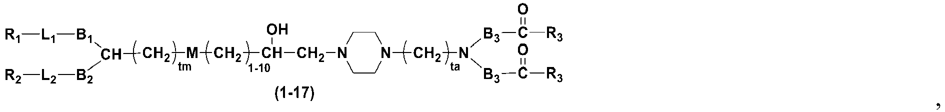 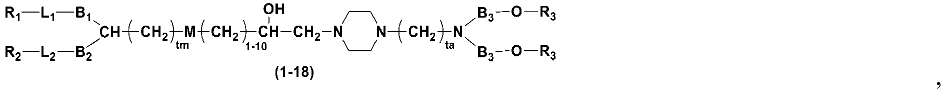 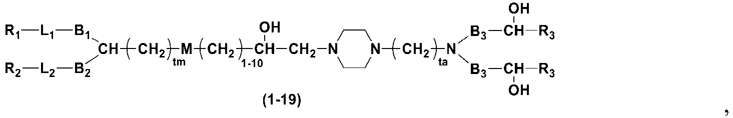 and 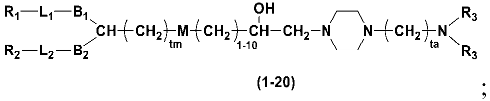 more preferably, B₁ and B₂ of the aforementioned formulas (1-1) to (1-10) are the same, and each a linking bond or a C₁₋₁₂ alkylene group; the L₁ and L₂ are the same and each selected from the group consisting of -O-, -OC(=O)-, -C(=O)O-, -NHC(=O)-, -C(=O)NH-, and -OC(=O)O-; R₁ and R₂ of the formulas (1-1) to (1-20) are the same and each a linear C₅₋₃₀ hydrocarbon group; R₃ is a linear C₅₋₃₀ hydrocarbon group.

### 1.7. Examples of specific structures

In a specific embodiment of the present application, the cationic lipid is preferably selected from the group consisting of the following structures: 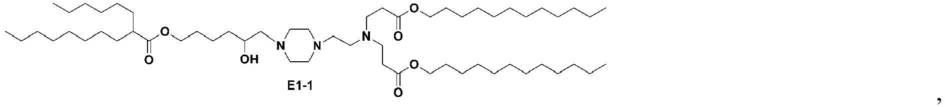 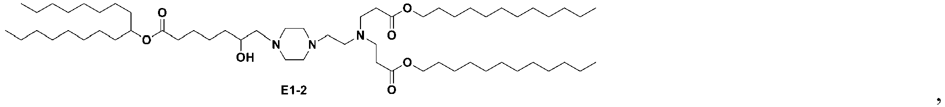 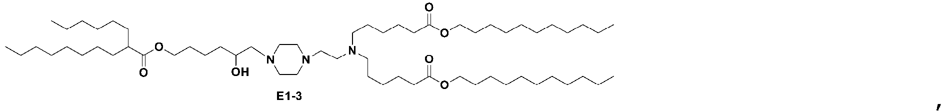 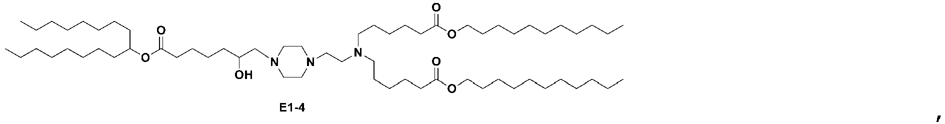 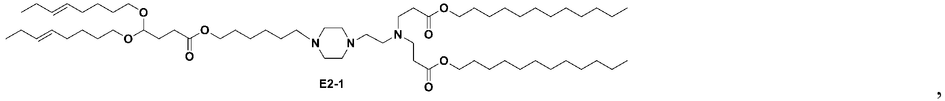 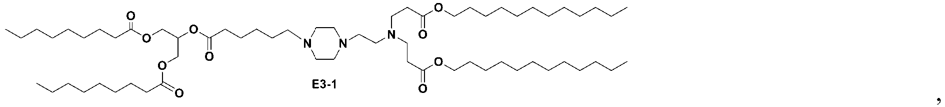 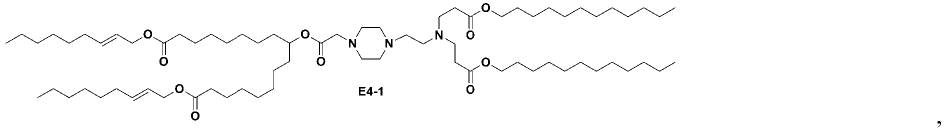 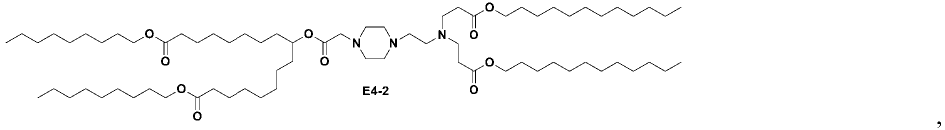 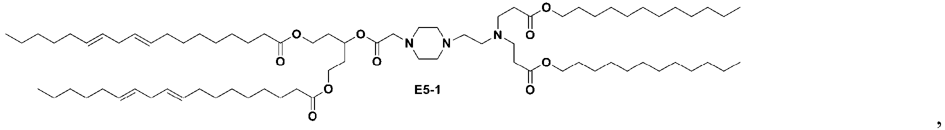 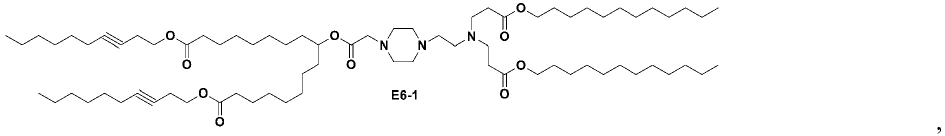 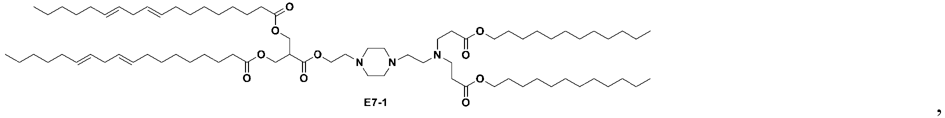 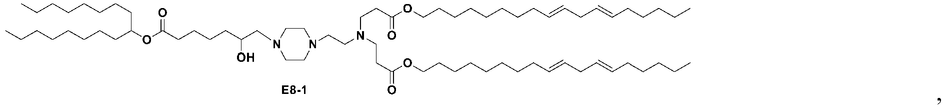 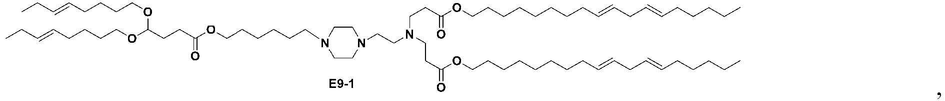 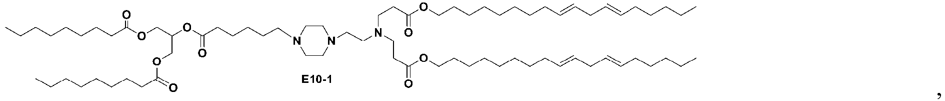 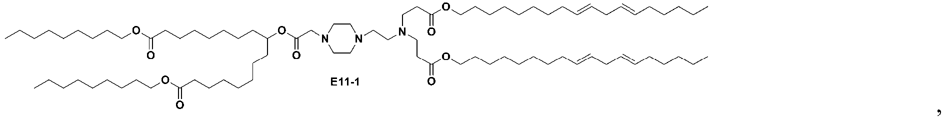 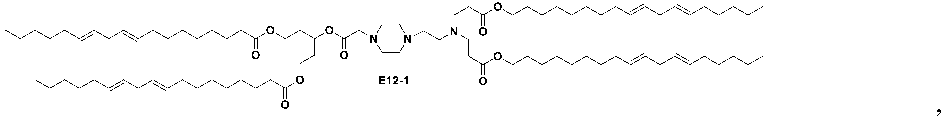 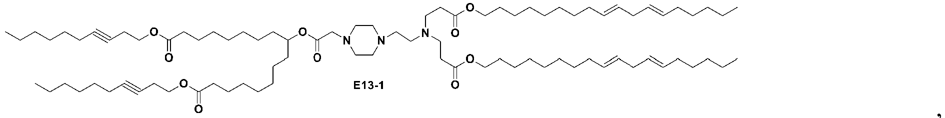 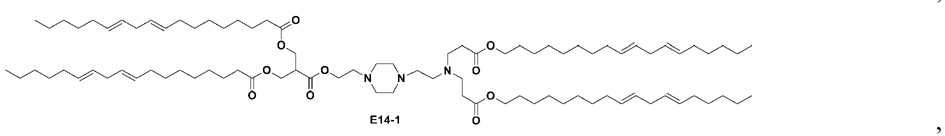 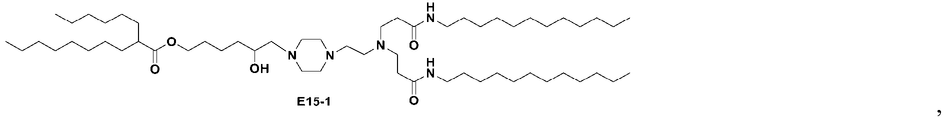 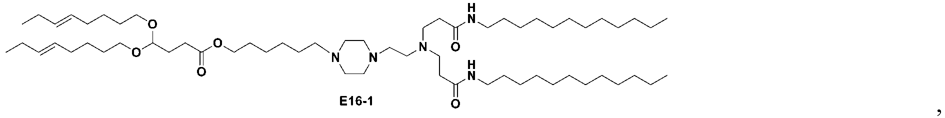 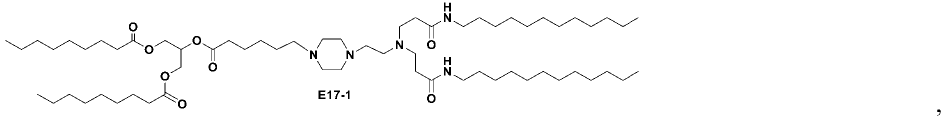 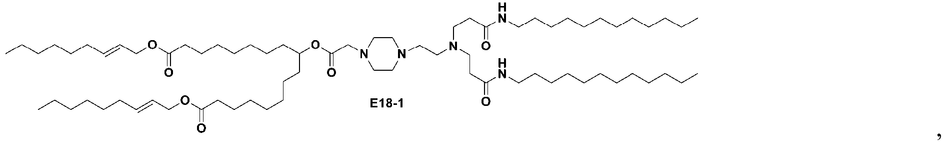 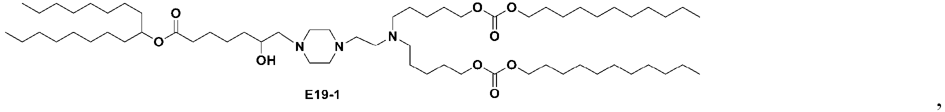 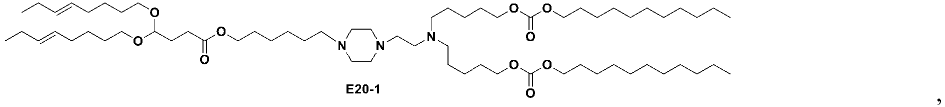 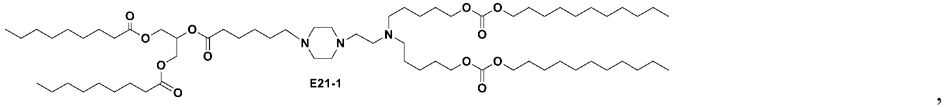 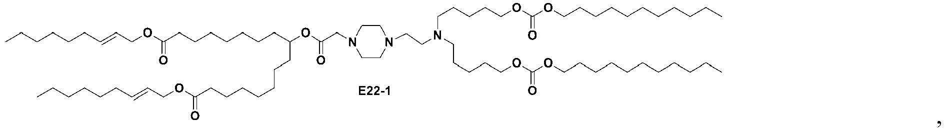 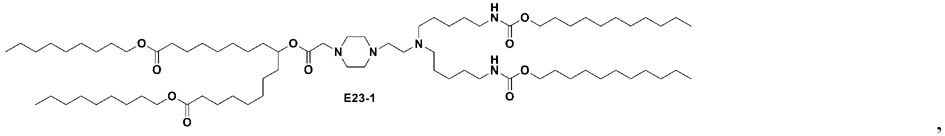 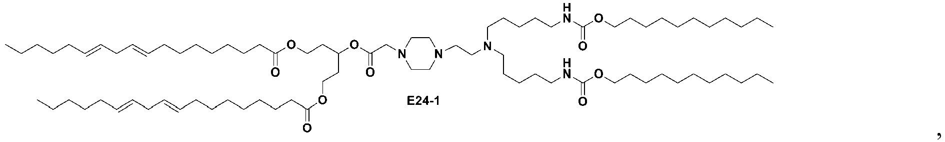 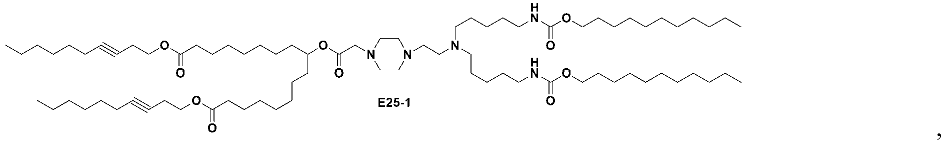 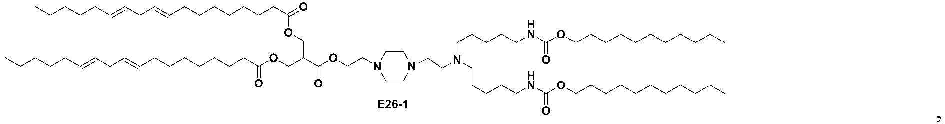 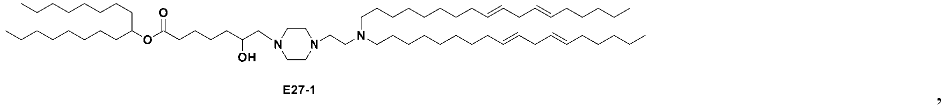 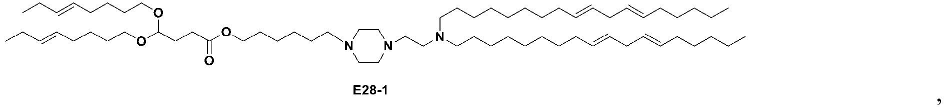 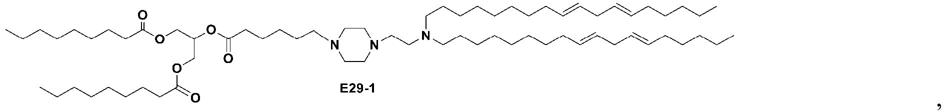 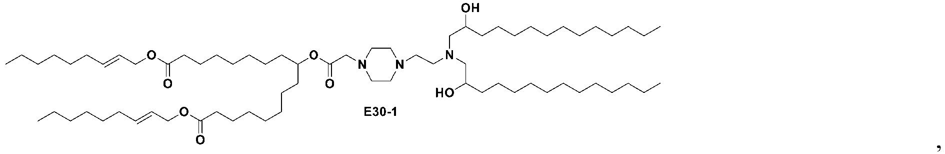 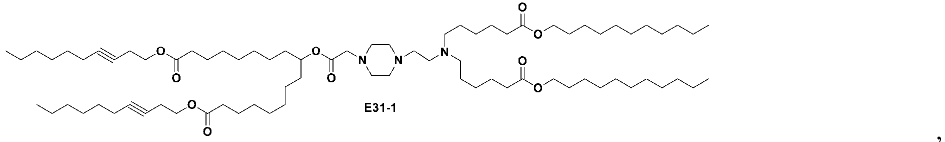 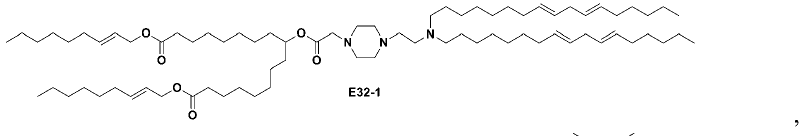 and 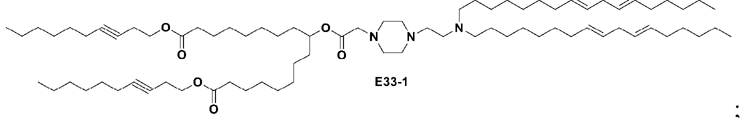 or the cationic lipid is selected from the group consisting of the following structures: 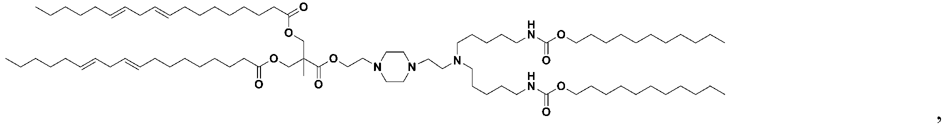 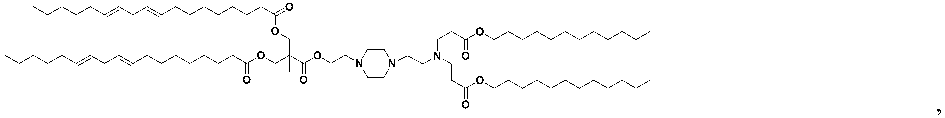 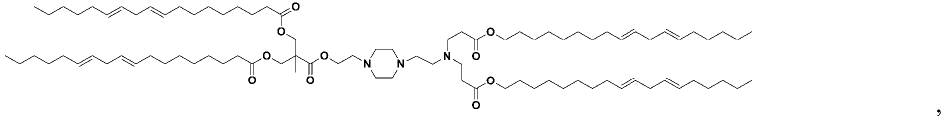 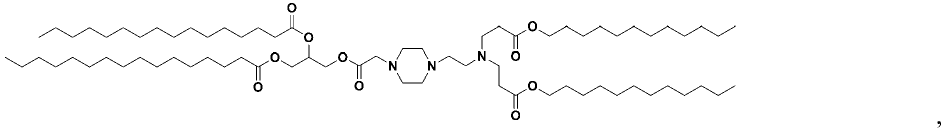 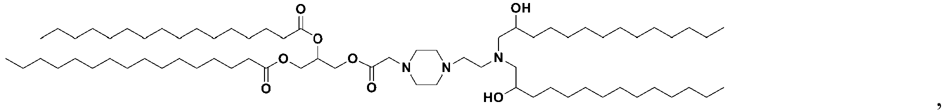 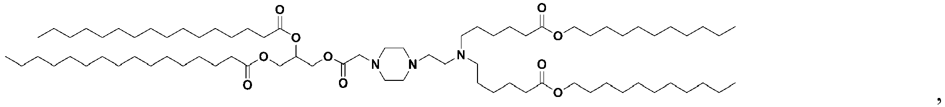 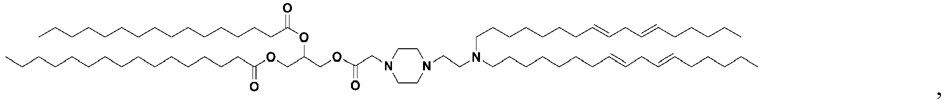 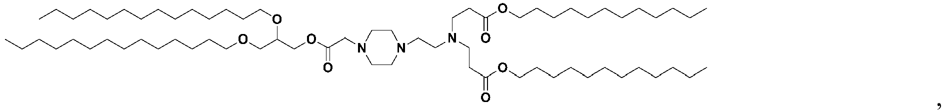 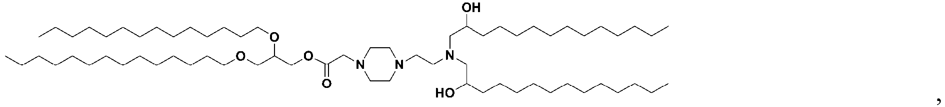 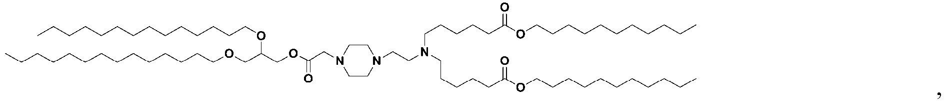 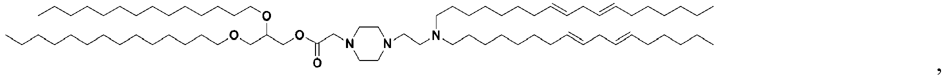 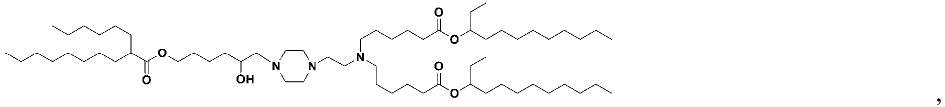 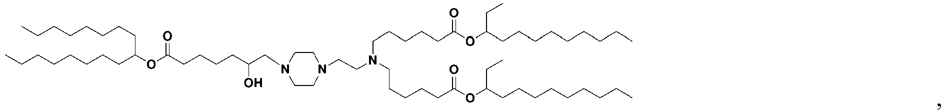 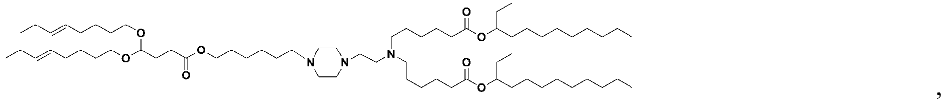 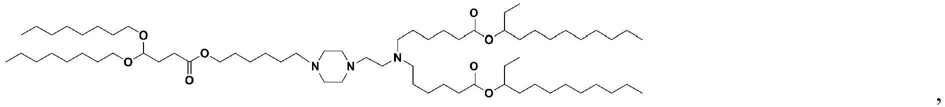 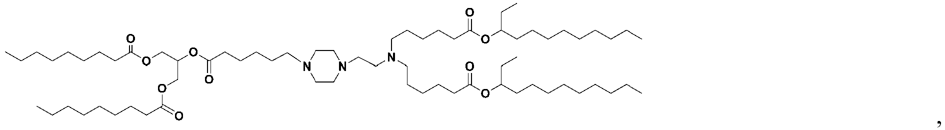 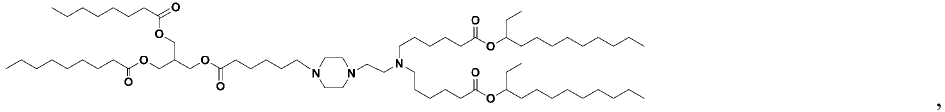 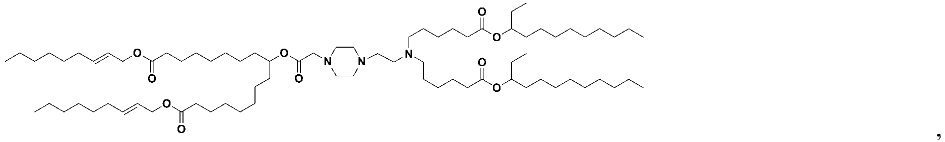 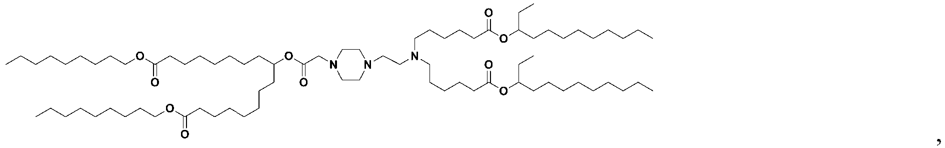 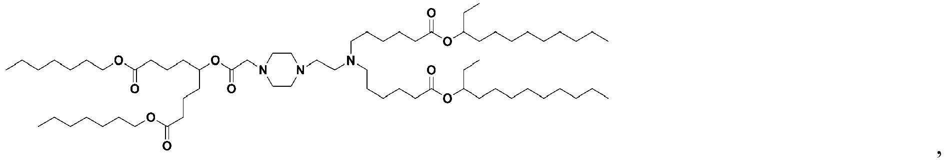 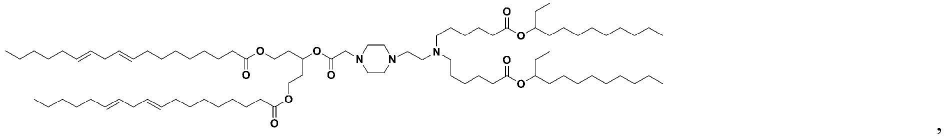 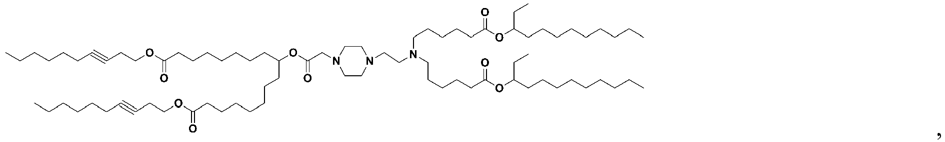 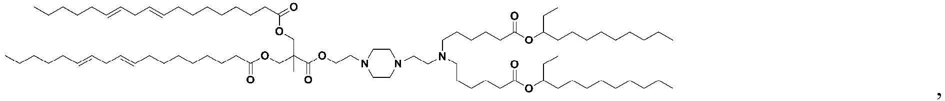 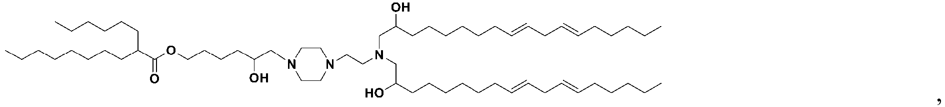 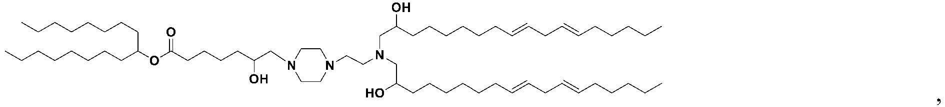 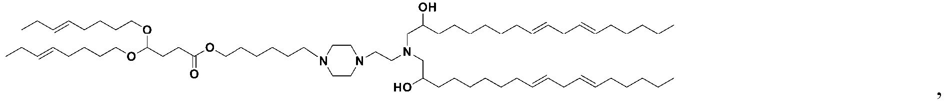 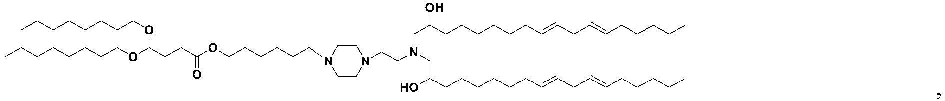 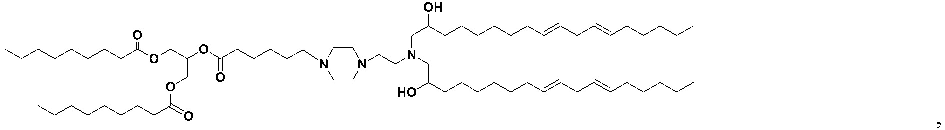 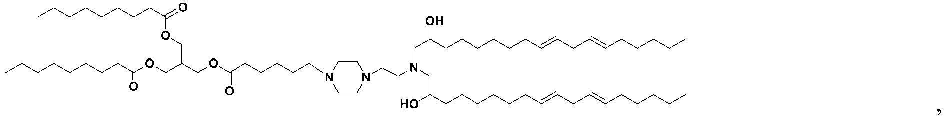 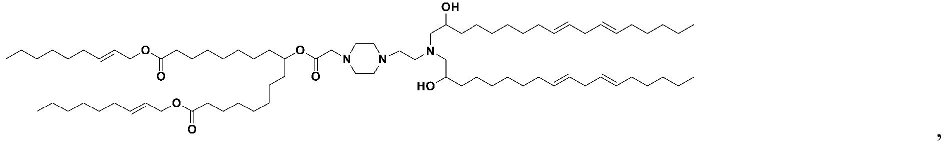 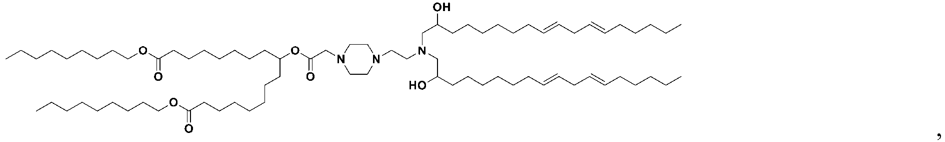 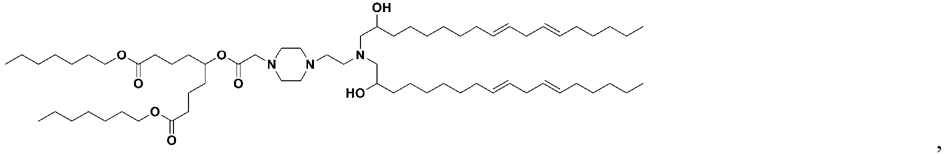 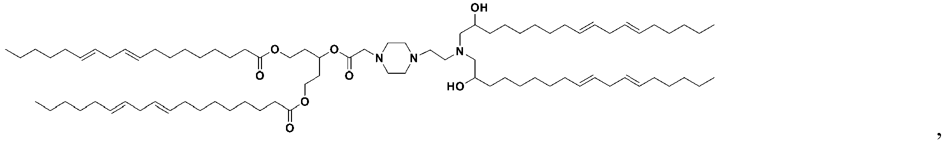 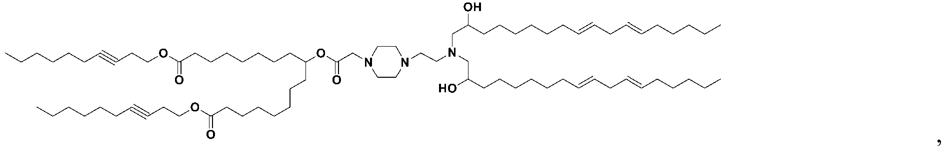 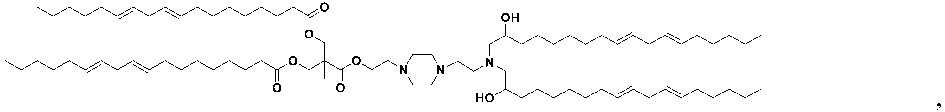 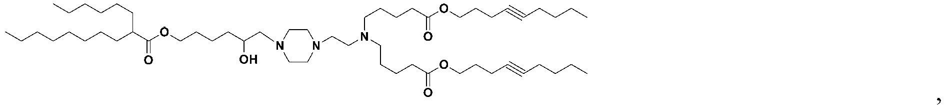 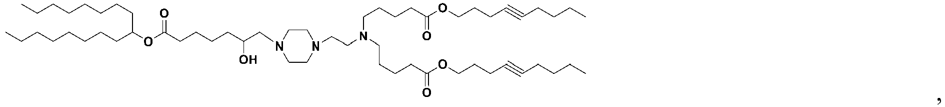 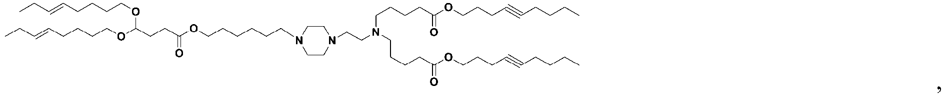 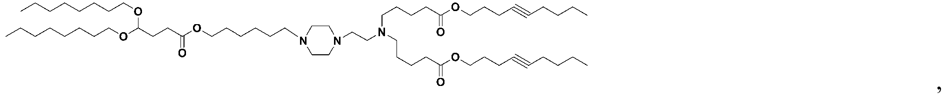 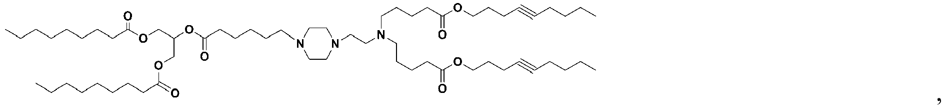 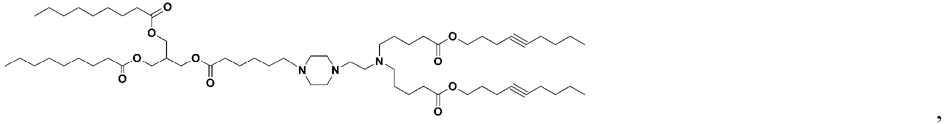 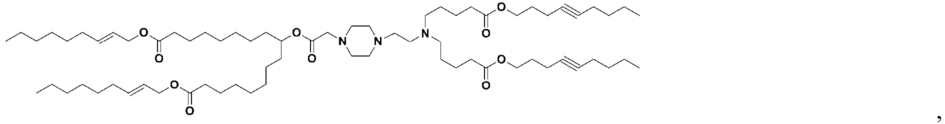 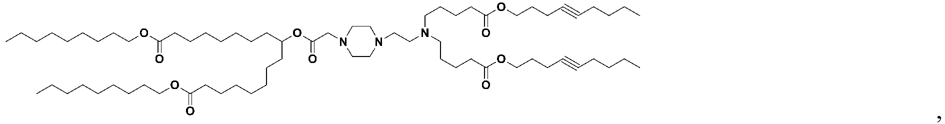 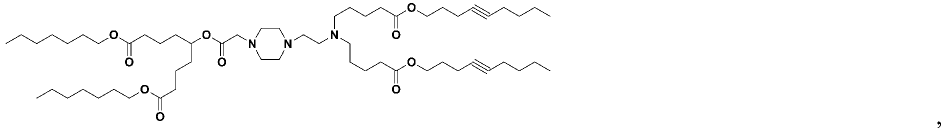 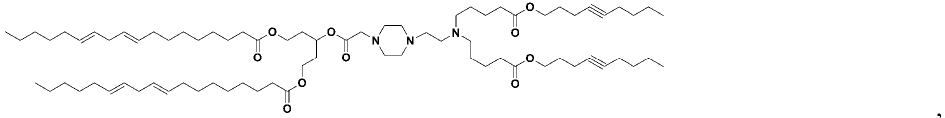 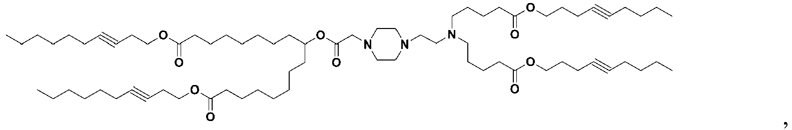 and

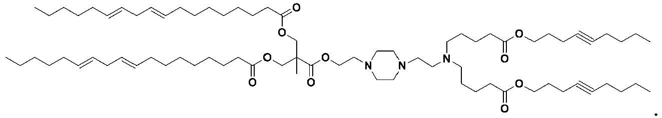

### 2. Preparation of Cationic Lipids

**2.1.** In a specific embodiment of the present application, the preparation process of the cationic lipid represented by formula (1) is as follows:
The intermediates/starting materials involved in the preparation process of cationic lipids of the present application include but are not limited to PIP₀, IM-N, IM-C, PIP-N, and PIP-C.

The preparation process of the present application may involve a starting material PIP₀ containing a piperazine ring, wherein PIP₀ contains F_{q} and F₀ as the same or different functional groups, and the structures is represented by 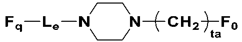 or 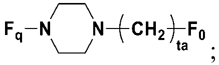 wherein, F_{q} is H, a protected or unprotected form of -OH, a protected or unprotected form of -COOH, F₀ is a protected or unprotected form of -NH₂, and the definitions of other symbols are the same as those in formula (1).

In the present application, PIP₀ is selected from the group consisting of the following structures: 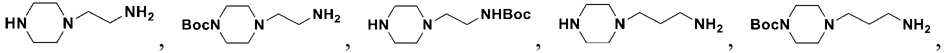 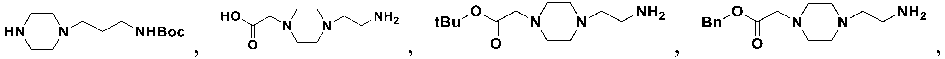 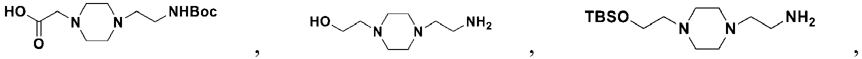 and

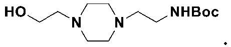

The preparation process of the present application may involve a small-molecule intermediate IM-N with a reactive group and a hydrophobic tail chain, and its structure is represented by F_{B}-B₃-L₃-R₃; wherein, F_{B} is a reactive group that can react with F₀, preferably selected from -OH, -COOH, -F, -Cl, -Br, -CHO, 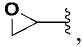 or -CH=CH-; the definitions of other symbols are the same as those in formula (1). IM-N can be prepared by any suitable chemical reaction, which includes simple esterification, amidation, alkylation, addition, and substitution reactions, etc., either as single-step or multi-step processes. For example, in Example 8, the small-molecule intermediate IM-N ( 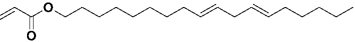 ) can be obtained by a substitution reaction between S8-1 ( 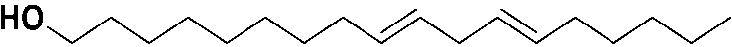 ) and S8-2 ( 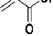 ).

In the present application, the structure of the aforementioned IM-N is preferably selected from the group consisting of F_{B}-B₃-L₃-R₃, F_{B}-L₃-R₃, and F_{B}-R₃; specifically, IM-N is more preferably selected from the group consisting of the following structures: 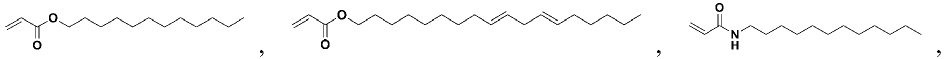 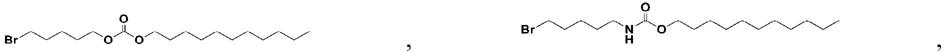 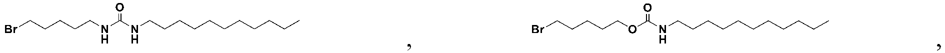 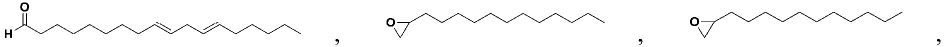 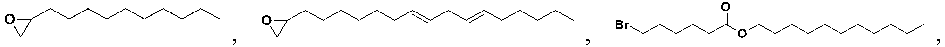 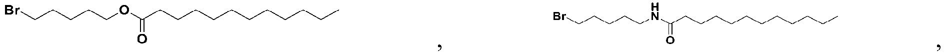 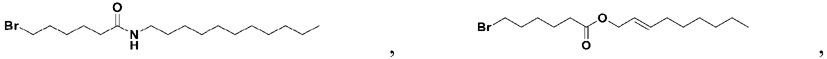 and

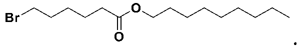

The preparation process of the present application may involve an intermediate IM-C containing one reactive group and a carbon-branched hydrophobic tail chain, and its structure is represented by 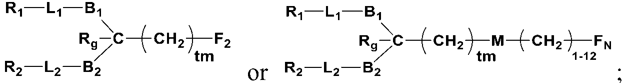 wherein F₂ or F_{N} are reactive groups that can react with the F_{q} of PIP₀, F₂ is preferably a protected or unprotected form of -OH, or a protected or unprotected form of -COOH, F_{N} is preferably selected from -Br, -CHO, -CH=CH-, or 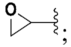 when B₁, B₂, L₁, and L₂ are both linking bonds, F_{N} is 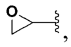 and the definitions of other symbols are the same as those in formula (1). IM-C can be prepared by any suitable chemical reaction, which includes simple esterification, amidation, alkylation, addition, and substitution reactions, etc., either as single-step or multi-step processes. For example, in Example 5, the small-molecule intermediate IM-C ( 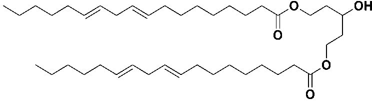 ) can be obtained by a esterification reaction between S5-1 ( 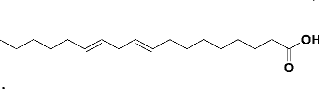 ) and S5-2 ( 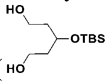 ), followed by the removal of protecting group.

In the present application, the structure of the aforementioned IM-C is preferably selected from the group consisting of 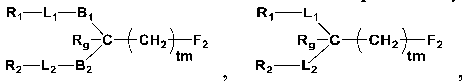 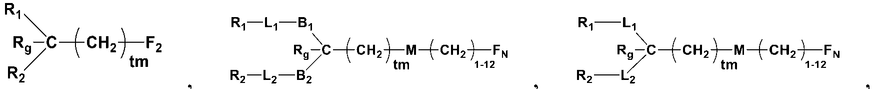 and 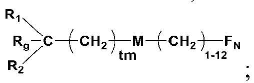 specifically, PIP-C is selected from the group consisting of the following structures:

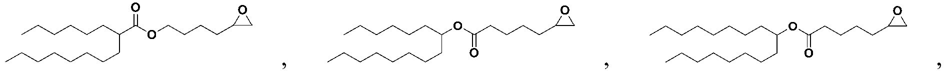

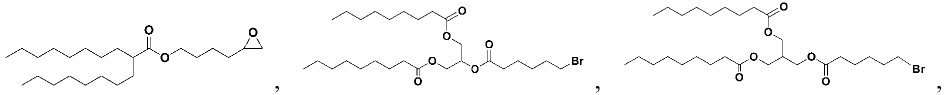

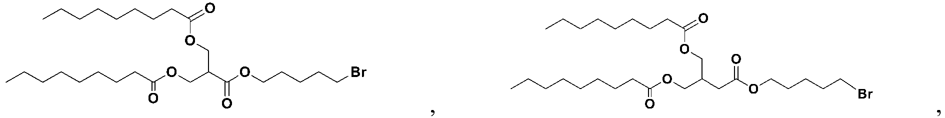

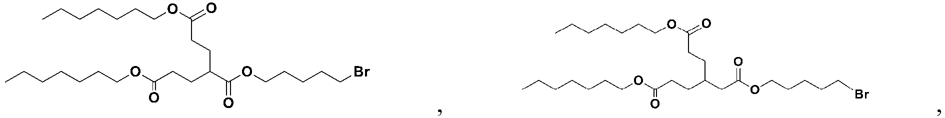

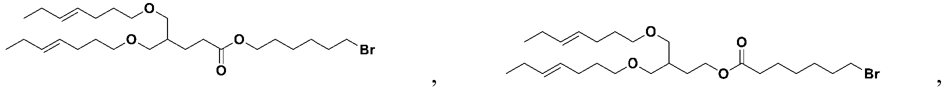

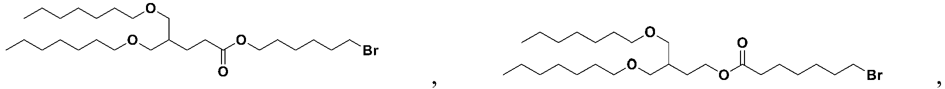

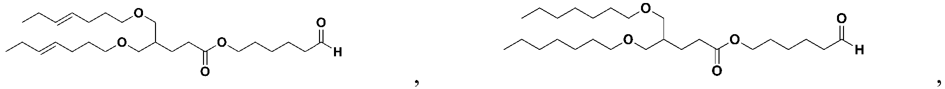

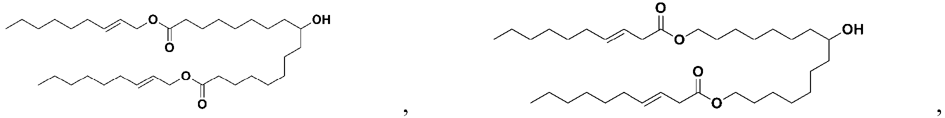

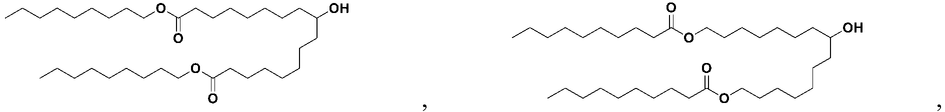

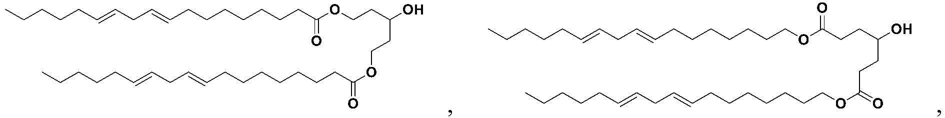

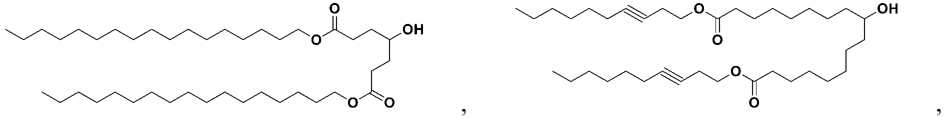

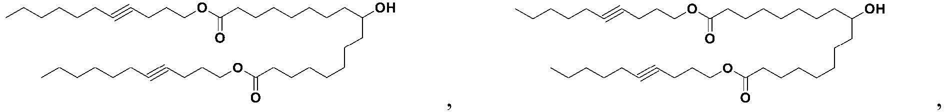

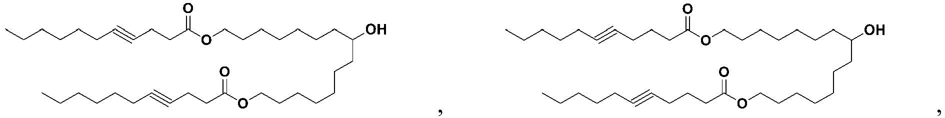

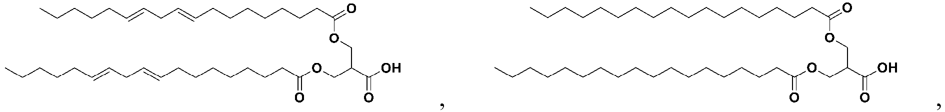

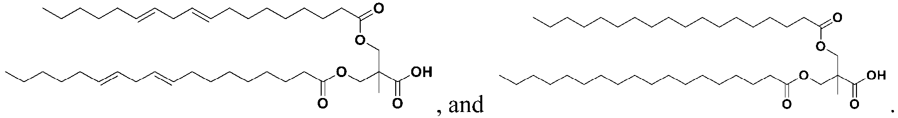

The preparation process of the present application may involve an intermediate PIP-N containing a piperazine ring, a reactive group, and a nitrogen-branched hydrophobic tail chain, and its structure is represented by 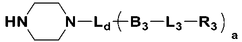 or 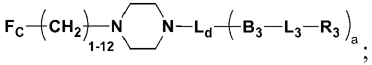 wherein, F_{C} is a reactive group that can react with the F₂ of IM-C, preferably selected from -OH or -COOH; the definitions of other symbols are the same as those in formula (1). PIP-N can be obtained by PIP₀ with IM-N through a single or multiple steps. For example, in Example 1, the intermediate PIP-N ( 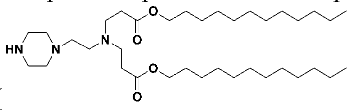 ) can be obtained by an addition reaction between S1-1 ( 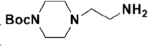 ) and S1-2 ( 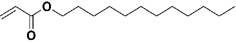 ), followed by the removal of protecting group.

In the present application, the structure of the aforementioned PIP-N is preferably selected from the group consisting of 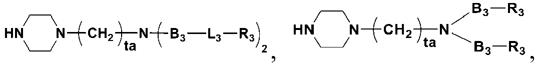 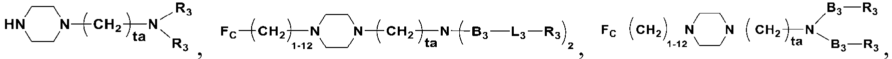 and 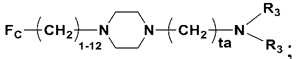 specifically, PIP-N is selected from the group consisting of the following structures: 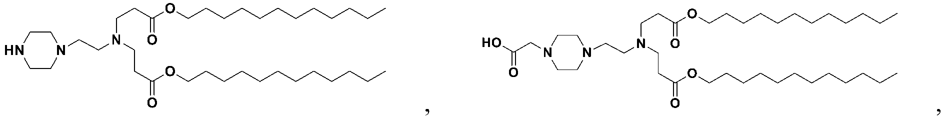 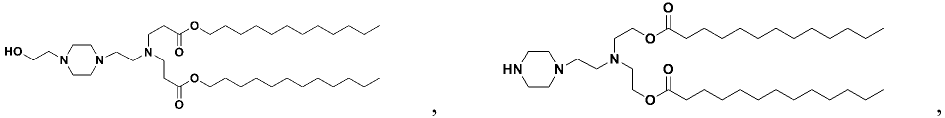 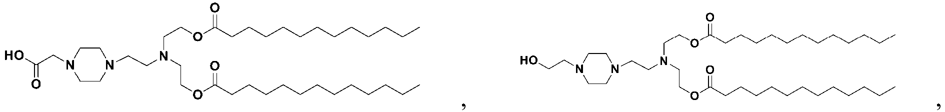 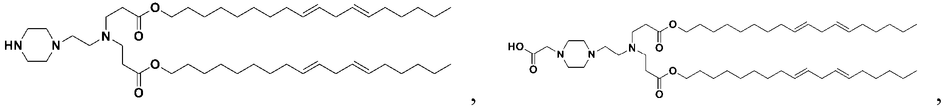 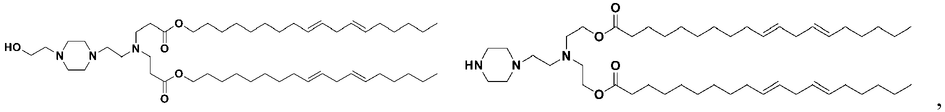 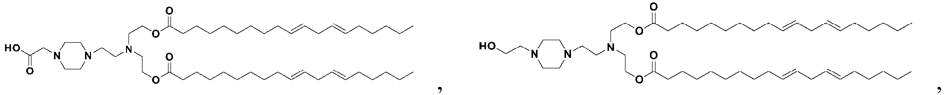 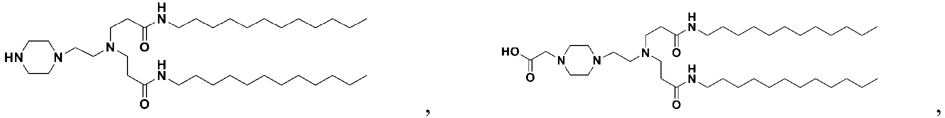 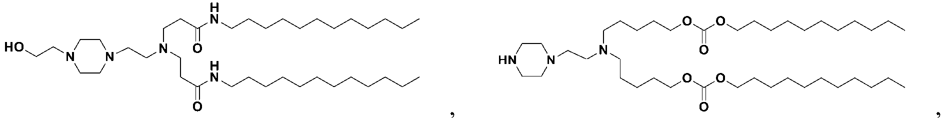 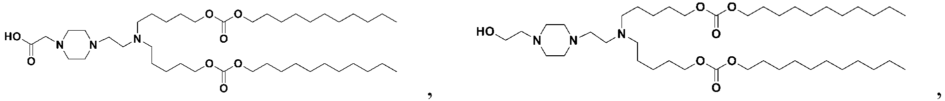 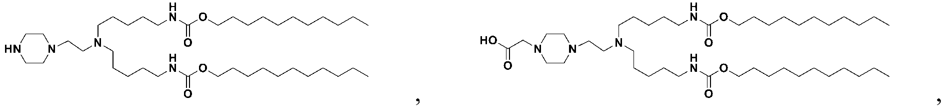 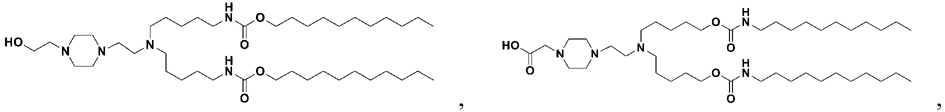 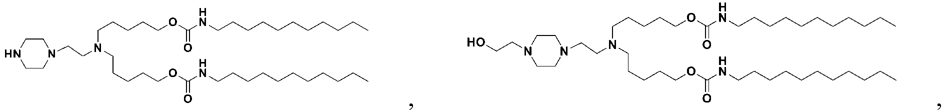 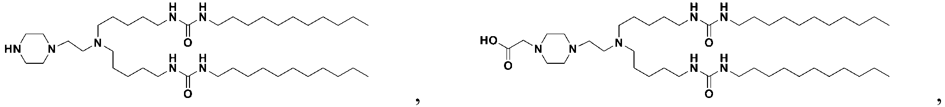 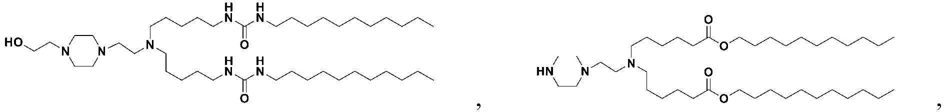 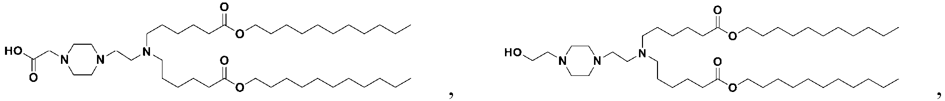 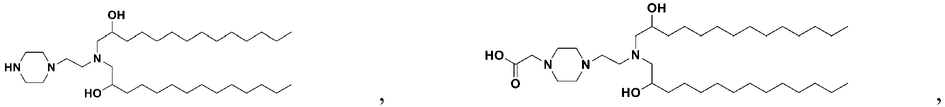 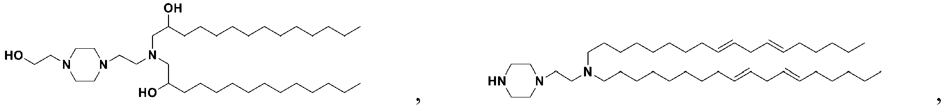 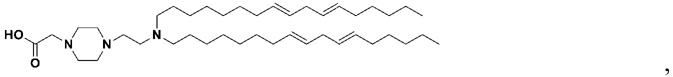 and

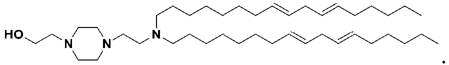

The preparation process of the present application may involve an intermediate PIP-C containing a piperazine ring, a primary amine, and a carbon-branched hydrophobic tail chain, and its structure is represented by 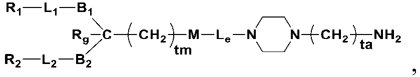 and the definitions of other symbols are the same as those in formula (1). PIP-C can be obtained by reacting the intermediate IM-C with the starting material PIP₀. For example, in Example 30, the intermediate PIP-C ( 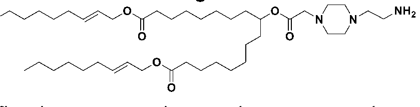 ) can be obtained by performing an esterification reaction between the intermediate IM-C ( 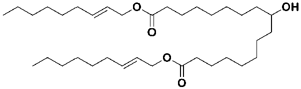 ) and PIP₀ ( 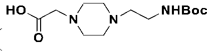 ).

In the present application, the structure of PIP-C is 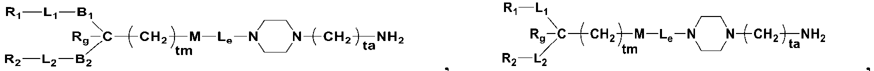 or 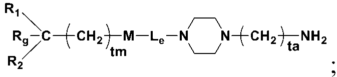 specifically, PIP-C is selected from the group consisting of the following structures: 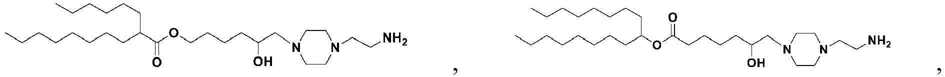 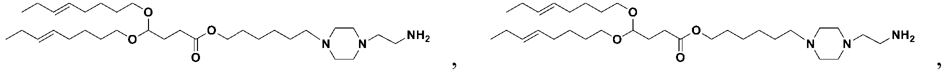 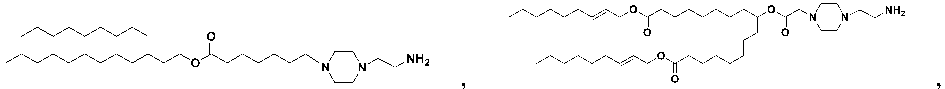 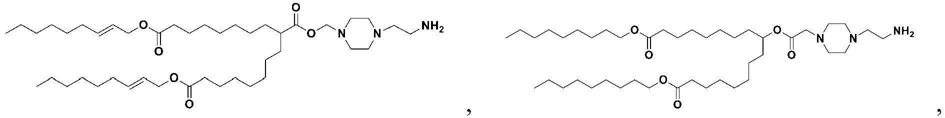 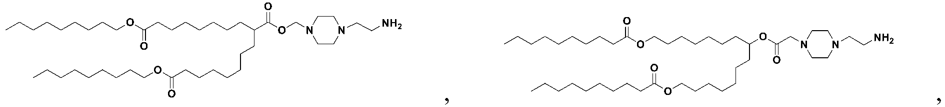 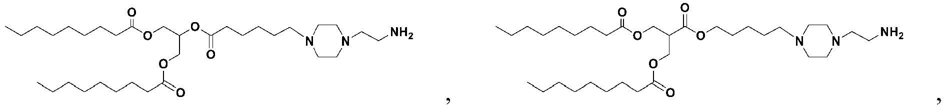 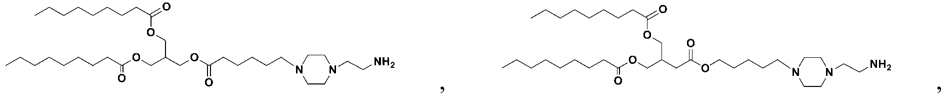 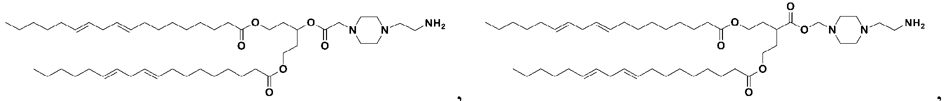 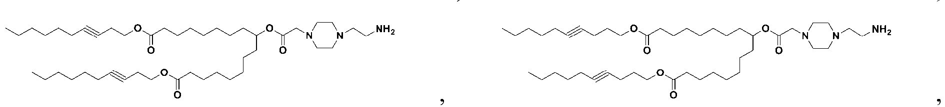 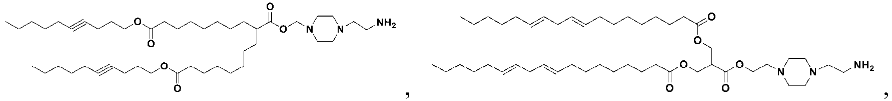 and

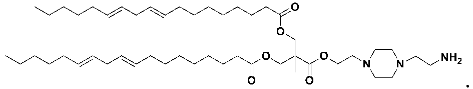

In the present application, the cationic lipid can be obtained by reacting any of the aforementioned IM-N with any of PIP₀ to obtain PIP-N, followed by reaction with IM-C; and it can also be obtained by reacting any of the aforementioned IM-C with any of PIP₀ to obtain PIP-C, followed by reaction with IM-N.

### 2.2. Description of Relevant Starting Materials and/or Steps in the Preparation Process

### 2.2.1. Condensation Agents, Oxidizing Agents, and Reducing Agents

In the present application, the condensation agent used in reactions is not particularly limited, but is preferably selected from the group consisting of *N,N*'-dicyclohexylcarbodiimide (DCC), 1-ethyl-(3-dimethylaminopropyl)carbodiimide hydrochloride (EDC·HCl, EDCI), 2-(7-azabenzotriazole)-*N*,*N*,*N*',*N*'-tetramethyluronium hexafluorophosphate (HATU), and benzotriazole-*N*,*N*,*N*',*N*'-tetramethyluronium hexafluorophosphate (HBTU), most preferably DCC. Relevant reactions can be facilitated by the addition of an appropriate catalyst (e.g., 4-dimethylaminopyridine).

In the present application, the oxidizing agent used in reactions is not particularly limited, as long as it is a compound or a combination of compounds that can increase the valency of the substrate, and is preferably selected from the group consisting of phenyliodine(III) bis(trifluoroacetate), 1,4-benzoquinone, benzyltrimethylamonium tribromide, pyridine dichromate, potassium dichromate, ozone, oxygen, hypofluorous acid, sodium hypochlorite, cobalt(III) acetate, cobalt acetate, manganese acetate, palladium acetate, copper acetate, monoperoxyphthalic acid, iodine, *N*-iodosuccinimide, iodoylbenzene, 2-iodoylbenzoic acid, dimethyldioxirane, dimethyl sulfoxide-chloroformyl, dimethyl sulfoxide-acetic anhydride, DDQ, dichlorotris(triphenylphosphine) ruthenium, manganese dioxide, diacetoxyiodobenzene, periodic acid, sodium periodate, sodium periodate-osmium tetroxide, potassium permanganate, sodium perborate, benzoic acid peroxide, dibenzoyl peroxide, nickel peroxide, hydrogen peroxide, cumene hydroperoxide, *t*-butyl hydroperoxide, peracetic acid, *m*-chloroperbenzoic acid, *N-*chlorosuccinimide, pyridinium chlorochromate, palladium chloride-copper chloride, urea-hydrogen peroxide complex, trityl tetrafluoroborate, tributyltin oxide, cobalt trifluoride, vanadium oxytrifluoride, chromium trioxide, manganese triacetate, TEMPO, ammonium ceric nitrate, bromine, *N*-oxidopyridine, silver oxide, *O*-ethylcarbonate peroxide, manganese acetylacetonate, vanadium oxyacetylacetonate, aluminum isoproxide, potassium peroxymonosulfate, dichloroiodobenzene, etc., and combinations thereof; more preferably, the oxidizing agent is selected from the group consisting of oxygen, sodium hypochlorite, hydrogen peroxide, dichloroiodobenzene, potassium peroxymonosulfate, etc., and combinations thereof.

In the present application, there is no particular limitation on the reducing agent used in reactions, as long as it can reduce the Schiff base formed from the reaction between ammonia and aldehydes or ketones to an amino group, and is preferably selected from the group consisting of sodium borohydride, sodium cyanoborohydride, lithium aluminum hydride, borane, diborane, diisobutyl aluminum hydride, diisopine campyl borane, lithium borohydride, zinc borohydride, borane-pyridine, borane-methyl sulfide, borane-tetrahydrofuran, etc., and combinations thereof; the reducing agent is more preferably sodium cyanoborohydride.

In the present application, the solvent used in reactions may be absent or selected from non-protonic solvents. The non-protonic solvents include toluene, benzene, xylene, acetonitrile, ethyl acetate, ether, methyl t-butyl ether, tetrahydrofuran, chloroform, dichloromethane, dimethyl sulfoxide, dimethylformamide or dimethylacetamide, preferably tetrahydrofuran, dichloromethane, dimethyl sulfoxide, and dimethylformamide.

In the present application, the base used in reactions is an inorganic base or an organic base, preferably an organic base (e.g., triethylamine, pyridine, 4-dimethylaminopyridine, imidazole, or diisopropylethylamine); preferably, the base is triethylamine or pyridine.

### 2.2.2. "Protection" and "Deprotection" of Relevant Groups Involved in the Reaction Process

In the present application, reaction processes may involve "protection" and "deprotection" processes of relevant groups. To prevent a certain functional group from affecting the reaction, it is usually protected. In addition, when two or more functional groups are present, only the target functional group is selectively required to react, and the other functional groups should therefore be protected. The protecting group not only protects the functional group stably but also needs to be easily removed as needed. Therefore, in organic synthesis, it is important to remove only the protecting group bonded to the specified functional group under appropriate conditions.

In the present application, the definitions of "carboxyl protecting group" and "amino protecting group" are consistent with those in the "Description of Terms" section, and will not be repeated herein.

In the present application, the hydroxyl groups protected by hydroxyl protecting groups are not particularly limited, e.g., the hydroxyl groups of alcoholic hydroxyl groups, phenolic hydroxyl groups, etc. The amino groups protected by amino protecting groups are not particularly limited, such as those from primary amines, secondary amines, hydrazines, amides, etc. The amino groups in the present application are not particularly limited, including but not limited to primary amino groups, secondary amino groups, tertiary amino groups, and quaternary ammonium ions.

In the present application, the deprotection of protected hydroxyl groups is related to the types of hydroxyl protecting groups. The types of hydroxyl protecting groups are not particularly limited; for example, benzyl groups, silyl ethers, and t-butyl groups can be used to protect terminal hydroxyl groups, and the corresponding deprotection methods include the following:

### A: Deprotection of benzyl protecting groups

The deprotection of benzyl groups can be achieved via hydrogenation using hydrogenation reducing agents and hydrogen donors. The water content in the reaction system should be less than 1% to facilitate the reaction.

The catalyst for hydrogenation reduction is not particularly limited, preferably palladium or nickel. The catalyst support is not particularly limited, preferably aluminum oxide or carbon, and more preferably carbon. The amount of palladium used is 1 to 100 wt% relative to the compounds containing protected hydroxyl groups, preferably 1 to 20 wt%.

The reaction solvent is not particularly limited, as long as both starting materials and products can be dissolved, but is preferably methanol, ethanol, ethyl acetate, tetrahydrofuran, or acetic acid, more preferably methanol. The hydrogen donor is not particularly limited but is preferably gaseous hydrogen, cyclohexene, 2-propanol, or ammonium formate, etc. The reaction temperature is preferably in the range of 25 to 40°C. The reaction time is not particularly limited and is negatively correlated with the amount of catalyst used and preferably in the range of 1 to 5 h.

### B: Deprotection of silyl ether protecting groups

The protected hydroxyl groups in the forms of silyl ethers include trimethyl silyl ether, triethyl silyl ether, t-butyl dimethyl silyl ether, t-butyl diphenyl silyl ether, etc. The deprotection of such silyl ethers is carried out using compounds containing fluoride ions; wherein, said compounds are preferably selected from the group consisting of tetrabutylammonium fluoride, tetraethylammonium fluoride, hydrofluoric acid, and potassium fluoride, and more preferably tetrabutylammonium fluoride or potassium fluoride. The molar equivalent of the fluoride-containing compound used is 5 to 20 times that of the protected hydroxyl group, preferably 8 to 15 times that of the initiator. When the molar equivalent of the fluoride-containing compound used is less than 5 times that of the protected hydroxyl group, the deprotection might be incomplete. When the molar equivalent of the deprotection reagent used exceeds 20 times that of the protected hydroxyl group, the excess reagents or compounds will bring difficulty in the purification and possibly cause side reactions in subsequent steps. The reaction solvent is not particularly limited, as long as the reactants and the products can be dissolved, but is preferably an aprotic solvent and more preferably tetrahydrofuran or dichloromethane. The reaction temperature is preferably 0 to 30°C; when the temperature is below 0°C, the reaction rate is relatively slow, and the protecting group cannot be completely removed.

### C: Deprotection of t-butyl protecting groups

The deprotection of *t*-butyl groups is carried out under acidic conditions wherein the pH of the solution is preferably 0 to 4. The acid used is not particularly limited, but is preferably acetic acid, phosphoric acid, sulfuric acid, hydrochloric acid, or nitric acid, and more preferably hydrochloric acid. The reaction solvent is not particularly limited, as long as the reactants and the products can be dissolved, but is preferably water. The reaction temperature is preferably 0 to 30°C.

### 2.2.3. Alkylation reactions

The alkylation reactions of the present application are preferably based on the alkylation of hydroxyl groups, sulfhydryl groups or amino groups, corresponding to the formation of ether bonds, thioether bonds, secondary amino groups or tertiary amino groups, respectively. Examples include the following:

### 2.2.3.1. Alkylation reaction of substrate alcohols with sulfonates or halides

Under basic conditions, an ether intermediate can be obtained via nucleophilic substitution of the substrate alcohol with a sulfonate derivative or halide. Wherein, the molar equivalent of the sulfonate or halide is 1 to 50 times that of the substrate alcohol, preferably 1 to 5 times. When the molar equivalent of the sulfonate or halide is less than 1 times that of the substrate alcohol, the substitution might be incomplete and cause difficulty in the purification process. When the molar equivalent of the sulfonate or halide exceeds 50 times that of the substrate alcohol, the excess sulfonate or halide tends to bring difficulty in the purification process, and might be brought into the subsequent step and therefore cause more side reactions which further increase difficulty in the purification.

The resulting product is a mixture of ether intermediate and excess sulfonate or halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or organic solvents; wherein, the organic solvent is not particularly limited, as long as it can dissolve the product, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50 times that of sulfonates or halides, and preferably 1 to 10 times.

### 2.2.3.2. Alkylation reaction of substrate amines with sulfonates or halides

Under basic condition, an amine intermediate can be obtained via nucleophilic substitution of the substrate amine with a sulfonate derivative or halide. Wherein, the molar equivalent of the sulfonate or halide is 1 to 50 times that of the substrate amine, and preferably 1 to 5 times. When the molar equivalent of sulfonate or halide is less than 1 times that of the substrate amine, the substitution may not be complete, causing difficulty in the purification process. When the molar equivalent of sulfonate or halide exceeds 50 times that of the substrate amine, the excess sulfonate or halide tends to cause difficulty in the purification process, and may be mixed into the subsequent step, resulting in an increase in side reactions in the subsequent step and increasing the difficulty of purification.

The resulting product is a mixture of amine intermediate and excess sulfonate or halide, and can be purified by a purification means such as column chromatography, anion exchange resin, osmosis treatment, ultrafiltration treatment, and the like. Wherein, the anion exchange resin is not particularly limited as long as the target product can undergo ion-exchange and adsorb on the resin, preferably the ion exchange resin of a tertiary amine or quaternary ammonia salt based on dextran, agarose, polyacrylate, polystyrene, poly(diphenylethylene), or the like. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, and diisopropylethylamine), or an inorganic base (e.g., sodium carbonate, sodium hydroxide, sodium bicarbonate, sodium acetate, potassium carbonate, and potassium hydroxide), preferably an organic base, and more preferably triethylamine or pyridine. The molar amount of base is 1 to 50 times that of the sulfonate or halide, preferably 1 to 10 times, and more preferably 3 to 5 times.

### 2.2.3.3. Alkylation reaction of substrate amines with aldehyde derivatives

The substrate amine reacts with an aldehyde derivative to obtain an imine intermediate, which forms an amine intermediate under the action of reducing agents. Wherein, the molar equivalent of the aldehyde derivative is 1 to 20 times that of the substrate amine, preferably 1 to 2 times, and more preferably 1 to 1.5 times. When the molar equivalent of aldehyde exceeds 20 times that of the substrate amine, the excess reagent tends to cause difficulty in the purification process and may be mixed into the subsequent step to increase the purification difficulty. When the molar equivalent of aldehyde is less than 1 times that of the substrate amine, the substitution may not be complete, causing the purification process to be difficult. Wherein, the resulting product can be obtained after purification by means such as cation exchange resin, osmosis treatment, ultrafiltration treatment, and the like. The cation exchange resin is not particularly limited as long as it can undergo ion-exchange with quaternary ammonium cations and realize the isolation. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited as long as the imine can be reduced to an amine, preferably sodium borohydride, lithium aluminum hydride, sodium cyanoborohydride, Zn/AcOH, etc., and more preferably sodium cyanoborohydride. The molar amount of reducing agent is generally 0.5 to 50 times that of the aldehyde derivatives, and preferably 1 to 10 times.

### 3.1. Lipid Compositions

In the present application, provided herein is a lipid composition comprising any cationic lipid whose structure is represented by the general formula (1).

In a specific embodiment of the present application, the lipid composition comprises a cationic lipid whose structure is represented by general formula (1), and preferably, further comprises one or more types of lipids selected from the group consisting of phospholipid, steroid lipid and PEGylated lipid; the lipid composition is selected from any one of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a neutral lipid, a steroid lipid, and a PEGylated lipid.

In a specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-*O*-octadecenyl-sn-glycero-3-phosphatidylcholine (18:0 Diether PC), 1-oleoyl-2-cholesterylhemisuccinyl-sn-glycero-3-phosphocholine (OChemsPC), 1-*O*-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid composition is preferably selected from the group consisting of cholesterol, fecal sterol, sitosterol, ergosterol, campesterol, stigmasterol, rapeseed sterol, lycopene, ursolic acid, α-tocopherol, and combinations thereof.

In a specific embodiment of the present application, the PEGylated lipid in the lipid composition is preferably selected from the group consisting of 1,2-dimyristoyl-sn-glycerol methoxypolyethylene glycol (PEG-DMG), 1,2-distearoyl-sn-glycero-3-phosphoethanolamine-*N*-[amino(polyethylene glycol)] (PEG-DSPE), PEG-cholesterol, PEG-diacylglycamide (PEG-DAG), and PEG-dialkyloxypropyl (PEG-DAA), specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol (PEG-DMG), and combinations thereof.

In a specific embodiment of the present application, the PEGylated lipid in the lipid composition is preferably selected from the group consisting of the following structures and combinations thereof: 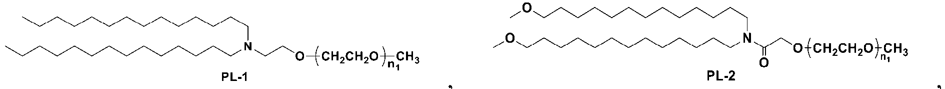 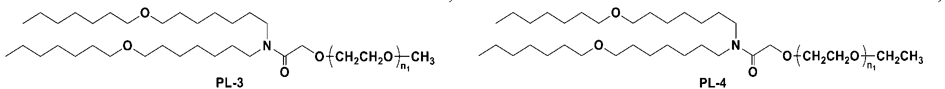 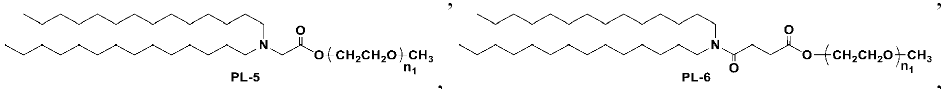 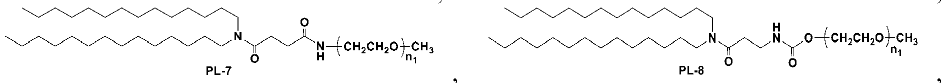 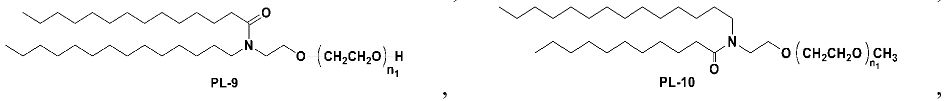 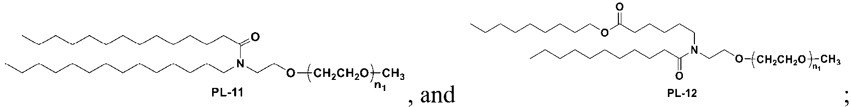 wherein, n₁ is an integer of 25 to 300, and is more preferably selected from the group consisting of 44, 45, 46, 47, and 48.

In a specific embodiment of the present application, the other cationic lipid in the lipid composition is preferably selected from the group consisting of 1,2-dioleoyl-3-trimethylammonium-propane (DOTAP), 1,2-bisoctadecoxy-3-methylammonium propane chloride (DOTMA), 1-[2-(oleoyloxy)ethyl]-2-oleo-3-(2-hydroxyethyl)imidazoline chloride (DOTIM), 1,2-diolecanol-3-dimethylamino-propane (DODMA), 2,3-bis(tetradecanoyloxy)propyltrimonium chloride (DMTAP), didecyldimonium chloride (DDAC), bisdecyldimonium bromide (DDAB), *N*,*N*-diole-*N*,*N*-dimethylammonium chloride (DODAC), *N*-(4-carboxybenzyl)-*N,N*-dimethyl-2,3-bis(oleoyloxy)propyl-1-ammonium (DOBAQ), 3,6-bis{4-[bis(2-hydroxydodecyl)amino]butyl}piperazine-2,5-dione (cKK-E12), 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol) (C12-200), 4-(*N*,*N*-dimethylamino)butyric acid(dilinole)methyl ester (DLin-MC3-DMA), 1,2-dimyristoyl-sn-glycero-3-ethylphosphocholine (EPC), ((4-hydroxybutyl) azadiyl)bis(hexane-6,1-diyl)bis(2-hexyldecanoate) (ALC-0315), 8-[(2-hydroxyethyl)(6-oxo-6-(undecoxy)hexyl)amino]caprylic acid (hepta-9-yl) ester (SM-102), and ((2-(2-hydroxyethoxy)ethyl)azadialkyl)bis(hexane-6,1-diyl)bis(2-hexyldecanoate) (DHA-1).

In a specific embodiment of the present application, the anionic lipid in the lipid composition is preferably selected from the group consisting of 1,2-dioleoyl-sn-glycerol-3-phosphate sodium salt (18:1 PA), 1,2-dimyristoyl-sn-glycerol-3-phosphate sodium salt (14:0 PA), bis(monoolein) phosphatidylmonomethylethanolamine (18:1 BMP), and cardiolipin (CL).

In a specific embodiment of the present application, the lipid composition comprises 20-80% cationic lipid represented by formula (1), 5-16% phospholipid, 25-55% steroid lipid, and 0.5-10% PEGylated lipid; the percentages refer to the molar percentages of each lipid in the total lipids in the solvent-comprising solution.

In a specific embodiment of the present application, in any of the aforementioned lipid compositions, the molar percentage of cationic lipids in the total lipids in the solvent-comprising solution is preferably 30%-65%, more preferably about 35%, 40%, 45%, 46%, 47%, 48%, 49%, 50%, or 55%.

In a specific embodiment of the present application, in any of the aforementioned lipid compositions, the molar percentage of phospholipids in the total lipids in the solvent-comprising solution is preferably about 7.5-16%, more preferably about 8%, 9%, 10%, 11%, 12%, or 16%.

In a specific embodiment of the present application, in any of the aforementioned lipid compositions, the molar percentage of steroid lipids in the total lipids in the solvent-comprising solution is preferably 35-50%, more preferably about 40%, 41%, 42%, 43%, 44%, 45%, 46%, 47%, 48%, 49%, or 50%.

In a specific embodiment of the present application, in any of the aforementioned lipid compositions, the molar percentage of PEGylated lipids in the total lipids in the solvent-comprising solution is preferably 0.5-5%, more preferably 1-3%, more preferably about 1.5%, 1.6%, 1.7%, 1.8%, 1.9%, 2.0%, or 2.5%.

### 3.2. Preparation of Lipid Compositions

In the present application, the lipid composition can be prepared by the following methods, including but not limited to ethanol injection, microfluidic method, T-tube mixing method, and periplasmic extrusion method, preferably ethanol injection and microfluidic method.

### 4. Lipid Pharmaceutical Compositions and Formulations Thereof

### 4.1. Lipid Pharmaceutical Compositions

In one embodiment of the present application, provided herein is a lipid pharmaceutical composition, which comprises any aforementioned lipid composition and a drug; wherein, the lipid composition comprises any aforementioned cationic lipid represented by formula (1), and the drug is selected from the group consisting of a nucleic acid drug, a gene vaccine, an antitumor drug, a small molecule drug, a peptide drug, and a protein drug.

In a specific embodiment of the present application, in the lipid pharmaceutical composition, the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

In a specific embodiment of the present application, the lipid pharmaceutical composition is preferably used as a drug, selected from any of the following drugs: an antineoplastic agent, an antiviral agent, an antifungal agent, and a vaccine.

In a specific embodiment of the present application, the drug in the lipid pharmaceutical composition includes, but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In a specific embodiment of the present application, the N/P ratio of the lipid composition to the nucleic acid is preferably (0.1~100): 1, more preferably (0.2~30): 1, and most preferably (0.5~20): 1.

### 4.2. Formulations of Lipid Pharmaceutical Compositions

In a specific embodiment of the present application, the drug in the lipid pharmaceutical composition is a nucleic acid drug, and the working solution in the formulation of lipid pharmaceutical composition is preferably deionized water, ultrapure water, phosphate buffer or normal brine, more preferably phosphate buffer or normal brine, and most preferably normal brine; the ratio of the lipid composition to the working solution is preferably (0.05~20) g: 100 mL, more preferably (0.1~10) g: 100 mL, and most preferably (0.2~5) g: 100 mL.

In a specific embodiment of the application, provided herein is a formulation of lipid pharmaceutical composition, which comprises the aforementioned lipid pharmaceutical composition and a pharmaceutically acceptable diluent or excipient; the diluent or excipient is preferably deionized water, ultrapure water, phosphate buffer or normal brine, more preferably phosphate buffer or normal brine, and most preferably normal brine.

In the present application, the preparation of the formulation of lipid pharmaceutical composition includes the following steps:
(1) equilibrate the lipid composition in the diluent or excipient;
(2) add the nucleic acid drug to the equilibrated mixture comprising the lipid composition and the diluent or excipient, for complexation;
wherein, the equilibration time is preferably 0.1~12 h, preferably 0.2~6 h, and more preferably 0.5~3 h; the complexation time is preferably 0.1~12 h, preferably 0.2~5 h, and more preferably 0.5~2 h.

### 5. Liposomes, Lipid Nanoparticles (LNPs) and Preparation Thereof

### 5.1. Liposomes or LNPs

In a specific embodiment of the present application, provided herein is a liposome or LNP, which comprises any aforementioned lipid pharmaceutical composition.

In a specific embodiment of the present application, the aforementioned LNP is preferably an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.

### 5.2. Preparation of Liposomes or LNPs

In a specific embodiment of the present application, liposomes can be prepared by the following methods, including but not limited to thin-film dispersion method, ultrasonic dispersion method, reverse-phase evaporation method, freeze-drying method, freeze-thaw method, double emulsion method, and injection method, preferably thin-film dispersion method, ultrasonic dispersion method and/or reverse-phase evaporation method.

In a specific embodiment of the present application, LNPs can be prepared by the following methods, including but not limited to microemulsion method, double emulsion method, high shear homogenization and ultrasonication, thin-film hydration extrusion method, and microfluidic method.

In a specific embodiment of the present application, liposomes are prepared by the thin-film dispersion method which includes the following steps:
Step (1): weigh cationic lipids, steroid lipids, neutral lipids, and PEGylated lipids, fully dissolve them in an organic solvent, shake well, remove the organic solvent by vacuum rotary evaporation to obtain an oil film, and furtherly dry with a vacuum pump to remove the organic solvent;
Step (2): add phosphate buffer with dissolved cryoprotectant, and use water-bath ultrasonication to obtain a translucent emulsion;
Step (3): add the emulsion to a high-pressure homogenizer for overpressure, and then add the over pressurized homogenized emulsion to the liposome extruder for film-passing to obtain the cationic liposome;
Step (4): optionally, dry the liposome in a freeze dryer to obtain a liposome powder.

In a specific embodiment of the present application, in the preparation methods of liposomes, the ratio of the liposome to the phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, the LNPs are preferably prepared by the method of microfluidics, vertexing, or simple mixing, and the steps are as follows:
Step (1): dissolve each lipid component in the organic solvent to obtain the lipid composition dissolved in organic phase, wherein the organic phase is preferably ethanol;
Step (2): add the nucleic acid drug to the buffer to obtain the aqueous solution, wherein the aqueous phase is preferably a citrate buffer or sodium acetate buffer;
Step (3): use a microfluidic device, a vortex or a pipette to mix the organic phase solution with the aqueous phase solution to obtain the LNP composition, and perform purification such as ultrafiltration and the like to remove the organic solvent and the free nucleic acid molecules.

The preparation method of the present application includes any protection and deprotection processes of specific groups required for the reaction. The intermediates and final products prepared in this application can be purified by methods including but not limited to extraction, recrystallization, adsorption treatment, precipitation, reverse precipitation, thin-film dialysis, supercritical extraction, and column chromatography (including gel columns, ion columns, silica columns, etc.). The structure, molecular weight, and purity of the intermediates and final products can be characterized using methods including but not limited to ¹H NMR, electrophoresis, UV-visible spectrophotometry, FTIR, AFM, GPC, HPLC, MALDI-TOF MS, and circular dichroism spectroscopy. In the present application, the structure of cationic lipid final product is characterized by NMR, and its molecular weight is confirmed by mass spectrometry; the molecular weight (defaulting to number-average molecular weight, Mn) and polydispersity index (PDI) of the polymer are determined by gel permeation chromatography (GPC), and the degree of polymerization (defaulting to number-average degree of polymerization) of the polymer is calculated based on its molecular weight. The following specific examples are further descriptions of the preparation methods of cationic lipids, lipid compositions, and formulations of lipid pharmaceutical compositions, and the biological activity assays for lipid pharmaceutical compositions. The specific examples are disclosed to further illustrate the application, but should not be regarded as a limitation of the scope of present application.

### Example-1.1: Cationic lipid (E1-1)

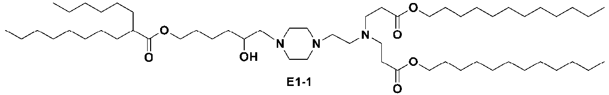

The preparation process is as follows:
Step a: 4-(2-aminoethyl)piperazine-1-carboxylate tert-butyl ester **(S1-1,** 0.69 g, 3.0 mmol) was dissolved in isopropanol, then sufficient amount of anhydrous potassium carbonate was added with stirring at room temperature until the reaction solution became alkaline. 2-dodecyl acrylate **(S1-2,** 2.16 g, 9.0 mmol) was added to the reaction solution, which was then placed under reflux (90°C) and stirred for 36 h. After completion of the reaction, the reaction solution was concentrated to obtain a crude product. The crude product was then dissolved with dichloromethane, TFA was added to 0.1 M, and the reaction was carried out for 4 h, then the reaction solution was adjusted to neutral pH and concentrated, diluted with purified water, and extracted with dichloromethane. Then the extract was dried with anhydrous magnesium sulfate, filtered, the filtrate was concentrated and recrystallized to obtain amine-deprotected intermediate **S1-3** (1.34 g).

Step b: under nitrogen atmosphere, *N*,*N*'-dicyclohexylcarbodiimide **(DCC,** 2.27 g, 11.0 mmol) was added to a round-bottom flask containing 2-hexyldecanoic acid **(S1-4,** 1.28 g, 5.0 mmol), 5-hexene-1-ol **(S1-5,** 0.6 g, 6.0 mmol) and 4-dimethylaminopyridine **(DMAP,** 0.15 g, 1.3 mmol) dissolved in dichloromethane (50 mL) and the reaction was carried out for 16 h at room temperature. After completion of the reaction, the precipitate was removed by filtration, the filtrate was concentrated, and then the crude product was purified by column chromatography to obtain **S1-6** (1.39 g).

Step c: **S1-6** (0.68 g, 2.0 mmol) was dissolved in 20 mL of dichloromethane, *m*-chloroperoxybenzoic acid **(m-CPBA,** 0.52 g, 3.0 mmol) was added under ice bath conditions, after stirring for 15 min, the ice bath was removed and the reaction was stirred overnight. After completion of the reaction, an excess of saturated sodium bisulfite solution was added, and the mixture was concentrated to remove dichloromethane. The residues were sequentially washed thrice with ethyl acetate (20 mL) and saturated sodium bicarbonate solution (20 mL), once with saturated sodium chloride solution (20 mL), and the organic phase was dried with anhydrous magnesium sulfate, filtered, the filtrate was concentrated and purified by column chromatography to obtain compound **S1-7** (0.53 g).

Step d: **S1-3** (0.49 g, 0.8 mmol) and *N*,*N*-diisopropylethylamine **(DIPEA,** 0.08 g, 0.6 mmol) were added to the methanol solution of **S1-7** (0.43 g, 1.2 mmol) sequentially, and the reaction solution was placed under reflux (90°C) for 24 h. After completion of the reaction, the reaction solution was concentrated, and the crude product was purified by column chromatography to obtain cationic lipid **E1-1** (0.56 g). ¹H NMR (400 MHz, CDCl₃) δ: 4.09-4.00 (m, 6H, -C(=O)OC*H*₂-), 3.68-3.60 (m, 1H, -C*H*(OH)-), 2.80 (t, 4H, >NC*H*₂CH₂C(=O)O-), 2.69-2.35 (m, 19H; 4H, >NCH₂C*H*₂C(=O)O-; 1H, >C*H*C(=O)O-; 4H, pip-C*H*₂CH₂-; 8H, pip-*H*; 2H, pip-CH₂C*H*₂-), 1.63-1.21 (m, 70H, -CH₂C*H*₂CH₂-, -C*H*₂CH₃), 0.87 (t, 12H, -CH₂C*H*₃). MS (ESI): m/z=963.9 ([M+H]⁺).

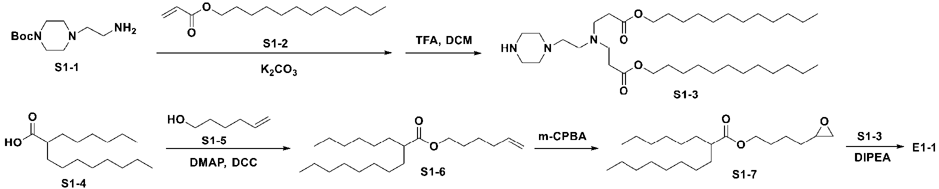

### Example-1.2: Cationic lipid (E1-2)

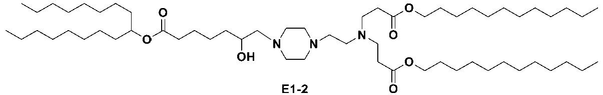

The preparation process is as follows:
Step a: under nitrogen atmosphere, **DCC** (2.27 g, 11.0 mmol) was added to a round-bottom flask containing 6-heptenoic acid **(S1-9,** 0.64 g, 5.0 mmol), 9-heptadecanol **(S1-8,** 1.54 g, 6.0 mmol) and **DMAP** (0.15 g, 1.3 mmol) dissolved in dichloromethane (50 mL) and the reaction was carried out for 16 h at room temperature. After completion of the reaction, the precipitate was removed by filtration, the filtrate was concentrated, and the crude product was purified by column chromatography to obtain **S1-10** (1.49 g).

Step b: **S1-10** (0.73 g, 2.0 mmol) was dissolved in 20 mL of dichloromethane, ***m*-CPBA** (0.52 g, 3.0 mmol) was added in ice bath conditions, after stirring for 15 min, the ice bath was removed and the reaction was stirred overnight. After completion of the reaction, an excess of saturated sodium bisulfite solution was added, and the solution was concentrated to remove dichloromethane. The residues were sequentially washed thrice with ethyl acetate (20 mL) and saturated sodium bicarbonate solution (20 mL), once with saturated sodium chloride solution (20 mL), and the organic phase was dried with anhydrous magnesium sulfate, filtered, and the filtrate was concentrated and purified by column chromatography to obtain compound **S1-11** (0.57 g).

Step c: **S1-3** (0.49 g, 0.8 mmol) and **DIPEA** (0.08 g, 0.6 mmol) were added to the methanol solution of **S1-11** (0.46 g, 1.2 mmol) sequentially, and the reaction solution was placed under reflux (90°C) and stirred for 24 h. After completion of the reaction, the reaction solution was concentrated, and the crude product was purified by column chromatography to obtain cationic lipid **E1-2** (0.58 g). ¹H NMR (400 MHz, CDCl₃) δ: 4.90-4.81 (m, 1H, *>CHOC(=O)-),* 4.09-4.00 (m, 4H, -C(=O)OC*H*₂-), 3.68-3.60 (m, 1H, -C*H*(OH)-), 2.80 (t, 4H, >NC*H*₂CH₂C(=O)O-), 2.69-2.35 (m, 18H; 2H, >CHOC(=O)C*H*₂-; 4H, >NCH₂C*H*₂C(=O)O-; 4H, pip-C*H*₂CH₂-; 8H, pip-*H*; 2H, pip-CH₂C*H*₂-), 1.63-1.21 (m, 74H, -CH₂C*H*₂CH₂-, -C*H*₂CH₃), 0.87 (t, 12H, -CH₂C*H*₃). MS (ESI): m/z=991.9 ([M+H]⁺).

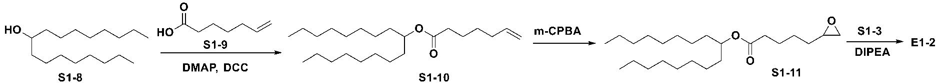

### Example-1.3: Cationic lipid (E1-3)

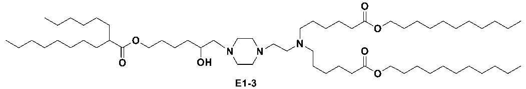

The preparation process is as follows:
Step a: under nitrogen atmosphere, **DCC** (3.17 g, 15.4 mmol) was added to a round-bottom flask containing 6-bromocaproic acid **(S1-12,** 1.37 g, 7.0 mmol), undecyl alcohol **(S1-13,** 1.44 g, 8.4 mmol), and **DMAP** (0.21 g, 1.8 mmol) dissolved in dichloromethane (30 mL), and the reaction was carried out for 16 h at room temperature. After completion of the reaction, the precipitate was removed by filtration, the filtrate was concentrated, and the crude product was purified by column chromatography to obtain **S1-14** (1.98 g).

Step b: under nitrogen atmosphere, compound **S1-1** (0.46 g, 2.0 mmol) was dissolved in acetonitrile (20 mL), and **S1-14** (1.75 g, 5.0 mmol) and **DIPEA** (0.52 g, 4.0 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and then the organic phases were combined, dried with anhydrous magnesium sulfate, filtered, then the filtrate was concentrated and purified by column chromatography to obtain compound **S1-15** (1.05 g).

Step c: **S1-15** (0.53 g, 0.8 mmol) and **DIPEA** (0.08 g, 0.6 mmol) were added to the methanol solution of **S1-7** (0.43 g, 1.2 mmol) sequentially, and the reaction solution was placed under reflux (90°C) to continue the stirring reaction for 24 h. After completion of the reaction, the reaction solution was concentrated, and the crude product was purified by column chromatography to obtain cationic lipid **E1-3** (0.59 g). ¹H NMR (400 MHz, CDCl₃) δ: 4.09-4.00 (m, 6H, -C(=O)OC*H*₂-), 3.68-3.60 (m, 1H, -CH(OH)-), 2.71-2.24 (m, 23H; 4H, >NC*H*₂CH₂-; 4H, -C*H*₂C(=O)O-; 1H, >C*H*C(=O)O-; 4H, pip-C*H*₂CH₂-; 8H, pip-*H*; 2H, pip-CH₂C*H*₂N<), 1.63-1.21 (m, 78H, -CH₂C*H*₂CH₂-, -C*H*₂CH₃), 0.87 (t, 12H, -CH₂C*H*₃). MS (ESI): m/z=1020.7 ([M+H]⁺).

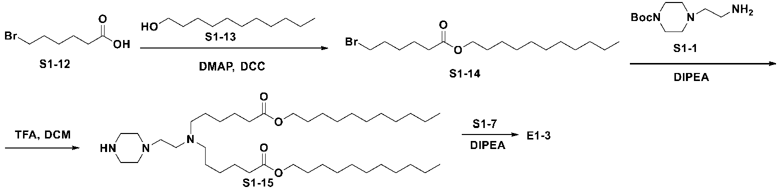

### Example-1.4: Cationic lipid (E1-4)

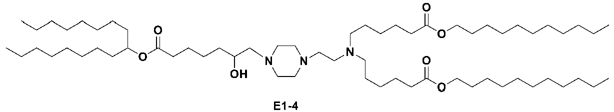

The preparation process is as follows:
**S1-15** (0.53 g, 0.8 mmol) and **DIPEA** (0.08 g, 0.6 mmol) were added to the methanol solution of **S1-11** (0.46 g, 1.2 mmol) sequentially, and the reaction solution was placed under reflux (90°C) for 24 h. After completion of the reaction, the reaction solution was concentrated, and the crude product was purified by column chromatography to obtain cationic lipid **E1-4** (0.61 g). ¹H NMR (400 MHz, CDCl₃) δ: 4.90-4.81 (m, 1H, *>CHOC(=O)-),* 4.05 (t, 4H, -C(=O)OC*H*₂-), 3.71-3.61 (m, 1H, -C*H*(OH)-), 2.76-2.24 (m, 24H; 8H, pip-H; 4H, pip-C*H*₂CH₂-; 2H, pip-CH₂C*H*₂N<; 4H, >NC*H*₂CH₂-; 6H, -C*H*₂C(=O)O-), 1.66-1.23 (m, 82H, -CH₂C*H*₂CH₂-, -C*H*₂CH₃), 0.89 (t, 12H, -CH₂C*H*₃). MS (ESI): m/z=1048.7 ([M+H]⁺).

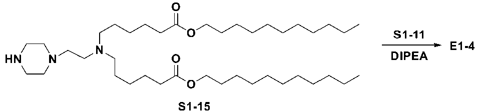

### Example-2: Cationic lipid (E2-1)

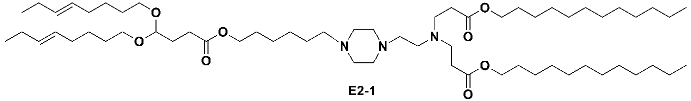

The preparation process is as follows:
Step a: 4,4-diethoxybutyronitrile **(S2-1,** 1.88 g, 12.0 mmol) and 2-octen-1-ol **(S2-2,** 4.61 g, 36.0 mmol) were added to a round-bottom flask containing pyridinium p-toluene sulfonate **(PPTS,** 0.15 g, 0.6 mmol) at room temperature, and the mixture was reacted at 105°C for 20 h. After completion of the reaction, the reaction solution was cooled to room temperature and purified by column chromatography to obtain compound **S2-3** (1.62 g).

Step b: **S2-3** (1.29 g, 4.0 mmol) was dissolved in ethanol (20 mL), then potassium hydroxide **(KOH,** 0.56 g, 10.0 mmol) was added dropwise to the mixture, and the reaction was conducted at 110°C for 24 h. After completion of the reaction, the reaction solution was cooled to room temperature, 1 N HCl was added to adjust the solution to pH=5, extracted with hexane twice (10 mL*2), the organic phases were combined and washed with water and brine, dried with anhydrous magnesium sulfate, filtered, and the filtrate was concentrated and purified by column chromatography to obtain target compound **S2-4** (1.30 g, 95%).

Step c: **DCC** (1.36 g, 6.6 mmol) was added to a round-bottom flask containing **S2-4** (1.02 g, 3.0 mmol), 6-bromo-n-hexanol **(S2-5,** 0.65 g, 3.6 mmol), and **DMAP** (0.09 g, 0.8 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain **S2-6** (1.23 g).

Step d: under nitrogen atmosphere, compound **S1-3** (0.61 g, 1.0 mmol) was dissolved in acetonitrile (20 mL), **S2-6** (0.66 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and then the organic phases were combined, dried with anhydrous magnesium sulfate, filtered, then the filtrate was concentrated and purified by column chromatography to obtain cationic lipid **E2-1** (0.84 g). ¹H NMR (400 MHz, CDCl₃) δ: 5.41-5.26 (m, 4H, *-CH=CH-),* 4.63 (t, 1H, >C*H*(OCH₂)₂-), 4.09-4.00 (m, 6H, -C(=O)OC*H*₂-), 3.52-3.36 (m, 4H, >CH(OC*H*₂)₂-), 2.80 (t, 4H, >NC*H*₂CH₂C(=O)O-), 2.69-2.36 (m, 20H; 2H, >CHCH₂C*H*₂C(=O)O-; 4H, >NCH₂C*H*₂C(=O)O-; 4H, pip-C*H*₂CH₂-; 8H, pip-*H*; 2H, pip-CH₂C*H*₂N<), 2.11-1.82 (m, 10H; 2H, -C*H*₂CH(OCH₂)₂-; 8H, -CH₂C*H*₂CH=CH-), 1.63-1.21 (m, 56H, -CH₂C*H*₂CH₂-, -C*H*₂CH₃), 0.87 (t, 12H, -CH₂C*H*₃). MS (ESI): m/z=1031.9 ([M+H]⁺).

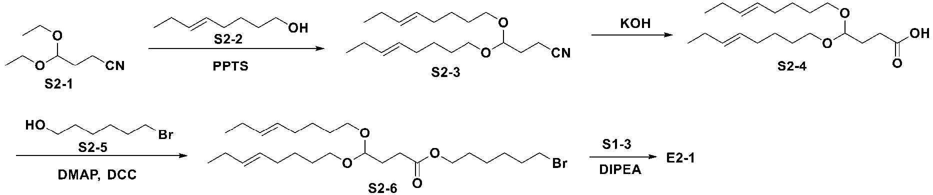

### Example-3: Cationic lipid (E3-1)

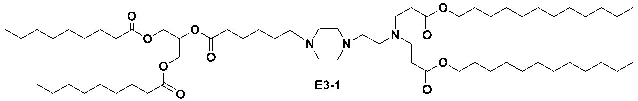

The preparation process is as follows:
Step a: Glycerol **(S3-1,** 0.37 g, 4.0 mmol) was adsorbed on the same weight of silica gel by vigorous stirring for 30 minutes, and then nonanoic acid **(S3-2,** 1.26 g, 8.0 mmol), immobilized **lipase R. miehei** (0.10 g) and **molecular sieve** (0.28 g) were added successively to the preparation, and then the mixture was suspended in diethyl ether (30 mL) and stirred for 48 h at room temperature. After completion of the reaction, the lipase and silica gel were separated by filtration, the filtrate was concentrated, and the crude product was recrystallized in methanol to obtain target compound **S3-3** (1.21 g).

Step b: under nitrogen atmosphere, **DCC** (0.91 g, 4.4 mmol) was added to a round-bottom flask containing 6-bromocaproic acid **(S1-12,** 0.39 g, 2.0 mmol), **S3-3** (0.90 g, 2.4 mmol), and **DMAP** (0.06 g, 0.6 mmol) dissolved in dichloromethane (20 mL), and the reaction was carried out for 16 h at room temperature. After completion of the reaction, the precipitate was removed by filtration, and the filtrate was concentrated, and then crude product was purified by column chromatography to obtain **S3-4** (0.93 g).

Step c: under nitrogen atmosphere, compound **S1-3** (0.61 g, 1.0 mmol) was dissolved in acetonitrile (20 mL), and **S3-4** (0.72 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and then the organic phases were combined, dried with anhydrous magnesium sulfate, filtered, then the filtrate was concentrated and purified by column chromatography to obtain cationic lipid **E3-1** (0.89 g). ¹H NMR (400 MHz, CDCl₃) δ: 5.37-5.31 (m, 1H, -C(=O)OC*H*<), 4.38-4.29 (m, 2H, >CH(C*H*₂OC(=O))₂-), 4.19-4.13 (m, 2H, >CH(C*H*₂OC(=O))₂-), 4.09-4.00 (m, 4H, -C(=O)OC*H*₂-), 2.80 (t, 4H, >NC*H*₂CH₂C(=O)O-), 2.69-2.35 (m, 24H; 4H, >CHCH₂OC(=O)C*H*₂-; 2H, >CHOC(=O)C*H*₂-; 4H, >NCH₂C*H*₂C(=O)O-; 4H, pip-C*H*₂CH₂-; 8H, pip-*H*; 2H, pip-CH₂C*H*₂N<), 1.63-1.21 (m, 70H, -CH₂C*H*₂CH₂-, -C*H*₂CH₃), 0.87 (t, 12H, -CH₂C*H*₃). MS (ESI): m/z=1077.9 ([M+H]⁺).

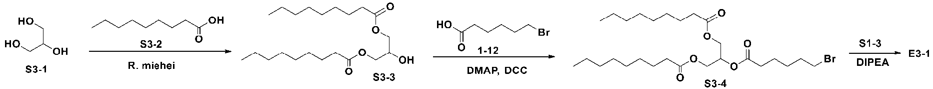

### Example-4.1: Cationic lipid (E4-1)

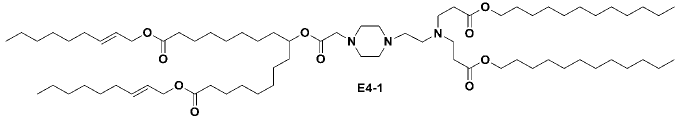

The preparation process is as follows:
Step a: newly activated **magnesium granules** (0.79 g, 33.1 mmol) and 2 mL of anhydrous ether were added to a clean round-bottom flask. Bromide 9-bromo-1-nonene **(S4-1,** 5.46 g, 26.6 mmol) was dissolved in anhydrous ether (80 mL) and transferred to a drop funnel. Under ice bath conditions, the bromide ether solution was added dropwise to the magnesium grains. After completion of the addition, the reaction solution was reacted at 35°C for 1 h and then cooled in ice bath conditions. **Ethyl formate** (0.89 g, 12.0 mmol) was dissolved in anhydrous ether (8 mL), transferred to a drop funnel, stirred and added to the reaction solution. After the reaction began to reflux, the ether solution of remaining formate was quickly added, and the reaction solution was stirred for 1 hour again at room temperature. The reaction was quenched by the addition of 5 mL of acetone and 15 mL of ice water, treated with aqueous H₂SO₄ (10% v/v, 100 mL) until the solution became homogeneous, and then allowed to stand for phase separation. The aqueous phase was extracted with ether (50 mL*2), the organic phases were combined, dried with anhydrous magnesium sulfate, filtered, and the filtrate was concentrated, and then the crude product was purified by column chromatography to obtain compound **S4-2** (3.02 g, 90%).

Step b: **S4-2** (2.80 g, 10.0 mmol) was dissolved in a mixed solution of dichloromethane (30 mL) and acetonitrile (30 mL), then ruthenium chloride (RuCl₃, 0.37 g, 1.8 mmol) was added and the mixture was cooled to 10°C, and an aqueous solution of **sodium periodate** (0.22 g, 1.0 mmol) was added dropwise, and then the resulting mixture was stirred at 10°C for 20 h. After completion of the reaction, the mixture was diluted with water, and the organic phase and the aqueous phase were separated. The organic phase was stirred with saturated brine, followed by dropwise addition of 3% sodium sulfide solution for decolorization. The organic phase and the aqueous phase were separated, the organic phase was dried with anhydrous magnesium sulfate, filtered, and the filtrate was concentrated to obtain compound **S4-3** (2.72 g).

Step c: under nitrogen atmosphere, ***tert*-butyldimethylchlorosilane** (1.16 g, 7.7 mmol) was added to a round-bottom flask containing **S4-3** (2.21 g, 7.0 mmol) and **imidazole** (1.19 g, 17.5 mmol) dissolved in DMF (50 mL), and the reaction solution was stirred overnight at 50°C. After completion of the reaction, the solution was cooled to room temperature, the reaction solution was diluted with water, and extracted three times with ethyl acetate. The organic phases were combined, washed once with saturated brine, dried with magnesium sulfate, filtered, and the filtrate was concentrated. The residue was purified by column chromatography to obtain a diacid that the hydroxyl group protected by TBS **(S4-4,** 2.72 g, 90%).

Step d: **DCC** (1.85 g, 9.0 mmol) was added to a round-bottom flask containing 2-nonen-1-ol (S4-5, 0.71 g, 5.0 mmol), **S4-4** (0.86 g, 2.0 mmol), and **DMAP** (0.12 g, 1.0 mmol) dissolved in dichloromethane (30 mL) for 16 h at room temperature. After completion of the reaction, the precipitate was removed by filtration and the filtrate was concentrated. The residue was dissolved with tetrahydrofuran (10 mL), and then 10 mL of TBAF tetrahydrofuran solution (1 M) was added to the reaction overnight to remove TBS protection. After completion of the reaction, the mixture was concentrated and extracted, and the organic phases were combined, dried with anhydrous magnesium sulfate, filtered, and the filtrate was concentrated to obtain S4-6 crude product, which was further purified by column chromatography to obtain compound **S4-6** (0.86 g).

Step e: **S4-7** (0.75 g, 4.0 mmol) was dissolved in isopropanol, then sufficient amount of anhydrous potassium carbonate was added with stirring at room temperature until the reaction solution became alkaline. **S1-2** (2.88 g, 12.0 mmol) was added to the reaction solution, which was then placed under reflux (90°C) and stirred for 36 h. After completion of the reaction, the reaction solution was concentrated to obtain a crude product, which was then purified by column chromatography to obtain compound **S4-8** (1.95 g).

Step f: under nitrogen atmosphere, **DCC** (0.45 g, 2.2 mmol) was added to a round-bottom flask containing **S4-8** (0.67 g, 1.0 mmol), **S4-6** (0.68 g, 1.2 mmol), and **DMAP** (0.03 g, 0.3 mmol) dissolved in dichloromethane (20 mL) for 16 h at room temperature. After completion of the reaction, the precipitate was removed by filtration, the filtrate was concentrated, and the crude product was purified by column chromatography to obtain the cationic lipid **E4-1** (1.01 g). ¹H NMR (400 MHz, CDCl₃) δ: 5.82-5.71 (m, 2H, -C(=O)OCH₂C*H*=CH-), 5.56-5.54 (m, 2H, -C(=O)OCH₂CH=C*H*-), 4.93-4.87 (m, 1H, *-C(=O)OCH<),* 4.55 (t, 4H, -C(=O)OC*H*₂CH=CH-), 4.04 (t, 4H, >N(CH₂)₂C(=O)OC*H*₂-), 3.16 (s, 2H, pip-C*H*₂C(=O)O-), 2.80 (t, 4H, >NC*H*₂CH₂C(=O)O-), 2.63-2.38 (m, 16H; 8H, pip-H; 2H, pip-C*H*₂CH₂-; 2H, pip-CH₂C*H*₂-; 4H, >NCH₂C*H*₂C(=O)O-), 2.18-2.09 (m, 4H, -OC(=O)C*H*₂(CH₂)₆-), 2.06-1.99 (m, 4H, -CH=CHC*H*₂CH₂-), 1.64-1.22 (m, 80H, -CH₂C*H*₂CH₂-, -C*H*₂CH₃), 0.88 (t, 12H, -CH₂C*H*₃). MS (ESI): m/z=1214.0 ([M+H]⁺).

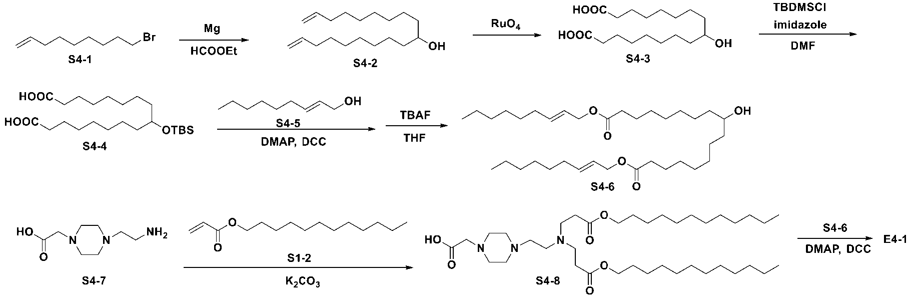

### Example-4.2: Cationic lipid (E4-2)

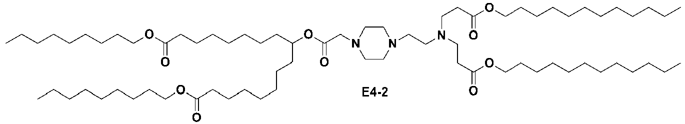

The preparation process is as follows:
Step a: **DCC** (1.85 g, 9.0 mmol) was added to a round-bottom flask containing 1-nonanol (S4-9, 0.72 g, 5.0 mmol), **S4-4** (0.86 g, 2.0 mmol), and **DMAP** (0.12 g, 1.0 mmol) dissolved in dichloromethane (30 mL), and the reaction was carried out for 16 h at room temperature. After completion of the reaction, the precipitate was removed by filtration and the filtrate was concentrated. The residue was dissolved with tetrahydrofuran (10 mL), and then 10 mL of TBAF tetrahydrofuran solution (1 M) was added to the reaction overnight to remove TBS protection. After completion of the reaction, the mixture was concentrated, extracted, and the organic phases were combined, dried with anhydrous magnesium sulfate, filtered, and the filtrate was concentrated to obtain crude S4-10, which was further purified by column chromatography to obtain compound **S4-10** (0.88 g).

Step b: **DCC** (0.45 g, 2.2 mmol) was added to a round-bottom flask containing **S4-8** (0.67 g, 1.0 mmol), **S4-10** (0.68 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain cationic lipid **E4-2** (1.02 g). ¹H NMR (400 MHz, CDCl₃) δ: 4.93-4.87 (m, 1H, *-C(=O)OCH<),* 4.04 (t, 8H; 4H, >N(CH₂)₂C(=O)OC*H*₂-; 4H, -C(=O)OC*H*₂(CH₂)₇-), 3.16 (s, 2H, pip-C*H*₂C(=O)O-), 2.80 (t, 4H, >NC*H*₂CH₂C(=O)O-), 2.63-2.38 (m, 16H; 8H, pip-H; 2H, pip-C*H*₂CH₂-; 2H, pip-CH₂C*H*₂-; 4H, >NCH₂C*H*₂C(=O)O-), 2.29 (t, 4H, -(CH₂)₆C*H*₂C(=O)O-), 1.84-1.76 (m, 8H, -C(=O)OCH₂C*H*₂-), 1.64-1.22 (m, 76H, -CH₂C*H*₂CH₂-, -C*H*₂CH₃), 0.86 (t, 12H, -CH₂C*H*₃). MS (ESI): m/z=1218.1 ([M+H]⁺).

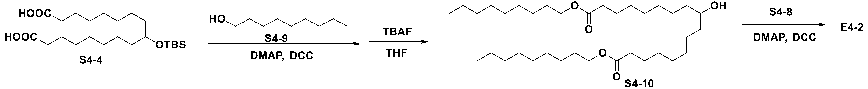

### Example-5: Cationic lipid (E5-1)

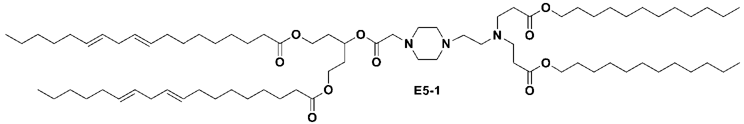

The preparation process is as follows:
Step a: **DCC** (1.85 g, 9.0 mmol) was added to a round-bottom flask containing **S5-2** (0.47 g, 2.0 mmol), linoleic acid **(S5-1,** 1.40 g, 5.0 mmol), and **DMAP** (0.12 g, 1.0 mmol) dissolved in dichloromethane (30 mL), and the reaction was carried out for 16 h at room temperature. After completion of the reaction, the precipitate was removed by filtration and the filtrate was concentrated. The residue was dissolved with tetrahydrofuran (10 mL), and then 10 mL of TBAF tetrahydrofuran solution (1 M) was added to the reaction overnight to remove TBS protection. After completion of the reaction, the mixture was concentrated, extracted, and the organic phases were combined, dried with anhydrous magnesium sulfate, filtered, and the filtrate was concentrated to obtain crude S5-3, which was further purified by column chromatography to obtain compound **S5-3** (1.00 g).

Step b: **DCC** (0.45 g, 2.2 mmol) was added to a round-bottom flask containing **S4-8** (0.67 g, 1.0 mmol), **S5-3** (0.77 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E5-1** (1.09 g). ¹H NMR (400 MHz, CDCl₃) δ: 5.46-5.29 (m, 8H, *-CH=CH-),* 5.17-5.08 (m, 1H, -C(=O)OC*H*<), 4.19-4.01 (m, 8H, -C(=O)OC*H*₂-), 3.19 (s, 2H, pip-C*H*₂C(=O)O-), 2.87-2.74 (m, 8H; 4H, -CH=CHC*H*₂CH=CH-; 4H, >NC*H*₂CH₂C(=O)O-), 2.73-2.35 (m, 16H; 8H, pip-H; 2H, pip-C*H*₂CH₂-; 2H, pip-CH₂C*H*₂-; 4H, >NCH₂C*H*₂C(=O)O-), 2.29 (t, 4H; >CH(CH₂)₂OC(=O)C*H*₂-), 2.10-1.99 (m, 8H, -CH=CHC*H*₂-), 1.97-1.89 (m, 4H, >CHC*H*₂-), 1.66-1.22 (m, 72H, -CH₂C*H*₂CH₂-, -C*H*₂CH₃), 0.88 (t, 12H, -CH₂C*H*₃). MS (ESI): m/z=1294.8 ([M+H]⁺).

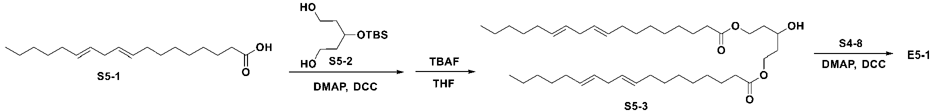

### Example-6: Cationic lipid (E6-1)

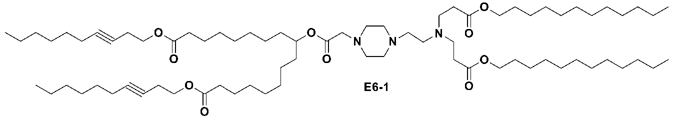

The preparation process is as follows:
Step a: **DCC** (1.85 g, 9.0 mmol) was added to a round-bottom flask containing **S4-4** (0.86 g, 2.0 mmol), 3-decyn-1-ol **(S6-1,** 0.72 g, 5.0 mmol), and **DMAP** (0.12 g, 1.0 mmol) dissolved in dichloromethane (30 mL), and the reaction was carried out for 16 h at room temperature. After completion of the reaction, the precipitate was removed by filtration and the filtrate was concentrated. The residue was dissolved with tetrahydrofuran (10 mL), and then 10 mL of TBAF tetrahydrofuran solution (1 M) was added to the reaction overnight to remove TBS protection. After completion of the reaction, the mixture was concentrated, extracted, and the organic phases were combined, dried with anhydrous magnesium sulfate, filtered, and the filtrate was concentrated to obtain crude S6-2, which was further purified by column chromatography to obtain compound **S6-2** (0.90 g).

Step b: **DCC** (0.45 g, 2.2 mmol) was added to a round-bottom flask containing **S4-8** (0.67 g, 1.0 mmol), **S6-2** (0.71 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain cationic lipid **E6-1** (1.03 g). ¹H NMR (400 MHz, CDCl₃) δ: 4.93-4.87 (m, 1H, *-C(=O)OCH<),* 4.12 (t, 4H, -C≡CCH₂C*H*₂OC(=O)-), 4.04 (t, 4H, >NCH₂CH₂C(=O)OC*H*₂-), 3.16 (s, 2H, pip-C*H*₂C(=O)O-), 2.80 (t, 4H, >NC*H*₂CH₂C(=O)O-), 2.63-2.38 (m, 20H; 8H, pip-*H*; 2H, pip-C*H*₂CH₂-; 2H, pip-CH₂C*H*₂-; 4H, >NCH₂C*H*₂C(=O)O-; 4H, -C≡CCH₂C*H*₂OC(=O)-), 2.30 (t, 4H, -(CH₂)₄C*H*₂C≡C-), 2.15-2.09 (m, 4H, -OC(=O)C*H*₂(CH₂)₆-), 1.64-1.23 (m, 80H, -CH₂C*H*₂CH₂-, -C*H*₂CH₃), 0.88 (t, 12H, -CH₂C*H*₃). MS (ESI): m/z=1239.0 ([M+H]⁺).

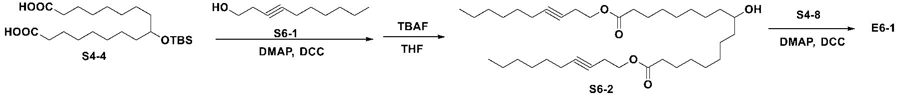

### Example-7: Cationic lipid (E7-1)

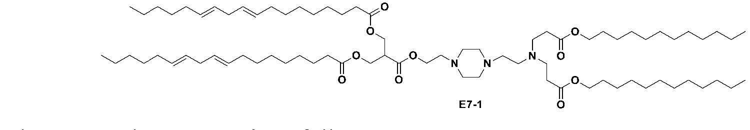

The preparation process is as follows:
Step a: 2-(4-(2-aminoethyl)piperazine-1-yl)ethanol **(S7-1,** 0.52 g, 3.0 mmol) was dissolved in isopropanol, then sufficient amount of anhydrous potassium carbonate was added with stirring at room temperature until the reaction solution became alkaline. **S1-2** (2.16 g, 9.0 mmol) was added to the reaction solution, which was then placed under reflux (90°C) and stirred for 36 h. After completion of the reaction, the reaction solution was concentrated to obtain a crude product, and the crude product was purified by column chromatography to obtain compound **S7-2** (1.46 g).

Step b: under ice bath conditions, **S7-3** (0.60 g, 5.0 mmol) was dissolved in dichloromethane, then **DCC** (1.13 g, 5.5 mmol) and **DMAP** (0.31 g, 2.5 mmol) were added, after stirring the mixture for 10 min, *tert*-butanol **(*t*-BuOH,** 0.66 g, 7.5 mmol) was slowly added, and the reaction was stirred overnight at room temperature. After completion of the reaction, the reaction solution was filtered through diatomaceous earth, washed three times with ether, washed once with saturated brine, then the organic phases were combined, dried with anhydrous magnesium sulfate, filtered, and the filtrate was concentrated, and then S7-4 crude product was obtained, which was further purified by column chromatography to obtain **S7-4** (0.80 g, 90.8%).

Step c: **DCC** (1.85 g, 9.0 mmol) was added to a round-bottom flask containing **S7-4** (0.35 g, 2.0 mmol), **S5-1** (1.40 g, 5.0 mmol), and **DMAP** (0.12 g, 1.0 mmol) dissolved in dichloromethane (30 mL), and the reaction was carried out for 16 h at room temperature. After completion of the reaction, the precipitate was removed by filtration and the filtrate was concentrated to obtain a crude product. Trifluoroacetic acid/dichloromethane (1:2, v/v) solution was prepared in a dry and clean round-bottom flask, and the dichloromethane solution of the above crude product was slowly added dropwise under ice bath conditions, and then the reaction was carried out at room temperature for 2 h. After completion of the reaction, the reaction solution was concentrated, diluted with purified water, extracted with dichloromethane. The extract was dried with anhydrous magnesium sulfate, filtered, the filtrate was concentrated and recrystallized to obtain compound **S7-5** (1.03 g).

Step d: **DCC** (0.45 g, 2.2 mmol) was added to a round-bottom flask containing **S7-5** (0.65 g, 1.0 mmol), **S7-2** (0.78 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E7-1** (1.07 g). ¹H NMR (400 MHz, CDCl₃) δ: 5.45-5.25 (m, 8H, *-CH=CH-),* 4.39-4.29 (m, 4H, >CH(C*H*₂OC(=O)₂-), 4.24 (t, 2H, pip-CH₂C*H*₂OC(=O)-), 4.05 (t, 4H, -C(=O)OC*H*₂-), 3.02 (q, 1H, >C*H*C(=O)OCH₂-), 2.80 (t, 4H, >NC*H*₂CH₂C(=O)O-), 2.63-2.38 (m, 22H; 8H, pip-H; 4H, pip-C*H*₂CH₂-; 2H, pip-CH₂C*H*₂N<; 4H, >NCH₂C*H*₂C(=O)O-; 4H, -CH=CHC*H*₂CH=CH-), 2.29 (t, 4H, -C*H*₂C(=O)OCH₂CH<), 2.03-1.97 (m, 8H, -CH=CHC*H*₂-), 1.65-1.23 (m, 72H, -CH₂C*H*₂CH₂-, -C*H*₂CH₃), 0.86 (t, 12H, -CH₂C*H*₃). MS (ESI): m/z=1280.1 ([M+H]⁺).

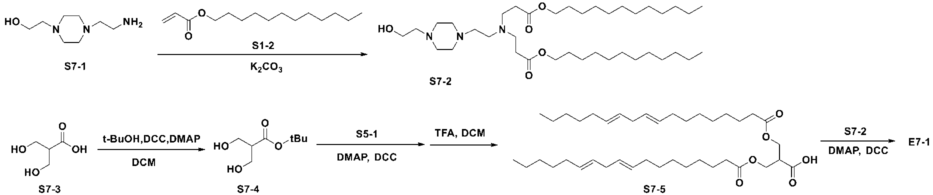

### Example-8: Cationic lipid (E8-1)

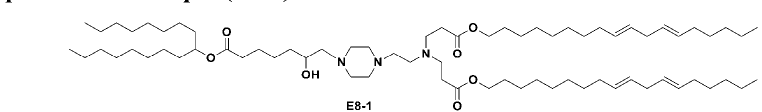

The preparation process is as follows:
Step a: (9Z,12Z)-9,12-octadecadiene-1-ol **(S8-1,** 2.93 g, 11.0 mmol) and **triethylamine** (2.59 g, 31.4 mmol) were added to dichloromethane (30 mL) at 0°C, followed by a dichloromethane (15 mL) solution containing acryloyl chloride **(S8-2,** 1.50 g, 16.5 mmol) was added dropwise to the reaction system, and the reaction solution was stirred at 20°C for 2 h. After completion of the reaction, the precipitate was removed by filtration, and the filtrate was washed with water and 5% hydrochloric acid in turn, and the organic phase was dried with magnesium sulfate. The filtrate was filtered, the filtrate was concentrated, and the residue was purified by column chromatography to obtain the target compound (9Z,12Z)-9,12-diene octadecyl acrylate **(S8-3,** 2.65 g).

Step b: **S1-1** (0.57 g, 2.5 mmol) was dissolved in isopropanol, then sufficient amount of anhydrous potassium carbonate was added with stirring at room temperature until the reaction solution became alkaline. **S8-3** (2.41 g, 7.5 mmol) was added to the reaction solution, which was then placed under reflux (90°C) and stirred for 36 h. After completion of the reaction, the solvent was concentrated to remove the crude product. The crude product was dissolved in dichloromethane, and TFA was added to 0.1 M, the reaction was carried out for 4 h, the reaction solution was adjusted to neutral pH, concentrated, diluted with purified water and then extracted with dichloromethane. The extract was dried with anhydrous magnesium sulfate, filtered, and the filtrate was concentrated, and then recrystallized to obtain intermediate **S8-4** (1.41 g).

Step c: **S8-4** (0.77 g, 1.0 mmol) and **DIPEA** (0.10 g, 0.8 mmol) were added to the methanol solution of **S1-11** (0.57 g, 1.5 mmol) sequentially, and the reaction solution was placed under reflux (90°C) to continue the stirring reaction for 24 h. After completion of the reaction, the reaction solution was concentrated, and the crude product was purified by column chromatography to obtain the cationic lipid **E8-1** (0.84 g). ¹H NMR (400 MHz, CDCl₃) δ: 5.45-5.25 (m, 8H, *-CH=CH-),* 4.90-4.81 (m, 1H, *>*C*H*OC(=O)-), 4.05 (t, 4H, -C(=O)OC*H*₂-), 3.71-3.61 (m, 1H, -C*H*(OH)-), 2.84-2.24 (m, 28H; 8H, pip-*H*; 4H, pip-C*H*₂CH₂-; 2H, pip-CH₂C*H*₂-; 4H, >NC*H*₂CH₂-; 6H, -C*H*₂C(=O)O-; 4H, -CH=CHC*H*₂CH=CH-), 2.03-1.97 (m, 8H, -CH=CHC*H*₂-), 1.84-1.76 (m, 4H, -C(=O)OCH₂C*H*₂-), 1.65-1.23 (m, 66H, -CH₂C*H*₂CH₂-, -C*H*₂CH₃), 0.89 (t, 12H, -CH₂C*H*₃). MS (ESI): m/z=1152.0 ([M+H]⁺).

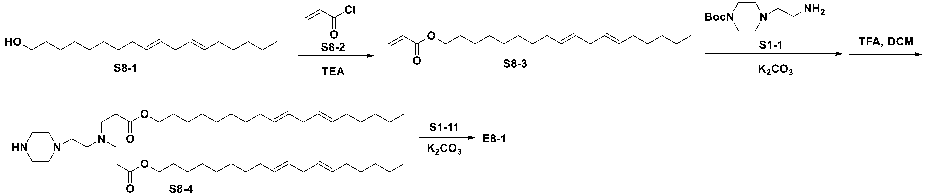

### Example-9: Cationic lipid (E9-1)

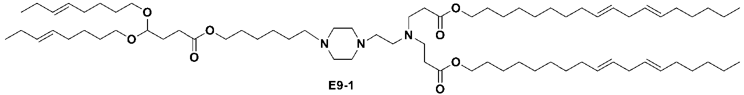

The preparation process is as follows:
Under nitrogen atmosphere, compound **S8-4** (0.77 g, 1.0 mmol) dissolved in acetonitrile (20 mL), **S2-6** (0.66 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution. The organic phases were combined, dried with anhydrous magnesium sulfate, filtered, then the filtrate was concentrated and purified by column chromatography to obtain cationic lipid **E9-1** (0.99 g). ¹H NMR (400 MHz, CDCl₃) δ: 5.45-5.25 (m, 12H, *-CH=CH-),* 4.63 (t, 1H, >C*H*(OCH₂)₂-), 4.09-4.00 (m, 6H, -C(=O)OC*H*₂-), 3.52-3.36 (m, 4H, >CH(OC*H*₂)₂-), 2.84-2.24 (m, 28H; 8H, pip-H; 4H, pip-C*H*₂CH₂-; 2H, pip-CH₂C*H*₂N<; 4H, >NC*H*₂CH₂-; 6H, -C*H*₂C(=O)O-; 4H, -CH=CHC*H*₂CH=CH-), 2.11-1.76 (m, 24H; 2H, -C*H*₂CH(OCH₂)₂-; 16H, -CH₂C*H*₂CH=CH-; 6H, -C(=O)OCH₂C*H*₂-), 1.65-1.23 (m, 46H, -CH₂C*H*₂CH₂-, -C*H*₂CH₃), 0.86 (t, 12H, -CH₂C*H*₃). MS (ESI): m/z=1192.0 ([M+H]⁺).

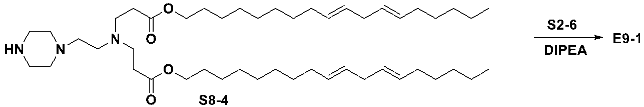

### Example-10: Cationic lipid (E10-1)

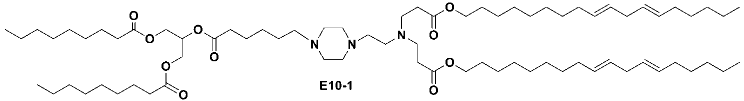

The preparation process is as follows:
Under nitrogen atmosphere, compound **S8-4** (0.77 g, 1.0 mmol) was dissolved in acetonitrile (20 mL), **S3-4** (0.72 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution. The organic phases were combined, dried with anhydrous magnesium sulfate, filtered, then the filtrate was concentrated and purified by column chromatography to obtain cationic lipid **E10-1** (1.03 g). ¹H NMR (400 MHz, CDCl₃) δ: 5.45-5.25 (m, 9H; 8H, *-CH=*C*H-;* 1H, *-C(=O)OCH<),* 4.38-4.29 (m, 2H, >CH(C*H*₂OC(=O))₂-), 4.19-4.13 (m, 2H, >CH(C*H*₂OC(=O))₂-), 4.09-4.00 (m, 4H, -C(=O)OC*H*₂-), 2.80 (t, 4H, >NC*H*₂CH₂C(=O)O-), 2.69-2.35 (m, 28H; 4H, >CHCH₂OC(=O)C*H*₂-; 2H, >CHOC(=O)C*H*₂-; 4H, >NCH₂C*H*₂C(=O)O-; 4H, -CH=CHC*H*₂CH=CH-; 4H, pip-C*H*₂CH₂-; 8H, pip-H; 2H, pip-CH₂C*H*₂N<), 2.03-1.97 (m, 8H, -CH=CHC*H*₂-), 1.84-1.76 (m, 4H, -C(=O)OCH₂C*H*₂-), 1.63-1.21 (m, 62H, -CH₂C*H*₂CH₂-, -C*H*₂CH₃), 0.87 (t, 12H, -CH₂C*H*₃). MS (ESI): m/z=1238.0 ([M+H]⁺).

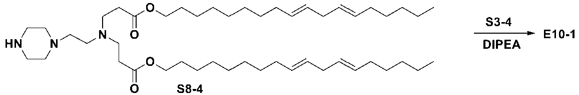

### Example-11: Cationic lipid (E11-1)

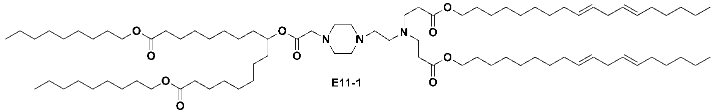

The preparation process is as follows:
Step a: **S4-7** (0.56 g, 3.0 mmol) was dissolved in isopropanol, then sufficient amount of anhydrous potassium carbonate was added with stirring at room temperature until the reaction solution became alkaline. **S8-3** (2.89 g, 9.0 mmol) was added to the reaction solution, which was placed under reflux (90°C) and stirred for 36 h. After completion of the reaction, the reaction solution was concentrated to obtain a crude product, which was then purified by column chromatography to obtain compound **S11-1** (1.87 g).

Step b: under nitrogen atmosphere, **DCC** (0.45 g, 2.2 mmol) was added to a round-bottom flask containing **S11-1** (0.83 g, 1.0 mmol), **S4-10** (0.68 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E11-1** (1.14 g). ¹H NMR (400 MHz, CDCl₃) δ: 5.45-5.25 (m, 8H, *-CH=CH-),* 4.90-4.81 (m, 1H, *>CHOC(=O)-),* 4.05 (t, 8H, -C(=O)OC*H*₂-), 3.16 (s, 2H, pip-C*H*₂C(=O)O-), 2.84-2.24 (m, 28H; 8H, pip-*H*; 2H, pip-C*H*₂CH₂-; 2H, pip-CH₂C*H*₂-; 4H, >NC*H*₂CH₂-; 8H, -C*H*₂C(=O)O-; 4H, -CH=CHC*H*₂CH=CH-), 2.03-1.97 (m, 8H, -CH=CHC*H*₂-), 1.84-1.76 (m, 8H, -C(=O)OCH₂C*H*₂-), 1.64-1.23 (m, 80H, -CH₂C*H*₂CH₂-, -C*H*₂CH₃), 0.89 (t, 12H, -CH₂C*H*₃). MS (ESI): m/z=1378.2 ([M+H]⁺).

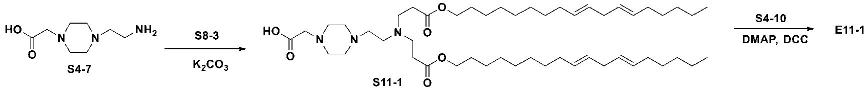

### Example-12: Cationic lipid (E12-1)

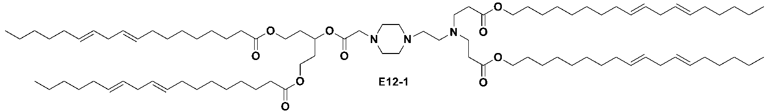

The preparation process is as follows:
Under nitrogen atmosphere, **DCC** (0.45 g, 2.2 mmol) was added to a round-bottom flask containing **S11-1** (0.83 g, 1.0 mmol), **S5-3** (0.77 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E12-1** (1.20 g). ¹H NMR (400 MHz, CDCl₃) δ: 5.46-5.29 (m, 16H, *-CH=CH-),* 5.17-5.08 (m, 1H, *-C(=O)OCH<),* 4.19-4.00 (m, 8H, -C(=O)OC*H*₂-), 3.19 (s, 2H, pip-C*H*₂C(=O)O-), 2.87-2.74 (m, 12H; 8H, -CH=CHC*H*₂CH=CH-; 4H, >NC*H*₂CH₂C(=O)O-), 2.73-2.35 (m, 16H; 8H, pip-H; 2H, pip-C*H*₂CH₂-; 2H, pip-CH₂C*H*₂-; 4H, >NCH₂C*H*₂C(=O)O-), 2.29 (t, 4H; >CH(CH₂)₂OC(=O)C*H*₂-), 2.10-1.99 (m, 16H, -CH=CHC*H*₂-), 1.97-1.89 (m, 4H, >CHC*H*₂-), 1.66-1.22 (m, 68H, -CH₂C*H*₂CH₂-, -C*H*₂CH₃), 0.88 (t, 12H, -CH₂C*H*₃). MS (ESI): m/z=1454.2 ([M+H]⁺).

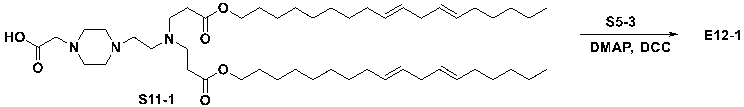

### Example-13: Cationic lipid (E13-1)

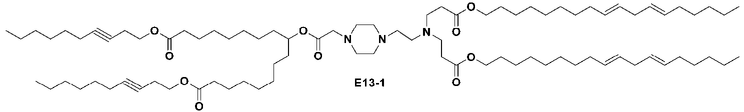

The preparation process is as follows:
Under nitrogen atmosphere, **DCC** (0.45 g, 2.2 mmol) was added to a round-bottom flask containing **S11-1** (0.83 g, 1.0 mmol), **S6-2** (0.71 g, 1.2 mmol) and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E13-1** (1.16 g). ¹H NMR (400 MHz, CDCl₃) δ: 5.46-5.29 (m, 8H, *-CH=CH-),* 4.93-4.87 (m, 1H, -C(=O)OCH<), 4.12 (t, 4H, -C=CCH₂C*H*₂OC(=O)-), 4.04 (t, 4H, >NCH₂CH₂C(=O)OC*H*₂-), 3.16 (s, 2H, pip-C*H*₂C(=O)O-), 2.87-2.74 (m, 8H; 4H, -CH=CHC*H*₂CH=CH-; 4H, >NC*H*₂CH₂C(=O)O-), 2.63-2.38 (m, 20H; 8H, pip-H; 2H, pip-C*H*₂CH₂-; 2H, pip-CH₂C*H*₂-; 4H, >NCH₂C*H*₂C(=O)O-; 4H, -C≡CCH₂C*H*₂OC(=O)-), 2.30 (t, 4H, -(CH₂)₄C*H*₂C≡C-), 2.18-1.99 (m, 12H; 8H, -CH=CHC*H*₂-; 4H, -OC(=O)C*H*₂(CH₂)₆-), 1.65-1.23 (m, 76H, -CH₂C*H*₂CH₂-, -C*H*₂CH₃), 0.86 (t, 12H, -CH₂C*H*₃). MS (ESI): m/z=1398.1 ([M+H]⁺).

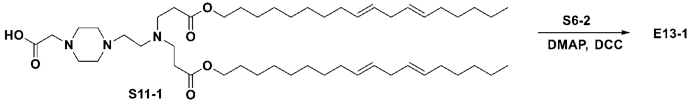

### Example-14: Cationic lipid (E14-1)

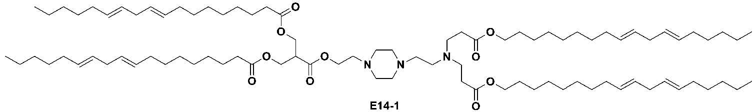

The preparation process is as follows:
Step a: **S7-1** (0.52 g, 3.0 mmol) was dissolved in isopropanol, then sufficient amount of anhydrous potassium carbonate was added with stirring at room temperature until the reaction solution became alkaline. **S8-3** (2.89 g, 9.0 mmol) was added to the reaction solution, which was placed under reflux (90°C) and stirred for 36 h. After completion of the reaction, the reaction solution was concentrated to obtain a crude product, which was purified by column chromatography to obtain compound **S14-1** (1.83 g).

Step b: under nitrogen atmosphere, **DCC** (0.45 g, 2.2 mmol) was added to a round-bottom flask containing **S7-5** (0.65 g, 1.0 mmol), **S14-1** (0.98 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E14-1** (1.18 g). ¹H NMR (400 MHz, CDCl₃) δ: 5.45-5.25 (m, 16H, *-CH=CH-),* 4.39-4.29 (m, 4H, >CH(C*H*₂OC(=O)₂-), 4.24 (t, 2H, pip-CH₂C*H*₂OC(=O)-), 4.05 (t, 4H, -C(=O)OC*H*₂-), 3.02 (q, 1H, >C*H*C(=O)OCH₂-), 2.80 (t, 4H, >NC*H*₂CH₂C(=O)O-), 2.63-2.38 (m, 26H; 8H, pip-*H*; 4H, pip-C*H*₂CH₂-; 2H, pip-CH₂C*H*₂N<; 4H, >NCH₂C*H*₂C(=O)O-; 8H, -CH=CHC*H*₂CH=CH-), 2.29 (t, 4H, -C*H*₂C(=O)OCH₂CH<), 2.03-1.97 (m, 16H, -CH=CHC*H*₂-), 1.65-1.23 (m, 68H, -CH₂C*H*₂CH₂-, -C*H*₂CH₃), 0.90 (t, 12H, -CH₂C*H*₃). MS (ESI): m/z=1440.2 ([M+H]⁺).

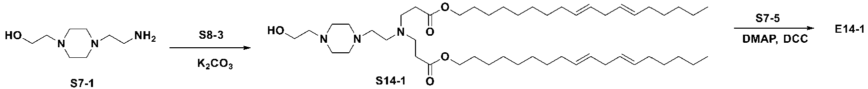

### Example-15: Cationic lipid (E15-1)

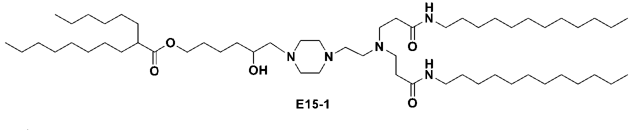

The preparation process is as follows:
Step a: **S1-1** (0.57 g, 2.5 mmol) was dissolved in isopropanol, then sufficient amount of anhydrous potassium carbonate was added with stirring at room temperature until the reaction solution became alkaline. N-dodecylacrylamide **(S15-1,** 1.79 g, 7.5 mmol) was added to the reaction solution, which was placed under reflux (90°C) and stirred for 36 h. After completion of the reaction, the reaction solution was concentrated to obtain a crude product. The crude product was then dissolved with dichloromethane, TFA was added to 0.1 M, the reaction was carried out for 4 h, the reaction solution was adjusted to neutral pH and concentrated, diluted with purified water, and extracted with dichloromethane. The extract was dried with anhydrous magnesium sulfate, filtered, the filtrate was concentrated and recrystallized to obtain amine-deprotected intermediate **S15-2** (1.11 g).

Step b: **S15-2** (0.61 g, 1.0 mmol) and **DIPEA** (0.10 g, 0.8 mmol) were added to the methanol solution of **S1-7** (0.53 g, 1.5 mmol) sequentially, and the reaction solution was placed under reflux (90°C) to continue stirring for 24 h. After completion of the reaction, the reaction solution was concentrated, and the crude product was purified by column chromatography to obtain the cationic lipid **E15-1** (0.70 g). ¹H NMR (400 MHz, CDCl₃) δ: 4.09-4.00 (m, 2H, -C(=O)OC*H*₂-), 3.68-3.60 (m, 1H, -C*H*(OH)-), 3.28 (dd, 4H, -C(=O)NHC*H*₂-), 2.82-2.35 (m, 23H; 4H, >NCH₂C*H*₂C(=O)NH-; 1H, >C*H*C(=O)O-; 4H, pip-C*H*₂CH₂-; 8H, pip-*H*; 2H, pip-CH₂C*H*₂-; 4H, >NC*H*₂CH₂-), 1.63-1.21 (m, 70H, -CH₂C*H*₂CH₂-, -C*H*₂CH₃), 0.87 (t, 12H, -CH₂C*H*₃). MS (ESI): m/z=961.9 ([M+H]⁺).

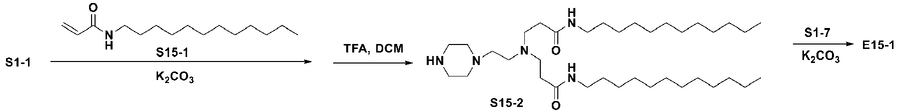

### Example-16: Cationic lipid (E16-1)

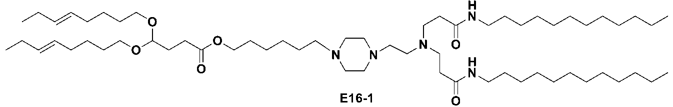

The preparation process is as follows:
Under nitrogen atmosphere, compound **S15-2** (0.61 g, 1.0 mmol) was dissolved in acetonitrile (20 mL), then **S2-6** (0.66 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and then the organic phases were combined, dried with anhydrous magnesium sulfate, filtered, then the filtrate was concentrated and purified by column chromatography to obtain cationic lipid **E16-1** (0.85 g). ¹H NMR (400 MHz, CDCl₃) δ: 5.41-5.26 (m, 4H, -C*H*=C*H*-), 4.63 (t, 1H, >C*H*(OCH₂)₂-), 4.09-4.00 (m, 2H, -C(=O)OC*H*₂-), 3.52-3.36 (m, 4H, >CH(OC*H*₂)₂-), 3.28 (dd, 4H, -C(=O)NHC*H*₂-), 2.80-2.35 (m, 24H; 2H, >CHCH₂C*H*₂C(=O)O-; 4H, pip-C*H*₂CH₂-; 8H, pip-*H*; 2H, pip-CH₂C*H*₂N<; 4H, >NC*H*₂CH₂-; 4H, >NCH₂C*H*₂C(=O)NH-), 2.11-1.82 (m, 10H; 2H, -C*H*₂CH(OCH₂)₂-; 8H, -CH₂C*H*₂CH=CH-), 1.63-1.21 (m, 56H, -CH₂C*H*₂CH₂-, -C*H*₂CH₃), 0.87 (t, 12H, -CH₂C*H*₃). MS (ESI): m/z=1029.9 ([M+H]⁺).

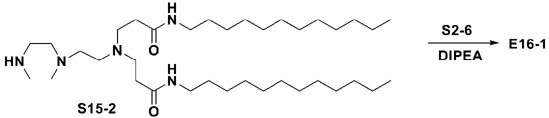

### Example-17: Cationic lipid (E17-1)

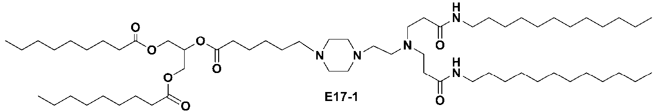

The preparation process is as follows:
Under nitrogen atmosphere, compound **S15-2** (0.61 g, 1.0 mmol) was dissolved in acetonitrile (20 mL), then **S3-4** (0.72 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, and then extracted with 0.6 M hydrochloric acid/10% sodium chloride solution and saturated sodium bicarbonate solution. aThe organic phases were combined, dried with anhydrous magnesium sulfate, filtered, then the filtrate was concentrated and purified by column chromatography to obtain cationic lipid **E17-1** (0.90 g). ¹H NMR (400 MHz, CDCl₃) δ: 5.37-5.31 (m, 1H, -C(=O)OC*H*<), 4.38-4.29 (m, 2H, >CH(C*H*₂OC(=O))₂-), 4.19-4.13 (m, 2H, >CH(C*H*₂OC(=O))₂-), 3.28 (dd, 4H, -C(=O)NHC*H*₂-), 2.80-2.35 (m, 28H; 4H, >CHCH₂OC(=O)C*H*₂-; 2H, >CHOC(=O)C*H*₂-; 4H, -C*H*₂C(=O)NH-; 4H, >NC*H*₂CH₂-; 4H, pip-C*H*₂CH₂-; 8H, pip-*H*; 2H, pip-CH₂C*H*₂N<), 1.63-1.21 (m, 70H, -CH₂C*H*₂CH₂-, -C*H*₂CH₃), 0.88 (t, 12H, -CH₂C*H*₃). MS (ESI): m/z=1075.9 ([M+H]⁺).

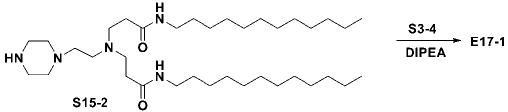

### Example-18: Cationic lipid (E18-1)

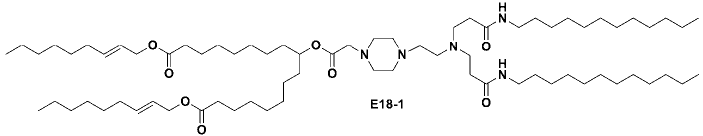

The preparation process is as follows:
Step a: **S4-7** (0.75 g, 4.0 mmol) was dissolved in isopropanol, then sufficient amount of anhydrous potassium carbonate was added with stirring at room temperature until the reaction solution became alkaline. **S15-1** (2.87 g, 12.0 mmol) was added to the reaction solution, which was placed under reflux (90°C) and stirred for 36 h. After completion of the reaction, the reaction solution was concentrated to obtain a crude product, which was then purified by column chromatography to obtain compound **S18-1** (1.94 g).

Step b: under nitrogen atmosphere, **DCC** (0.45 g, 2.2 mmol) was added to a round-bottom flask containing **S18-1** (0.67 g, 1.0 mmol), **S4-6** (0.68 g, 1.2 mmol), and **DMAP** (0.03 g, 0.3 mmol) dissolved in dichloromethane (20 mL), and the reaction was carried out for 16 h at room temperature. After completion of the reaction, the precipitate was removed by filtration, the filtrate was concentrated, and the crude product was purified by column chromatography to obtain the cationic lipid **E18-1** (1.01 g). ¹H NMR (400 MHz, CDCl₃) δ: 5.82-5.71 (m, 2H, -C(=O)OCH₂C*H*=CH-), 5.56-5.54 (m, 2H, -C(=O)OCH₂CH=C*H*-), 4.93-4.87 (m, 1H, -C(=O)OC*H*<), 4.55 (t, 4H, -C(=O)OC*H*₂CH=CH-), 3.28 (dd, 4H, -C(=O)NHC*H*₂-), 3.16 (s, 2H, pip-C*H*₂C(=O)O-), 2.80-2.38 (m, 20H; 4H, >NC*H*₂CH₂-; 8H, pip-H; 2H, pip-C*H*₂CH₂-; 2H, pip-C*H*₂CH₂-; 4H, -C*H*₂C(=O)NH-), 2.18-2.09 (m, 4H, -OC(=O)C*H*₂(CH₂)₆-), 2.06-1.99 (m, 4H, -CH=CHC*H*₂CH₂-), 1.64-1.22 (m, 80H, -CH₂C*H*₂CH₂-, -C*H*₂CH₃), 0.87 (t, 12H, -CH₂C*H*₃). MS (ESI): m/z=1212.1 ([M+H]⁺).

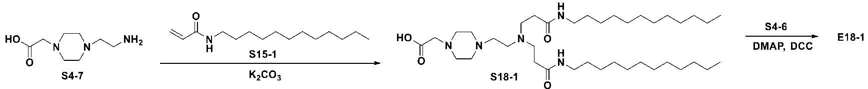

### Example-19: Cationic lipid (E19-1)

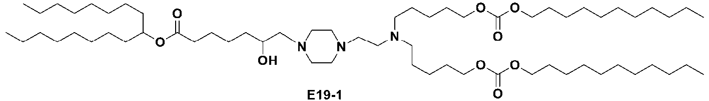

The preparation process is as follows:
Step a: 5 bromopentyl alcohol **(S19-1,** 1.34 g, 8.0 mmol) was dissolved in 50 mL of dichloromethane, **DMAP** (2.06 g, 16.0 mmol) was added, and ***p*-nitrophenyl chloroformate** (1.77 g, 8.8 mmol) was added sequentially, the reaction was stirred at room temperature for 3 h, then **S1-3** (1.54 g, 9.0 mmol) was added to the reaction solution, and the mixture was stirred overnight at room temperature. After completion of the reaction, 20 mL of dichloromethane was added, then washed with 30 mL of saturated brine, the organic phase was dried with anhydrous magnesium sulfate, filtered, then the filtrate was concentrated and purified by column chromatography to obtain 5-bromopentylundecyl carbonate **(S19-2,** 2.11 g).

Step b: under nitrogen atmosphere, compound **S1-1** (0.46 g, 2.0 mmol) was dissolved in acetonitrile (30 mL), then **S19-2** (1.83 g, 5.0 mmol) and **DIPEA** (0.52 g, 4.0 mmol) were added sequentially under slow stirring, and the reaction was stirred for about 20 h at room temperature. After completion of the reaction, the reaction solution was concentrated and dissolved with dichloromethane, and then extracted with 0.6 M hydrochloric acid/10% sodium chloride solution and saturated sodium bicarbonate solution, then the organic phases were combined, dried with anhydrous magnesium sulfate, filtered, and then the filtrate was concentrated to obtain a crude product. The crude product was then dissolved with dichloromethane, TFA was added to 0.1 M. After reacting for 4 h, the reaction was adjusted to neutral pH, concentrated, diluted with purified water, and extracted with dichloromethane. The extract was dried with anhydrous magnesium sulfate, filtered, then the filtrate was concentrated and recrystallized to obtain amine-deprotected intermediate **S19-3** (1.11 g).

Step c: **S19-3** (0.70 g, 1.0 mmol) and **DIPEA** (0.10 g, 0.8 mmol) were added to the methanol solution of **S1-11** (0.57 g, 1.5 mmol) sequentially, and the reaction solution was placed under reflux (90°C) and stirred for 24 h. After completion of the reaction, the reaction solution was concentrated, and the crude product was purified by column chromatography to obtain the cationic lipid **E19-1** (0.78 g). ¹H NMR (400 MHz, CDCl₃) δ: 4.90-4.81 (m, 1H, >C*H*OC(=O)-), 4.15-4.10 (m, 8H, -C*H*₂OC(=O)OC*H*₂-), 3.71-3.61 (m, 1H, -C*H*(OH)-), 2.84-2.24 (m, 20H; 8H, pip-*H*; 4H, pip-C*H*₂CH₂-; 2H, pip-CH₂C*H*₂N<; 4H, >NC*H*₂CH₂-; 2H, -C*H*₂C(=O)O-), 1.66-1.23 (m, 82H, -CH₂C*H*₂CH₂-, -C*H*₂CH₃), 0.89 (t, 12H, -CH₂C*H*₃). MS (ESI): m/z=1079.9 ([M+H]⁺).

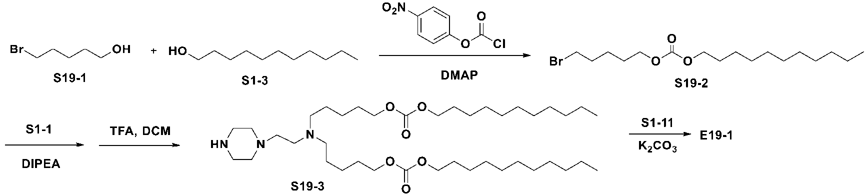

### Example-20: Cationic lipid (E20-1)

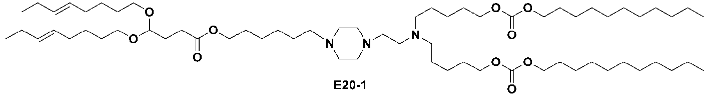

The preparation process is as follows:
Under nitrogen atmosphere, compound **S19-3** (0.70 g, 1.0 mmol) was dissolved in acetonitrile (20 mL), then **S2-6** (0.66 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and the organic phases were combined, dried with anhydrous magnesium sulfate, filtered, the filtrate was concentrated and purified by column chromatography to obtain cationic lipid **E20-1** (0.93 g). ¹H NMR (400 MHz, CDCl₃) δ: 5.41-5.26 (m, 4H, -C*H*=C*H*-), 4.63 (t, 1H, >C*H*(OCH₂)₂-), 4.15-4.10 (m, 8H, -C*H*₂OC(=O)OC*H*₂-), 4.09-4.00 (m, 2H, -C(=O)OC*H*₂-), 3.52-3.36 (m, 4H, >CH(OC*H*₂)₂-), 2.80-2.35 (m, 20H; 4H, >NC*H*₂CH₂-; 2H, >CHCH₂C*H*₂C(=O)O-; 4H, pip-C*H*₂CH₂-; 8H, pip-H; 2H, pip-CH₂C*H*₂N<), 2.11-1.82 (m, 10H; 2H, -C*H*₂CH(OCH₂)₂-; 8H, -CH₂C*H*₂CH=CH-), 1.63-1.21 (m, 64H, -CH₂C*H*₂CH₂-, -C*H*₂CH₃), 0.87 (t, 12H, -CH₂C*H*₃). MS (ESI): m/z=1119.9 ([M+H]⁺).

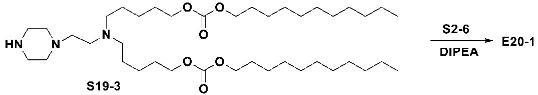

### Example-21: Cationic lipid (E21-1)

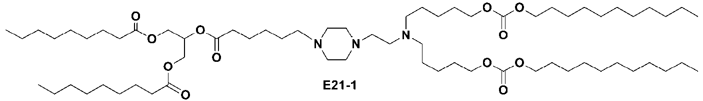

Under nitrogen atmosphere, compound **S19-3** (0.70 g, 1.0 mmol) was dissolved in acetonitrile (20 mL), then **S3-4** (0.72 g, 1.3 mmol) and **DIPEA** (0.17 g, 1.3 mmol) were added sequentially under slow stirring, and the reaction was stirred for about 20 h at room temperature. After completion of the reaction, the reaction solution was concentrated and dissolved with dichloromethane, then extracted with 0.6 M hydrochloric acid/10% sodium chloride solution and saturated sodium bicarbonate solution, and the organic phases were combined, dried with anhydrous magnesium sulfate, filtered, then the filtrate was concentrated and purified by column chromatography to obtain cationic lipid **E21-1** (0.97 g). ¹H NMR (400 MHz, CDCl₃) δ: 5.37-5.31 (m, 1H, -C(=O)OC*H*<), 4.38-4.29 (m, 2H, >CH(C*H*₂OC(=O))₂-), 4.19-4.13 (m, 2H, >CH(C*H*₂OC(=O))₂-), 4.15-4.10 (m, 8H, -C*H*₂OC(=O)OC*H*₂-), 2.80-2.35 (m, 24H; 4H, >NC*H*₂CH₂-; 4H, >CHCH₂OC(=O)C*H*₂-; 2H, >CHOC(=O)C*H*₂-; 4H, pip-C*H*₂CH₂-; 8H, pip-*H*; 2H, pip-CH₂C*H*₂N<), 1.63-1.21 (m, 78H, -CH₂C*H*₂CH₂-, -C*H*₂CH₃), 0.87 (t, 12H, -CH₂C*H*₃). MS (ESI): m/z=1165.9 ([M+H]⁺).

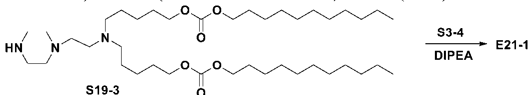

### Example-22: Cationic lipid (E22-1)

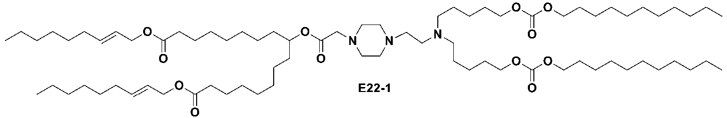

The preparation process is as follows:
Step a: under nitrogen atmosphere, compound **S4-7** (0.37 g, 2.0 mmol) was dissolved in acetonitrile (30 mL), then **S19-2** (1.83 g, 5.0 mmol) and **DIPEA** (0.52 g, 4.0 mmol) were added successively under slow stirring. The reaction was stirred at room temperature for about 20 h. After completion of the reaction, the reaction solution was concentrated and dissolved in dichloromethane, and then successively extracted with 0.6 M hydrochloric acid/10% sodium chloride solution and saturated sodium bicarbonate solution. The organic phases were combined, dried with anhydrous magnesium sulfate, filtered, and the filtrate was concentrated, and then the crude product was purified by column chromatography to obtain the compound **S22-1** (1.26 g).

Step b: under nitrogen atmosphere, **DCC** (0.45 g, 2.2 mmol) was added to a round-bottom flask containing **S22-1** (0.76 g, 1.0 mmol), **S4-6** (0.68 g, 1.2 mmol), and **DMAP** (0.03 g, 0.3 mmol) dissolved in dichloromethane (20 mL). The reaction was carried out at room temperature for 16 h. After completion of the reaction, the precipitate was removed by filtration, and the filtrate was concentrated. The crude product was purified by column chromatography to obtain the cationic lipid **E22-1** (1.09 g). ¹H NMR (400 MHz, CDCl₃) δ: 5.82-5.71 (m, 2H, -C(=O)OCH₂C*H*=CH-), 5.56-5.54 (m, 2H, -C(=O)OCH₂CH=C*H*-), 4.93-4.87 (m, 1H, -C(=O)OC*H*<), 4.55 (t, 4H, -C(=O)OC*H*₂CH=CH-), 4.15-4.10 (m, 8H, -C*H*₂OC(=O)OC*H*₂-), 3.16 (s, 2H, pip-C*H*₂C(=O)O-), 2.80-2.38 (m, 16H; 4H, >NC*H*₂CH₂-; 8H, pip-*H*; 2H, pip-C*H*₂CH₂-; 2H, pip-CH₂C*H*₂-), 2.18-2.09 (m, 4H, -OC(=O)C*H*₂(CH₂)₆-), 2.06-1.99 (m, 4H, -CH=CHC*H*₂CH₂-), 1.64-1.22 (m, 88H, -CH₂C*H*₂CH₂-, -C*H*₂CH₃), 0.91 (t, 12H, -CH₂C*H*₃). MS (ESI): m/z=1302.1 ([M+H]⁺).

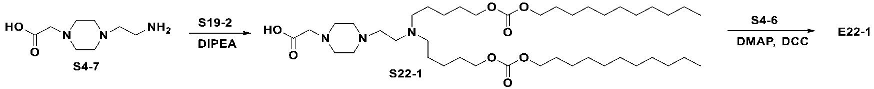

### Example-23: Cationic lipid (E23-1)

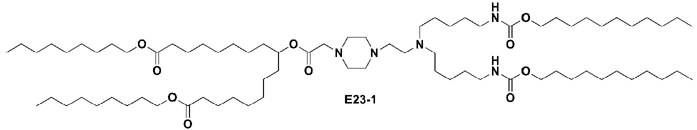

The preparation process is as follows:
Step a: **S1-3** (2.58 g, 15.0 mmol) was dissolved in dichloromethane (80 mL), triethylamine (5 mL) and N,N'-carbonyldiimidazole **(CDI,** 2.43 g, 15.0 mmol) were added sequentially, and the reaction was carried out for 1.5 h at 50°C. Then 5-amino-1-butanol **(S23-1,** 2.32 g, 22.5 mmol) was added, and the reaction was continued at 50°C for 16 h. After completion of the reaction, the reaction solution was cooled to room temperature, washed with 5% citric acid (40 mL*2) and saturated brine (40 mL), and the organic phase was dried by anhydrous magnesium sulfate, filtered, and the filtrate was concentrated to obtain a crude product. Dichloromethane (40 mL) was added to the crude product, stirred for 10 min, filtered, and the filter was washed with a small amount of dichloromethane. The product **S23-2** (2.96 g) was obtained after concentration.

Step b: **S23-2** (2.41 g, 8.0 mmol) was dissolved in dichloromethane (100 mL), and then triphenylphosphine (**PPh₃**, 3.14 g, 12.0 mmol) was added. Under ice bath conditions, carbon tetrabromide (**CBr₄**, 3.97 g, 12.0 mmol) was added in batches and the reaction was carried out for 20 minutes under the ice bath conditions. The reaction was quenched with 10 mL of methanol and the mixture was directly concentrated to obtain the crude product. The crude product was purified by column chromatography to obtain the product **S23-3** (1.71 g).

Step c: under nitrogen atmosphere, compound **S4-7** (0.28 g, 1.5 mmol) was dissolved in acetonitrile (20 mL), then **S23-3** (1.37 g, 3.8 mmol) and **DIPEA** (0.39 g, 3.0 mmol) were added successively under slow stirring. The reaction was stirred at room temperature for about 20 h. After completion of the reaction, the reaction solution was concentrated and dissolved in dichloromethane, and then successively extracted with 0.6 M hydrochloric acid/10% sodium chloride solution and saturated sodium bicarbonate solution. The organic phases were combined, dried with anhydrous magnesium sulfate, filtered, and the filtrate was concentrated, and then the crude product was purified by column chromatography to obtain the compound **S23-4** (0.95 g).

Step d: under nitrogen atmosphere, **DCC** (0.45 g, 2.2 mmol) was added to a round-bottom flask containing **S23-4** (0.75 g, 1.0 mmol), **S4-10** (0.68 g, 1.2 mmol), and **DMAP** (0.03 g, 0.3 mmol) dissolved in dichloromethane (20 mL). The reaction was carried out at room temperature for 16 h. After completion of the reaction, the precipitate was removed by filtration, and the filtrate was concentrated. The crude product was purified by column chromatography to obtain the cationic lipid **E23-1** (1.07 g). ¹H NMR (400 MHz, CDCl₃) δ: 4.93-4.87 (m, 1H, -C(=O)OC*H*<), 4.11 (t, 4H, -NHC(=O)OC*H*₂-), 4.04 (t, 4H, -C(=O)OC*H*₂-), 3.16 (s, 2H, pip-C*H*₂C(=O)O-), 3.15 (t, 4H, -C*H*₂NHC(=O)O-), 2.80-2.38 (m, 16H; 4H, >NC*H*₂CH₂-; 8H, pip-*H*; 2H, pip-C*H*₂CH₂-; 2H, pip-CH₂C*H*₂-), 2.29 (t, 4H, -(CH₂)₆C*H*₂C(=O)O-), 1.84-1.76 (m, 4H, -C(=O)OCH₂C*H*₂-), 1.64-1.22 (m, 96H, -CH₂C*H*₂CH₂-, -C*H*₂CH₃), 0.88 (t, 12H, -CH₂C*H*₃). MS (ESI): m/z=1304.1 ([M+H]⁺).

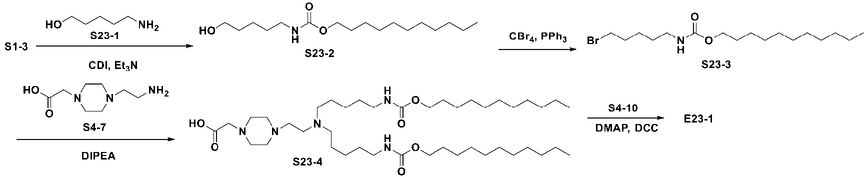

### Example-24: Cationic Lipid (E24-1)

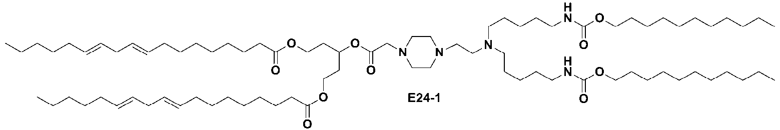

The preparation process is as follows:
Under nitrogen atmosphere, **DCC** (0.45 g, 2.2 mmol) was added to a round-bottom flask containing **S23-4** (0.75 g, 1.0 mmol), **S5-3** (0.77 g, 1.2 mmol), and **DMAP** (0.03 g, 0.3 mmol) dissolved in dichloromethane (20 mL). The reaction was carried out at room temperature for 16 h. After completion of the reaction, the precipitate was removed by filtration, and the filtrate was concentrated. The crude product was purified by column chromatography to obtain the cationic lipid **E24-1** (1.15 g). ¹H NMR (400 MHz, CDCl₃) δ: 5.46-5.29 (m, 8H, -C*H*=C*H*-), 5.17-5.08 (m, 1H, -C(=O)OC*H*<), 4.19-4.00 (m, 8H; 4H, -C(=O)OC*H*₂-; 4H, -NHC(=O)OC*H*₂-), 3.19 (s, 2H, pip-C*H*₂C(=O)O-), 3.15 (t, 4H, -C*H*₂NHC(=O)O-), 2.87-2.74 (m, 4H, -CH=CHC*H*₂CH=CH-), 2.73-2.35 (m, 16H; 4H, >NC*H*₂CH₂-; 8H, pip-*H*; 2H, pip-C*H*₂CH₂-; 2H, pip-CH₂C*H*₂-), 2.29 (t, 4H; >CH(CH₂)₂OC(=O)C*H*₂-), 2.10-1.99 (m, 8H, -CH=CHC*H*₂-), 1.97-1.89 (m, 4H, >CHC*H*₂-), 1.66-1.22 (m, 80H, -CH₂C*H*₂CH₂-, -C*H*₂CH₃), 0.88 (t, 12H, -CH₂C*H*₃). MS (ESI): m/z=1380.2 ([M+H]⁺).

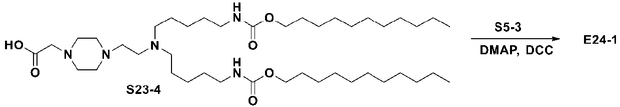

### Example-25: Cationic Lipid (E25-1)

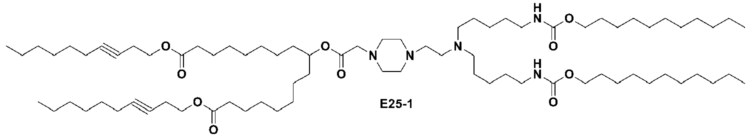

The preparation process is as follows:
Under nitrogen atmosphere, **DCC** (0.45 g, 2.2 mmol) was added to a round-bottom flask containing **S23-4** (0.75 g, 1.0 mmol), **S6-2** (0.71 g, 1.2 mmol), and **DMAP** (0.03 g, 0.3 mmol) dissolved in dichloromethane (20 mL). The reaction was carried out at room temperature for 16 h. After completion of the reaction, the precipitate was removed by filtration, and the filtrate was concentrated. The crude product was purified by column chromatography to obtain the cationic lipid **E25-1** (1.09 g). ¹H NMR (400 MHz, CDCl₃) δ: 4.93-4.87 (m, 1H, -C(=O)OC*H*<), 4.12 (t, 4H, -C≡CCH₂C*H*₂OC(=O)-), 4.11 (t, 4H, -NHC(=O)OC*H*₂-), 3.17 (s, 2H, pip-C*H*₂C(=O)O-), 3.15 (t, 4H, -C*H*₂NHC(=O)O-), 2.80-2.38 (m, 20H; 4H, >NC*H*₂CH₂-; 8H, pip-H; 2H, pip-C*H*₂CH₂-; 2H, pip-CH₂C*H*₂-; 4H, -C≡CC*H*₂CH₂OC(=O)-), 2.30 (t, 4H, -(CH₂)₄C*H*₂C≡C-), 2.18-2.09 (m, 4H, -OC(=O)C*H*₂(CH₂)₆-), 1.65-1.23 (m, 88H, -CH₂C*H*₂CH₂-, -C*H*₂CH₃), 0.88 (t, 12H, -CH₂C*H*₃). MS (ESI): m/z=1324.1 ([M+H]⁺).

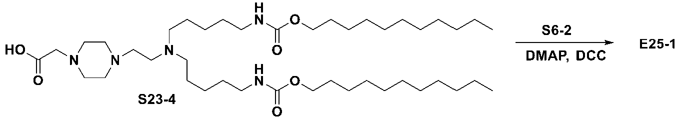

### Example-26: Cationic Lipid (E26-1)

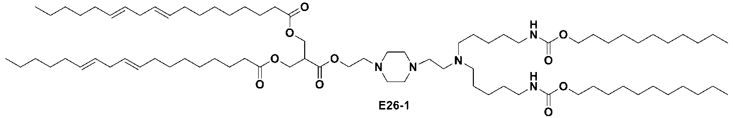

The preparation process is as follows:
Step a: under nitrogen atmosphere, compound **S7-1** (0.35 g, 2.0 mmol) was dissolved in acetonitrile (30 mL), then **S23-3** (1.82 g, 5.0 mmol) and **DIPEA** (0.52 g, 4.0 mmol) were added successively under slow stirring. The reaction was stirred at room temperature for about 20 h. After completion of the reaction, the reaction mixture was concentrated and dissolved in dichloromethane, then successively extracted with 0.6 M hydrochloric acid/10% sodium chloride solution and saturated sodium bicarbonate solution. The organic phases were combined, dried with anhydrous magnesium sulfate, filtered, and the filtrate was concentrated. The compound **S26-1** (1.24 g) was obtained after purification by column chromatography.

Step b: under nitrogen atmosphere, **DCC** (0.45 g, 2.2 mmol) was added to a round-bottom flask containing **S7-5** (0.65 g, 1.0 mmol), **S26-1** (0.89 g, 1.2 mmol), and **DMAP** (0.03 g, 0.3 mmol) dissolved in dichloromethane (20 mL). The reaction was carried out at room temperature for 16 h. After completion of the reaction, the precipitate was removed by filtration, and the filtrate was concentrated. The crude product was purified by column chromatography to obtain the cationic lipid **E26-1** (1.13 g). ¹H NMR (400 MHz, CDCl₃) δ: 5.45-5.25 (m, 8H, -C*H*=C*H*-), 4.39-4.29 (m, 4H, >CH(C*H*₂OC(=O)₂-), 4.24 (t, 2H, pip-CH₂C*H*₂OC(=O)-), 4.11 (t, 4H, -NHC(=O)OC*H*₂-), 3.15 (t, 4H, -C*H*₂NHC(=O)O-), 3.02 (q, 1H, >C*H*C(=O)OCH₂-), 2.80-2.38 (m, 22H; 4H, >NC*H*₂CH₂-; 8H, pip-*H*; 4H, pip-C*H*₂CH₂-; 2H, pip-CH₂C*H*₂N<; 4H, -CH=CHC*H*₂CH=CH-), 2.29 (t, 4H, -C*H*₂C(=O)OCH₂CH<), 2.03-1.97 (m, 8H, -CH=CHC*H*₂-), 1.65-1.23 (m, 80H, -CH₂C*H*₂CH₂-, -C*H*₂CH₃), 0.88 (t, 12H, -CH₂C*H*₃). MS (ESI): m/z=1366.2 ([M+H]⁺).

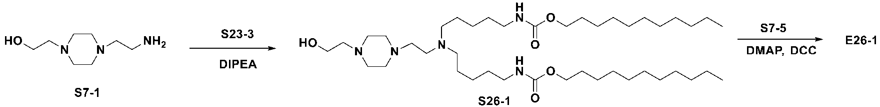

### Example-27: Cationic Lipid (E27-1)

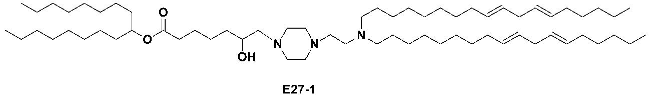

The preparation process is as follows:
Step a: **S1-1** (0.46 g, 2.0 mmol) was dissolved in dichloromethane (20 mL) and cooled to 0°C under ice bath conditions. Under vigorous stirring, (9Z,12Z)-octadeca-9,12-dienal **(S27-1,** 1.58 g, 6.0 mmol) was added, followed by the addition of sodium triacetoxyborohydride **(NaBH(OAc)₃,** 1.27 g, 6.0 mmol) in three portions over 10 minutes. Then the ice bath was removed, and the reaction solution was stirred at room temperature for 2 h. After completion of the reaction, an aqueous solution of NaOH (1 M, 10 mL) was added, and the solution was stirred for 15 minutes before being diluted with water. The product was extracted with dichloromethane twice (10 mL × 2). The combined organic phases were washed with brine (10 mL), dried with anhydrous magnesium sulfate, filtered, and the filtrate was concentrated to obtain the crude product. The crude product was dissolved in dichloromethane again, and TFA was added to a concentration of 0.1 M. After reacting for 4 h, the reaction solution was adjusted to neutral pH, concentrated, and diluted with purified water. The solution was then extracted with dichloromethane, the extract was dried with anhydrous magnesium sulfate, filtered, and the filtrate was concentrated, and the residue was recrystallized to obtain intermediate **S27-2** with a free amino group (1.15 g, 91.8%).

Step b: **S27-2** (0.63 g, 1.0 mmol) and **DIPEA** (0.10 g, 0.8 mmol) were successively added to a methanol solution of **S1-11** (0.57 g, 1.5 mmol). The reaction solution was then placed under reflux and stirred at 90°C for 24 h. After completion of the reaction, the reaction solution was concentrated. The crude product was purified by column chromatography to obtain the cationic lipid **E27-1** (0.74 g). ¹H NMR (400 MHz, CDCl₃) δ: 5.45-5.25 (m, 8H, -C*H*=C*H*-), 4.90-4.81 (m, 1H, >C*H*OC(=O)-), 3.71-3.61 (m, 1H, -CH(OH)-), 2.84-2.24 (m, 24H; 8H, pip-H; 4H, pip-C*H*₂CH₂-; 2H, pip-CH₂C*H*₂N<; 4H, >NC*H*₂CH₂-; 4H, -CH=CHC*H*₂CH=CH-; 2H, -C*H*₂C(=O)O-), 2.03-1.97 (m, 8H, -CH=CHC*H*₂-), 1.66-1.23 (m, 70H, -CH₂C*H*₂CH₂-, -C*H*₂CH₃), 0.89 (t, 12H, -CH₂C*H*₃). MS (ESI): m/z=1008.0 ([M+H]⁺).

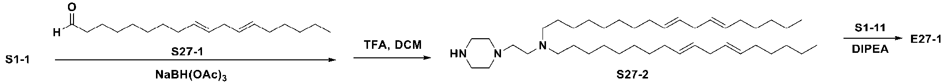

### Example-28: Cationic Lipid (E28-1)

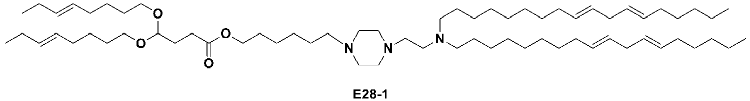

The preparation process is as follows:
Under nitrogen atmosphere, compound **S27-2** (0.63 g, 1.0 mmol) was dissolved in acetonitrile (20 mL). Then **S2-6** (0.66 g, 1.3 mmol) and **DIPEA** (0.17 g, 1.3 mmol) were added successively under slow stirring, and the reaction was stirred at room temperature for about 20 h. After completion of the reaction, the reaction solution was concentrated and dissolved in dichloromethane, then successively extracted with 0.6 M hydrochloric acid/10% sodium chloride solution and saturated sodium bicarbonate solution. The organic phases were combined, dried with anhydrous magnesium sulfate, filtered, and the filtrate was concentrated, and then the crude product was purified by column chromatography to obtain cationic lipid **E28-1** (0.87 g). ¹H NMR (400 MHz, CDCl₃) δ: 5.45-5.25 (m, 12H, *-CH=CH-),* 4.63 (t, 1H, >C*H*(OCH₂)₂-), 4.09-4.00 (m, 2H, -C(=O)OC*H*₂-), 3.52-3.36 (m, 4H, >CH(OC*H*₂)₂-), 2.80-2.35 (m, 24H; 4H, >NC*H*₂CH₂-; 2H, >CHCH₂C*H*₂C(=O)O-; 4H, pip-C*H*₂CH₂-; 8H, pip-H; 2H, pip-CH₂C*H*₂N<; 4H, -CH=CHC*H*₂CH=CH-), 2.11-1.82 (m, 18H; 2H, -C*H*₂CH(OCH₂)₂-; 16H, -CH₂C*H*₂CH=CH-), 1.63-1.21 (m, 56H, -CH₂C*H*₂CH₂-, -C*H*₂CH₃), 0.87 (t, 12H, -CH₂C*H*₃). MS (ESI): m/z=1048.0 ([M+H]⁺.

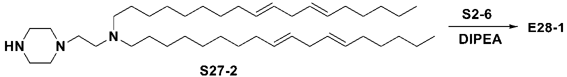

### Example-29: Cationic Lipid (E29-1)

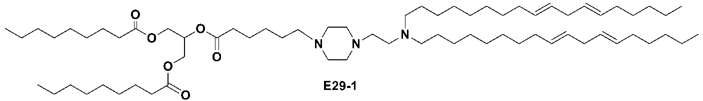

The preparation process is as follows:
Under nitrogen atmosphere, compound **S27-2** (0.63 g, 1.0 mmol) was dissolved in acetonitrile (20 mL), then **S3-4** (0.72 g, 1.3 mmol) and **DIPEA** (0.17 g, 1.3 mmol) were added successively under slow stirring. The reaction was stirred at room temperature for about 20 h. After completion of the reaction, the reaction solution was concentrated and dissolved in dichloromethane, and then successively extracted with 0.6 M hydrochloric acid/10% sodium chloride solution and saturated sodium bicarbonate solution. The organic phases were combined, dried with anhydrous magnesium sulfate, filtered, and the filtrate was concentrated, and then the crude product was purified by column chromatography to obtain cationic lipid **E29-1** (0.92 g). ¹H NMR (400 MHz, CDCl₃) δ: 5.45-5.25 (m, 9H; 1H, -C(=O)OC*H*<; 8H, -C*H*=C*H*-), 4.38-4.29 (m, 2H, >CH(C*H*₂OC(=O))₂-), 4.19-4.13 (m, 2H, >CH(C*H*₂OC(=O))₂-), 2.80-2.35 (m, 28H; 4H, >NC*H*₂CH₂-; 4H, >CHCH₂OC(=O)C*H*₂-; 2H, >CHOC(=O)C*H*₂-; 4H, pip-C*H*₂CH₂-; 8H, pip-H; 2H, pip-CH₂C*H*₂N<; 4H, -CH=CHC*H*₂CH=CH-), 2.03-1.97 (m, 8H, -CH=CHC*H*₂-), 1.63-1.21 (m, 66H, -CH₂C*H*₂CH₂-, -C*H*₂CH₃), 0.87 (t, 12H, -CH₂C*H*₃). MS (ESI): m/z=1094.0 ([M+H]⁺).

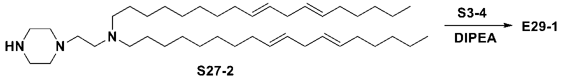

### Example-30: Cationic Lipid (E30-1)

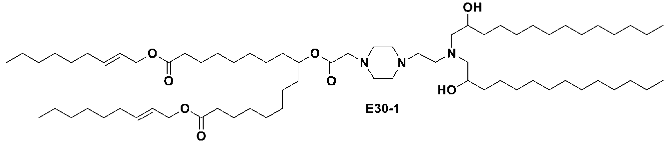

The preparation process is as follows:
Step a: under nitrogen atmosphere, **S4-7** (0.75 g, 4.0 mmol) and **NaOH** (0.16 g, 4.0 mmol) were dissolved in a prepared mixed solution of 1,4-dioxane/water (2:1, v/v) (10 mL), and the reaction solution was cooled to 0°C to obtain reaction solution A. Di-tert-butyl dicarbonate (Boc₂O, 1.40 g, 6.4 mmol) was dissolved in a prepared mixed solution of 1,4-dioxane/water (2:1, v/v) (10 mL) to obtain reaction solution B. Reaction solution B was then slowly added to reaction solution A, and the temperature was gradually increased to room temperature. The reaction was allowed to proceed overnight. After completion of the reaction, the reaction solution was concentrated, the residue was dissolved in water and washed thrice with ethyl acetate (5 mL × 3), then the pH was adjusted to 1-2 with HCl (1 M), and the solution was extracted thrice with ethyl acetate (10 mL × 3). The combined organic phases were dried with anhydrous magnesium sulfate, filtered, and the filtrate was concentrated to obtain compound **S30-1** (1.14 g, 99%). No further purification was required, and the compound could be used directly in the next step.

Step b: Under nitrogen atmosphere, **DCC** (1.36 g, 6.6 mmol) was added to a round-bottom flask containing **S4-6** (2.03 g, 3.6 mmol), **S30-1** (0.86 g, 3.0 mmol) and **DMAP** (0.09 g, 0.8 mmol) dissolved in dichloromethane (30 mL). The reaction was carried out at room temperature for 16 h. After completion of the reaction, the precipitate was removed by filtration, and the filtrate was concentrated to obtain the crude product. The crude product was dissolved in dichloromethane again, and TFA was added to a concentration of 0.1 M. The solution was reacted for 4 h, then the reaction solution was adjusted to neutral pH, concentrated, and diluted with purified water. The solution was then extracted with dichloromethane, and the extract was dried with anhydrous magnesium sulfate, filtered, and the filtrate was concentrated. The final product was obtained by recrystallization as compound **S30-2** (1.74 g).

Step c: **S30-2** (1.47 g, 2.0 mmol) and **DIPEA** (0.15 g, 1.2 mmol) were successively added to a methanol solution of 1,2-epoxytetradecane **(S30-3,** 1.27 g, 6.0 mmol). The reaction solution was then placed under reflux and stirred at 90°C for 24 h. After completion of the reaction, the reaction solution was concentrated. The crude product was purified by column chromatography to obtain the cationic lipid **E30-1** (1.84 g). ¹H NMR (400 MHz, CDCl₃) δ: 5.82-5.71 (m, 2H, -C(=O)OCH₂C*H=*CH-), 5.60-5.50 (m, 2H, -C(=O)OCH₂CH=C*H*-), 4.93-4.86 (m, 1H, -C(=O)OC*H*<), 4.54-4.46 (m, 4H, -C(=O)OC*H*₂CH=CH-), 3.63-3.49 (m, 2H, >C*H*OH), 3.15 (s, 2H, pip-C*H*₂C(=O)O-), 2.93-2.32 (m, 16H; 4H, >NC*H*₂CH<; 8H, pip-*H*; 2H, pip-C*H*₂CH₂-; 2H, pip-CH₂C*H*₂-), 2.29 (t, 4H, -OC(=O)C*H*₂CH₂-), 2.08-2.00 (m, 4H, -CH=CHC*H*₂CH₂-), 1.65-1.19 (m, 84H, -CH₂C*H*₂CH₂-, -C*H*₂CH₃), 0.88 (t, 12H, -CH₂C*H*₃). MS (ESI): m/z=1158.9 ([M+H]⁺).

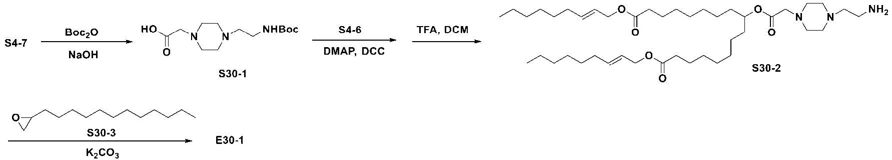

### Example-31: Cationic Lipid (E31-1)

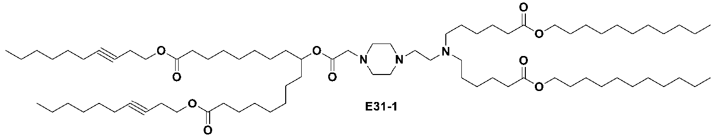

The preparation process is as follows:
Step a: under nitrogen atmosphere, **DCC** (1.36 g, 6.6 mmol) was added to a round-bottom flask containing **S6-2** (2.12 g, 3.6 mmol), **S30-1** (0.86 g, 3.0 mmol) and **DMAP** (0.09 g, 0.8 mmol) dissolved in dichloromethane (30 mL). The reaction was carried out at room temperature for 16 h. After completion of the reaction, the precipitate was removed by filtration, and the filtrate was concentrated to obtain the crude product. The crude product was dissolved in dichloromethane again, and TFA was added to a concentration of 0.1 M. The solution was reacted for 4 h, then the reaction solution was adjusted to neutral pH, concentrated, and diluted with purified water. The solution was then extracted with dichloromethane. The extract was dried with anhydrous magnesium sulfate, filtered, and the filtrate was concentrated, and then recrystallized to obtain compound **S31-1** (1.79 g).

Step b: under nitrogen atmosphere, compound **S31-1** (1.52 g, 2.0 mmol) was dissolved in acetonitrile (50 mL), then **S1-14** (1.75 g, 5.0 mmol) and **DIPEA** (0.52 g, 4.0 mmol) were added successively under slow stirring, and the reaction was stirred at room temperature for about 20 h. After completion of the reaction, the reaction solution was concentrated and dissolved in dichloromethane, then successively extracted with 0.6 M hydrochloric acid/10% sodium chloride solution and saturated sodium bicarbonate solution. The organic phases were combined, dried with anhydrous magnesium sulfate, filtered, and the filtrate was concentrated. The crude product was purified by column chromatography to obtain cationic lipid **E31-1** (2.16 g). ¹H NMR (400 MHz, CDCl₃) δ: 4.93-4.87 (m, 1H, -C(=O)OC*H*<), 4.12 (t, 4H, -C≡CCH₂C*H*₂OC(=O)-), 4.04 (t, 4H, >NCH₂CH₂C(=O)OC*H*₂-), 3.16 (s, 2H, pip-C*H*₂C(=O)O-), 2.80-2.38 (m, 28H; 4H, >NC*H*₂CH₂-; 8H, pip-*H*; 2H, pip-C*H*₂CH₂-; 2H, pip-CH₂C*H*₂-; 8H, -C*H*₂C(=O)O-; 4H, -C≡CC*H*₂CH₂OC(=O)-), 2.30 (t, 4H, -(CH₂)₄C*H*₂C≡C-), 1.84-1.76 (m, 4H, -C(=O)OCH₂C*H*₂CH₂-), 1.65-1.23 (m, 84H, -CH₂C*H*₂CH₂-, -C*H*₂CH₃), 0.89 (t, 12H, -CH₂C*H*₃). MS (ESI): m/z=1294.1 ([M+H]⁺).

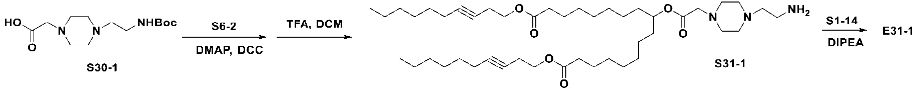

### Example-32: Cationic Lipid (E32-1)

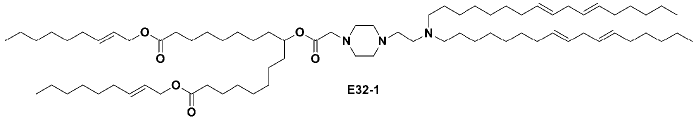

The preparation process is as follows:
Compound **S30-2** (1.47 g, 2.0 mmol) was dissolved in dichloromethane (20 mL) and cooled to 0°C in ice bath conditions. Under vigorous stirring, **S27-1** (1.58 g, 6.0 mmol) was added, followed by the addition of **NaBH(OAc)₃** (1.27 g, 6.0 mmol) in three portions over 10 minutes. The ice bath was then removed, and the reaction solution was stirred at room temperature for 2 h. After completion of the reaction, an aqueous solution of NaOH (1 M, 10 mL) was added, and the solution was stirred for 15 minutes, and then diluted with water. The product was extracted with dichloromethane twice (10 mL × 2), and the combined organic phases were washed with brine (10 mL), dried with anhydrous magnesium sulfate, filtered, and the filtrate was concentrated, and then the crude product was purified by column chromatography to obtain cationic lipid **E32-1** (2.23 g, 92.8%). ¹H NMR (400 MHz, CDCl₃) δ: 5.82-5.71 (m, 2H, -C(=O)OCH₂C*H*=CH-), 5.56-5.54 (m, 2H, -C(=O)OCH₂CH=C*H*-), 5.45-5.25 (m, 8H, -(CH₂)₇C*H*=C*H*(CH₂)₄-), 4.93-4.87 (m, 1H, -C(=O)OC*H*<), 4.55 (t, 4H, -C(=O)OC*H*₂CH=CH-), 3.16 (s, 2H, pip-C*H*₂C(=O)O-), 2.80-2.38 (m, 20H; 4H, >NC*H*₂CH₂-; 8H, pip-*H*; 2H, pip-C*H*₂CH₂-; 2H, pip-CH₂C*H*₂-; 4H, -CH=CHC*H*₂CH=CH-), 2.18-2.09 (m, 4H, -OC(=O)C*H*₂(CH₂)₆-), 2.06-1.99 (m, 12H, -CH=CHC*H*₂CH₂-), 1.64-1.22 (m, 72H, -CH₂C*H*₂CH₂-, -C*H*₂CH₃), 0.91 (t, 12H, -CH₂C*H*₃). MS (ESI): m/z=1202.1 ([M+H]⁺).

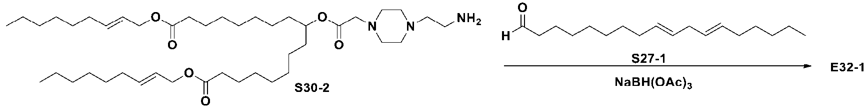

### Example-33: Cationic Lipid (E33-1)

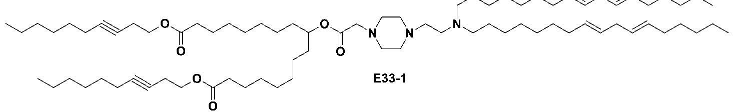

The preparation process is as follows:
Compound **S31-1** (1.52 g, 2.0 mmol) was dissolved in dichloromethane (20 mL) and cooled to 0°C in ice bath conditions. Under vigorous stirring, **S27-1** (1.58 g, 6.0 mmol) was added, followed by the addition of **NaBH(OAc)₃** (1.27 g, 6.0 mmol) in three portions over 10 minutes. The ice bath was then removed, and the reaction solution was stirred at room temperature for 2 h. After completion of the reaction, an aqueous solution of NaOH (1 M, 10 mL) was added, and the solution was stirred for 15 minutes, and then diluted with water. The product was extracted with dichloromethane twice (10 mL × 2), then the combined organic phases were washed with brine (10 mL), dried with anhydrous magnesium sulfate, filtered, and the filtrate was concentrated, and then the crude product was purified by column chromatography to obtain cationic lipid **E33-1** (2.28 g, 93.1%). ¹H NMR (400 MHz, CDCl₃) δ: 5.45-5.25 (m, 8H, *-CH=CH-),* 4.93-4.87 (m, 1H, *-C(=O)OCH<),* 4.12 (t, 4H, -C≡CCH₂C*H*₂OC(=O)-), 3.16 (s, 2H, pip-C*H*₂C(=O)O-), 2.80-2.38 (m, 24H; 4H, >NC*H*₂CH₂-; 8H, pip-*H*; 2H, pip-C*H*₂CH₂-; 2H, pip-CH₂C*H*₂-; 4H, -CH=CHC*H*₂CH=CH-; 4H, -C≡CC*H*₂CH₂OC(=O)-), 2.30 (t, 4H, -(CH₂)₄C*H*₂C≡C-), 2.18-2.09 (m, 4H, -OC(=O)C*H*₂(CH₂)₆-), 2.03-1.97 (m, 8H, -CH=CHC*H*₂-), 1.65-1.23 (m, 72H, -CH₂C*H*₂CH₂-, -C*H*₂CH₃), 0.90 (t, 12H, -CH₂C*H*₃). MS (ESI): m/z=1226.1 ([M+H]⁺).

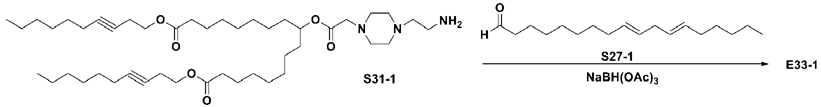

### Example 34: Preparation of LNP-mRNA Pharmaceutical Compositions and Characterization of Physicochemical Properties Thereof

### Example 34.1: Preparation of LNP-mRNA Pharmaceutical Compositions

In the present embodiment, LNP-mRNA pharmaceutical compositions (LNP-mRNAs) containing Fluc-mRNA were prepared; wherein, the phospholipid contained in each group was DSPC, the steroid lipid contained in each group was cholesterol, and the PEGylated lipid contained in each group was PEG2k-DMG; the difference is the cationic lipid.

The preparation method of LNP-mRNA is as follows:
Step a: A certain amount of cationic lipid, DSPC, cholesterol, and PEGylated lipid stock solutions were pipetted and dissolved in ethanol at a molar ratio of 50:10:38:1.5 to obtain an ethanol phase solution (the specific lipid formulations for each group are shown in Table 1, the control groups **L-CT1** and **L-CT2** used cationic lipids from prior art, while the experimental groups **L-1-1** to **L-33** used cationic lipids containing a piperazine ring as described in present application);
Wherein, the cationic lipid used in L-CT1 was C-1, prepared according to the method disclosed in patent literature CN115010681A; the cationic lipid used in L-CT2 was C-2, prepared according to the method disclosed in patent literature US20220040308A1; The structures of C-1 and C-2 are as follows:

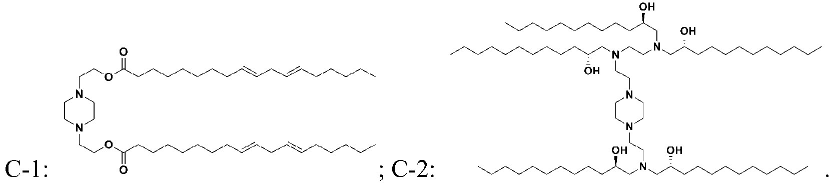

Step b: Fluc-mRNA was added to 10~50 mM citrate buffer (pH=4) to obtain the aqueous solution.

Step c: the ethanol phase solution and the aqueous phase solution were mixed (1:3, v/v) to prepare LNP-mRNA, which was washed by multiple DPBS ultrafiltration to remove ethanol and free molecules. Finally, it was filtered through a 0.2 µm sterile filter and stored for subsequent use.

**Table 1: Summary of Formulations of Lipid Compositions and Particle Sizes and Encapsulation Efficiency of the Prepared LNP-mRNAs**

| **LNP-mRNA** | **L-CT1** | **L-CT2** | **L-1-1** | **L-1-2** | **L-1-3** | **L-1-4** |
|---|---|---|---|---|---|---|
| **Cationic Lipid, CL** | C-1 | C-2 | E1-1 | E1-2 | E1-3 | E1-4 |
| **Encapsulation Efficiency /%** | 90.78 | 91.61 | 91.21 | 92.34 | 93.93 | 93.25 |
| **Particle Size /nm** | 94.88 | 93.05 | 94.27 | 91.41 | 93.06 | 92.04 |

| **LNP-mRNA** | **L-2** | **L-3** | **L-4-1** | **L-4-2** | **L-5** | **L-6** |
|---|---|---|---|---|---|---|
| **Cationic Lipid, CL** | E2-1 | E3-1 | E4-1 | E4-2 | E5-1 | E6-1 |
| **Encapsulation Efficiency /%** | 91.35 | 90.37 | 88.43 | 87.36 | 87.34 | 89.10 |
| **Particle Size /nm** | 92.36 | 91.16 | 92.55 | 94.21 | 95.59 | 94.04 |

| **LNP-mRNA** | **L-7** | **L-8** | **L-9** | **L-10** | **L-11** | **L-12** |
|---|---|---|---|---|---|---|
| **Cationic Lipid, CL** | E7-1 | E8-1 | E9-1 | E10-1 | E11-1 | E12-1 |
| **Encapsulation Efficiency /%** | 88.12 | 92.34 | 91.88 | 91.90 | 90.23 | 89.12 |
| **Particle Size /nm** | 92.23 | 93.38 | 92.75 | 95.10 | 92.90 | 92.37 |

| **LNP-mRNA** | **L-13** | **L-14** | **L-15** | **L-16** | **L-17** | **L-18** |
|---|---|---|---|---|---|---|
| **Cationic Lipid, CL** | E13-1 | E14-1 | E15-1 | E16-1 | E17-1 | E18-1 |
| **Encapsulation Efficiency /%** | 88.72 | 88.34 | 90.87 | 91.29 | 90.95 | 89.76 |
| **Particle Size /nm** | 91.35 | 90.12 | 89.26 | 92.34 | 94.52 | 93.89 |

| **LNP-mRNA** | **L-19** | **L-20** | **L-21** | **L-22** | **L-23** | **L-24** |
|---|---|---|---|---|---|---|
| **Cationic Lipid, CL** | E19-1 | E20-1 | E21-1 | E22-1 | E23-1 | E24-1 |
| **Encapsulation Efficiency /%** | 91.34 | 92.15 | 92.16 | 91.32 | 91.35 | 91.03 |
| **Particle Size /nm** | 93.42 | 92.81 | 92.13 | 93.41 | 89.28 | 91.11 |

| **LNP-mRNA** | **L-25** | **L-26** | **L-27** | **L-28** | **L-29** | **L-30** |
|---|---|---|---|---|---|---|
| **Cationic Lipid, CL** | E25-1 | E26-1 | E27-1 | E28-1 | E29-1 | E30-1 |
| **Encapsulation Efficiency /%** | 91.38 | 90.87 | 93.11 | 92.23 | 92.17 | 93.26 |
| **Particle Size /nm** | 89.41 | 92.35 | 92.01 | 91.02 | 91.95 | 94.51 |

| **LNP-mRNA** | **L-31** | **L-32** | **L-33** | | | |
|---|---|---|---|---|---|---|
| **Cationic Lipid, CL** | E31-1 | E32-1 | E33-1 | | | |
| **Encapsulation Efficiency /%** | 90.94 | 91.65 | 91.47 | | | |
| **Particle Size /nm** | 92.14 | 92.34 | 96.06 | | | |

### Example 34.2: Characterization of Physicochemical Properties of LNP-mRNA Pharmaceutical Compositions

Determination of encapsulation rate: The encapsulation efficiency of LNP-mRNA compositions was determined using the Quant-it Ribogreen RNA Quantification Kit. The results showed that the lipid compositions of the present application (L-1~L-33) exhibited high nucleic acid encapsulation efficiencies for nucleic acid drugs, all in the range of 80%-95%, and most in the range of 90%-95%. The results showed that mRNA could be effectively encapsulated in the experimental groups with azacycle-containing cationic lipids, wherein the encapsulation efficiencies were comparable to or better than those of C-1 and C-2; there were also differences in the encapsulation efficiency among the azacycle-containing cationic lipids with different structures.

Determination of particle size: In the present embodiment, the particle size of LNP-mRNA was determined by dynamic light scattering (DLS). The measured LNP-mRNAs exhibited high dimensional uniformity, with PDI values less than 0.3. The particle sizes of the LNP-mRNAs prepared from the lipid compositions of the present application were in the range of 80-120 nm, which meets the requirements of particle size for gene vectors.

### Example 35: Biological Activity Tests of LNP-mRNA Pharmaceutical Compositions

### (1) Serum Stability Evaluation

LNP-mRNA pharmaceutical compositions were added to the medium containing 10% fetal bovine serum (FBS) with agitation at 37°C. Samples was taken at regular intervals to determine the particle size change of LNP-mRNA, and the serum stability of the LNP-mRNA pharmaceutical composition formulation was analyzed by testing its particle size change. The experimental results showed that within 7 days, the particle size change of experimental groups were 0~14%, and the particle size change of experimental groups were 2~8%, indicating that the formulation of LNP-mRNA pharmaceutical composition prepared from the cationic lipids of the present application exhibited good serum stability.

### (2) Assays for Cytotoxicity (Biocompatibility)

DMEM high glucose complete culture medium containing 10% FBS was prepared. The LNP-mRNA Pharmaceutical Compositions of samples groups (L-1 to L-33 and L-CT1 to L-CT2) were respectively formulated into working solutions of 0.1, 0.15, 0.2, 0.25, 0.3 µg/100 µL using the complete culture medium, and were stored for subsequent use. 293T cells in the logarithmic growth phase was inoculated onto a 96-well plate at a density of 7×10³ cells/well, with 100 µL/well. Both the control and experimental groups were set with six replicate wells. After incubation in a constant temperature incubator with 5% CO₂ at 37°C for 24 h, the original culture medium was retained, the control group was supplemented with 100 µL/well of complete culture medium, and the sample group was supplemented with 100 µL/well of the working solution. After further incubation for 24 h, 100 µL/well of culture medium containing 10% CCK-8 was added, and the solution was cultured in a constant temperature incubator with 5% CO₂ at 37°C for 2 h. The absorbance at 450 nm was measured using a microplate reader. The relative cell viability was calculated using the following formula: 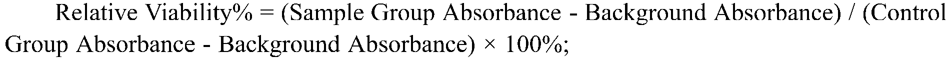 Relative Viability% = (Sample Group Absorbance - Background Absorbance) / (Control Group Absorbance - Background Absorbance) × 100%; wherein, the background absorbance
refers to the absorbance of the sample containing only CCK-8 reagent and culture medium.

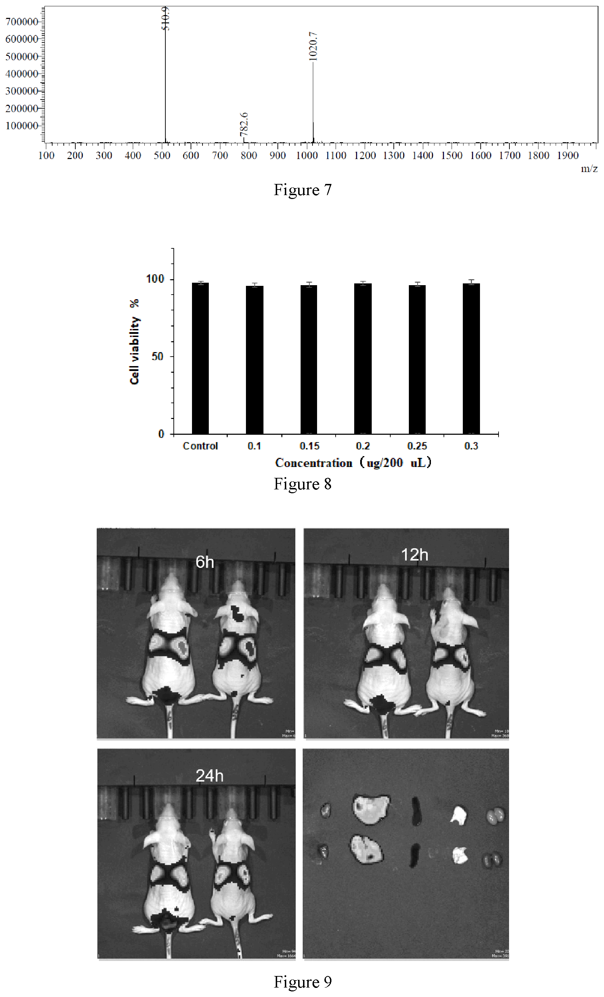

The experimental results showed that the LNP-mRNA pharmaceutical composition prepared with the cationic lipid of the present application has no obvious cytotoxicity at five concentration gradients, with cell viability above 95%. Specifically, for example, the results of the experimental group L-1-3 are shown in Figure 8.

### (3) Evaluation of In Vitro Transfection Efficiency

In order to investigate the mRNA transfection efficiency at the cellular level of each group of LNP-mRNA pharmaceutical compositions prepared in **Example 34** of the present application, luciferase bioluminescence was used for testing. The LNP-mRNA pharmaceutical composition was dissolved in medium to prepare the required dose. HeLa cells were used as the cell model, and cell suspensions of 100 µL/well were inoculated in a black-edged, clear-bottomed 96-well plate at a density of 6000 cells/well. After inoculation was completed, the cells were cultured in a cell culture incubator for 24 h, and then the cells were dosed with 0.2 µg mRNA per well, while the blank control group was added with the corresponding dose of free Fluc-mRNA. After 24 h of transfection, the old medium was replaced with fresh medium containing D-fluorescein sodium substrate (1.5 mg/mL). After 5 min of incubation, bioluminescence was detected using a microplate reader. The stronger the fluorescence, the more Fluc-mRNA is transported into the cytoplasm and translated the corresponding fluorescent protein. The results are shown in Table 2; wherein, the relative value of fluorescence intensity is the ratio of the fluorescence intensity of each group to the fluorescence intensity of the control group. The results show that the LNP-mRNA pharmaceutical compositions prepared in the present application exhibited excellent in vitro transfection effects, indicating that the LNPs in the experimental groups are effective nucleic acid delivery carriers. This is probably attributed to the fact that the cationic lipids of the present application contain three ionizable tertiary amine structures, which endow the LNPs with superior transfection efficiency compared to the L-CT1 and L-CT2 groups prepared from the azacycle-containing cationic lipids of the prior art; comparing the experimental groups L-1-1, L-1-2, L-1-3, and L-1-4, the carbon chain length between the tertiary amine and the ester bond of the linker affects the ionizability of the tertiary amine. Within a certain range, the longer the carbon chain, the higher the transfection efficiency. This may be because stronger ionizability allows for greater binding with nucleic acids, thereby increasing transfection efficiency. For example, in L-1-1 and L-1-2, the non-piperazine ring tertiary amines of the cationic lipids E1-1 and E1-2 have a carbon chain length of C2 between the tertiary amine and the ester bond, while in L-1-3 and L-1-4, the non-piperazine ring tertiary amines of the cationic lipids E1-3 and E1-4 have a carbon chain length of C5. As a result, L-1-3 and L-1-4 exhibited superior encapsulation and transfection efficiencies; furthermore, although groups L-27, L-28, L-29, L-32, and L-33 demonstrated high encapsulation efficiencies, their transfection efficiencies were not optimal. This may be because the cationic lipids E27-1, E28-1, E29-1, E32-1, and E33-1 contain fewer degradable groups, which hinder the endosomal escape of mRNA and result in lower transfection efficiencies compared to L-1-3. Comparing L-6 with L-8, the cationic lipid E8-1, which contains a hydroxyl group, exhibited superior encapsulation and transfection efficiencies to E6-1, which lacks a hydroxyl group. This may be because the hydroxyl group can interact with the phosphate groups of nucleic acids through hydrogen bonding, thereby enhancing delivery efficiency.

**Table 2: Results of Cell Transfection Tests**

| **Entry** | **Relative Fluorescence Value** | **Entry** | **Relative Fluorescence Value** | **Entry** | **Relative Fluorescence Value** |
|---|---|---|---|---|---|
| **Blank** | 1 | **L-8** | 10.5 | **L-22** | 10.4 |
| **L-CT1** | 6.5 | **L-9** | 10.7 | **L-23** | 10.7 |
| **L-CT2** | 5.3 | **L-10** | 10.3 | **L-24** | 10.5 |
| **L-1-1** | 10.1 | **L-11** | 10.2 | **L-25** | 10.2 |
| **L-1-2** | 10.3 | **L-12** | 9.1 | **L-26** | 9.9 |
| **L-1-3** | 12.6 | **L-13** | 8.7 | **L-27** | 9.5 |
| **L-1-4** | 12.3 | **L-14** | 9.0 | **L-28** | 9.7 |
| **L-2** | 9.7 | **L-15** | 10.8 | **L-29** | 9.6 |
| **L-3** | 9.8 | **L-16** | 10.6 | **L-30** | 9.8 |
| **L-4-1** | 9.5 | **L-17** | 10.8 | **L-31** | 10.3 |
| **L-4-2** | 8.8 | **L-18** | 9.2 | **L-32** | 9.5 |
| **L-5** | 9.1 | **L-19** | 12.0 | **L-33** | 9.3 |
| **L-6** | 9.5 | **L-20** | 11.9 | | |
| **L-7** | 8.7 | **L-21** | 11.8 | | |

### (4) Evaluation of In Vivo Transfection Efficiency

The lipid nanoparticle **L-1-3** was delivered to 6-8-week-old female BALB/c mice at a dose of 10 µg per mouse by tail vein injection, and in vivo fluorescence imaging of the mice was performed at 6, 12, and 24 h post-administration. After the last imaging was completed, the mice were euthanized, and imaging was conducted on the major organs including heart, liver, spleen, lungs, and kidneys (from left to right in the figure). 10 to 15 minutes prior to imaging, 0.2 mL of D-luciferin sodium salt (15 mg/mL) was injected intraperitoneally. The experimental results (Figure 9) indicate that the lipid-nucleic acid pharmaceutical composition prepared from the cationic lipid of the present application can achieve efficient in vivo delivery of nucleic acid drugs, and the LNP-mRNA pharmaceutical composition delivered into the body was primarily distributed in the liver and spleen.

Those described above are only embodiments of the present invention, not limiting the patent scope of the present invention. Any transformation of equivalent structure or equivalent route based on the specification content of the present invention, directly or indirectly applied in other related arts, should be included in the protected patent scope of the present invention in the same way.

For those skilled in the art, without departing from the purpose and the scope of the present invention, and without unnecessary experimentation, the present invention can be implemented in a wider range with equivalent parameters, concentrations, and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A cationic lipid, wherein the structure is represented by the general formula (1): 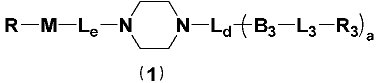
or a salt, tautomer, stereoisomer, deuterated derivative, or solvate thereof;
wherein, L_{d} is -(CH₂)ₜₐN<, and the left end is connected to the piperazine ring; ta is an integer from 1 to 6; a is 2;
Lₑ is a C₁₋₁₂ alkylene group or -CH₂CH(OH)-(CH₂)_{tb}-; wherein, tb is an integer from 1 to 10, and the left of -CH₂CH(OH)-(CH₂)_{tb}- is connected to the piperazine ring;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 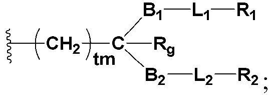
wherein, tm is an integer from 0 to 6; B₁, B₂, and B₃ are each independently a linking bond or a C₁₋₂₀ alkylene group at each occurrence; L₁, L₂, and L₃ are each independently a linking bond or L_{c}; the L_{c} is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O- at each occurrence; R₁, R₂, and R₃ are each independently a linear or branched C₅₋₃₀ hydrocarbon group at each occurrence, wherein the hydrocarbyl group is an alkyl group, an alkenyl group, or an alkynyl group; when B₁, B₂, L₁, and L₂ are all linking bonds, Lₑ is -CH₂CH(OH)-(CH₂)_{tb}-; s is 1, 2 or 3; R_{g} is H or C₁₋₆ alkyl.

2. The cationic lipid according to claim **1,** wherein the B₁, B₂, and B₃ are selected from any of the following cases:
Case (1): B₁, B₂, and B₃ are each independently a C₁₋₂₀ alkylene group;
Case (2): both B₁ and B₂ are linking bonds, and B₃ is independently a C₁₋₂₀ alkylene group at each occurrence;
Case (3): B₁ and B₂ are each independently a C₁₋₂₀ alkylene, and B₃ is a linking bond;
Case (4): B₁, B₂, and B₃ are all linking bonds;
the aforementioned C₁₋₂₀ alkylene group is preferably a C₁₋₁₀ alkylene group, more preferably selected from the group consisting of a methylene group, an ethylene group, a propylene group, a butylene group, a pentylene group, a hexylene group, and a heptylene group.

3. The cationic lipid according to claim **1,** wherein the L₁, L₂, and L₃ are selected from any of the following cases:
Case (1): L₁, L₂, and L₃ are each independently L_{c};
Case (2): one of L₁, L₂, and L₃ is a linking bond, and the other two are each independently L_{c}; preferably, L₃ is a linking bond, and L₁ and L₂ are each independently L_{c};
Case (3): two of L₁, L₂, and L₃ are linking bonds, and the other is L_{c}; preferably, L₁ and L₂ are linking bonds, and L₃ is L_{c};
Case (4): L₁, L₂, and L₃ are all linking bonds;
more preferably, L₁, L₂, and L₃ are selected from any of the following cases:
Case (a): L₁, L₂, and L₃ are each independently selected from the group consisting of -OC(=O)-, -C(=O)O-, -C(=O)NH-, -NHC(=O)-, -OC(=O)O-, -NHC(=O)O-, and -OC(=O)NH-;
Case (b): L₃ is a linking bond, L₁ and L₂ are each independently selected from the group consisting of -OC(=O)-, -C(=O)O-, -C(=O)NH-, -NHC(=O)-, -OC(=O)O-, -NHC(=O)O-, and -OC(=O)NH-;
Case (c): L₁ and L₂ are linking bonds, and L₃ is selected from the group consisting of -OC(=O)-, -C(=O)O-, -C(=O)NH-, -NHC(=O)-, -OC(=O)O-, -NHC(=O)O-, and -OC(=O)NH-.

4. The cationic lipid according to claim **1,** wherein the linear C₅₋₃₀ hydrocarbon group is selected from the group consisting of a linear C₅₋₃₀ alkyl group, a linear C₅₋₃₀ alkenyl group, and a linear C₅₋₃₀ alkynyl group, preferably each independently a linear C₅₋₂₅ alkyl group, a linear C₅₋₂₅ alkenyl group, or a linear C₅₋₂₅ alkynyl group; more preferably, the linear C₅₋₃₀ hydrocarbon group is selected from the group consisting of the following structures: 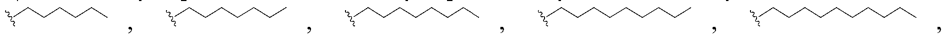 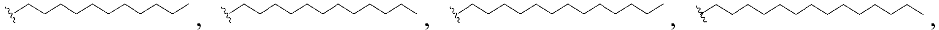 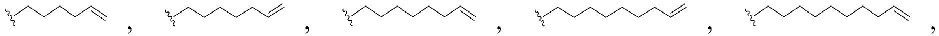 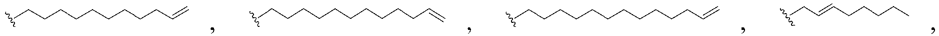 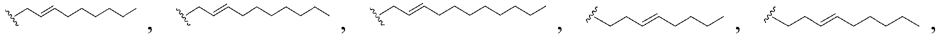 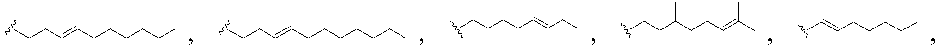 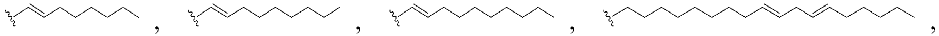 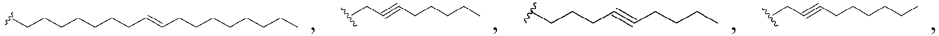 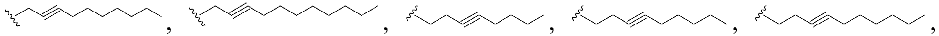 and 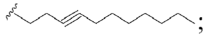 the branched C₅₋₃₀ hydrocarbon group is selected from a branched C₅₋₃₀ alkyl group, a branched C₅₋₃₀ alkenyl group, and a branched C₅₋₃₀ alkynyl group, each independently represented by 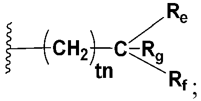 wherein, tn is an integer from 0 to 12; Rₑ and R_{f} are each independently selected from the group consisting of a C₁₋₁₅ alkyl group, a C₂₋₁₅ alkenyl group, and a C₂₋₁₅ alkynyl group; preferably, Rₑ and R_{f} are each independently selected from the group consisting of a butyl group, a pentyl group, a hexyl group, a heptyl group, a octyl group, a nonyl group, a decyl group, a undecyl group, a dodecyl group, a pentenyl group, a hexenyl group, a heptenyl group, a octenyl group, a nonenyl group, and a decenyl group; the branched C₅₋₃₀ hydrocarbon group is most preferably selected from the group consisting of the following structures:

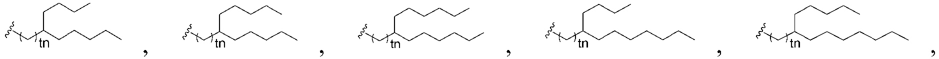

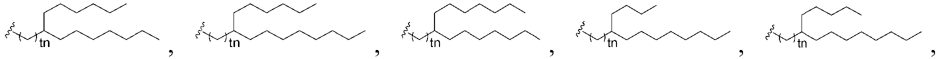

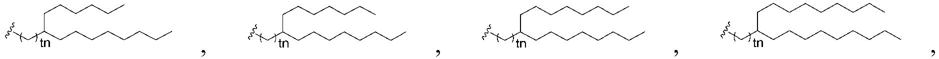

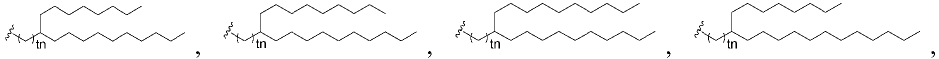

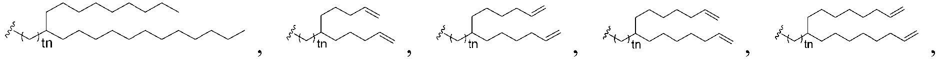

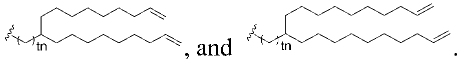

5. The cationic lipid according to claim **1,** wherein the R₁, R₂, and R₃ are selected from any of the following cases:
Case (1): the R₁, R₂, and R₃ are all linear C₅₋₃₀ hydrocarbon groups;
Case (2): the R₁, R₂, and R₃ are all branched C₅₋₃₀ hydrocarbon groups;
Case (3): any one of the R₁, R₂, and R₃ is a linear C₅₋₃₀ hydrocarbon group, and the other two are branched C₅₋₃₀ hydrocarbon groups;
Case (4): any two of the R₁, R₂, and R₃ are linear C₅₋₃₀ hydrocarbon groups, and the other is a branched C₅₋₃₀ hydrocarbon group;
preferably, R₁, R₂, and R₃ are all linear C₅₋₃₀ hydrocarbon groups.

6. The cationic lipid according to claim **1,** wherein the R is selected from the group consisting of the following structures: 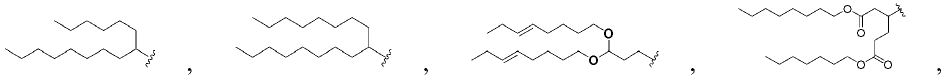 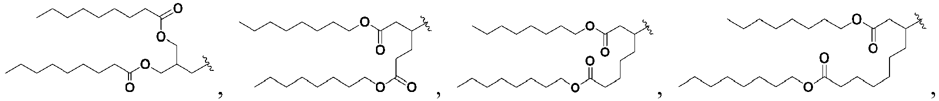 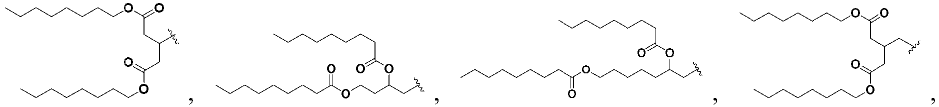 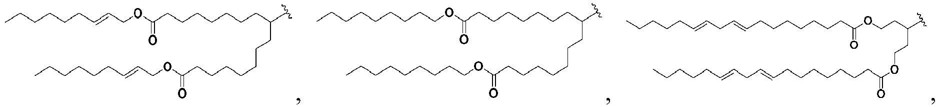 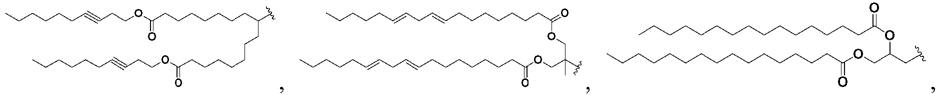 and

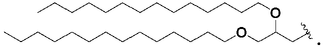

7. The cationic lipid according to claim **1,** wherein the fragment -B₃-L₃-R₃ is independently selected from the group consisting of the following structures: 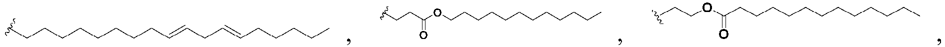 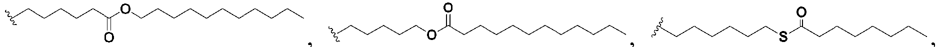 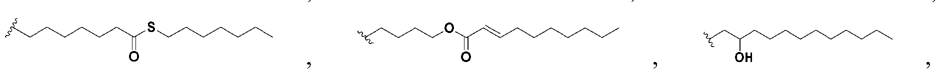 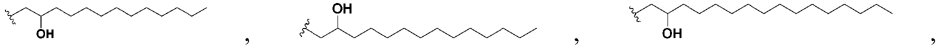 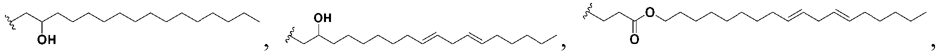 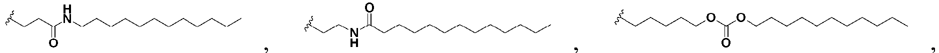 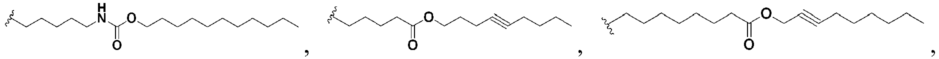 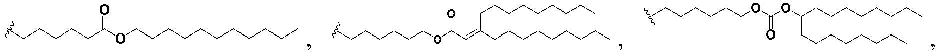 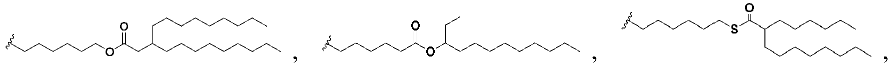 and

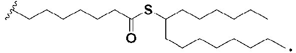

8. The cationic lipid according to claim **1,** wherein the cationic lipid satisfies any one of the following general formulas: 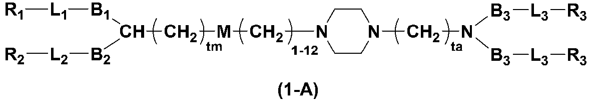 and 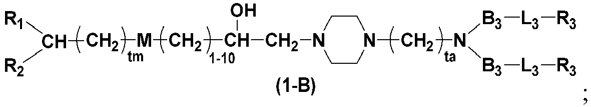 wherein, L₁ and L₂ in formula (1-A) are divalent linking bonds, and B₁, B₂, L₁, and L₂ in formula (1-B) are all linking bonds; wherein, M is -OC(=O)- or -C(=O)O-;
the cationic lipid more preferably satisfies any one of the following general formulas: 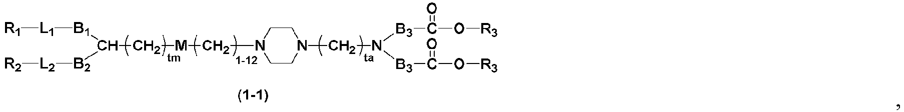 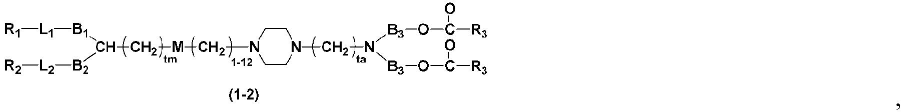 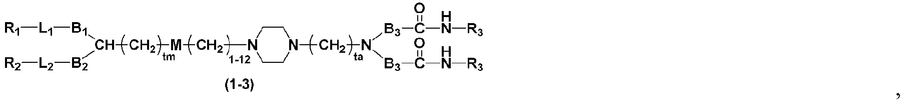 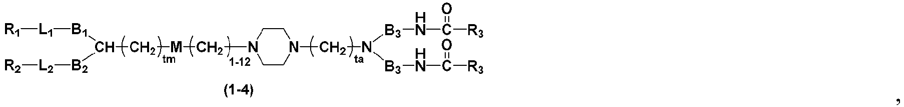 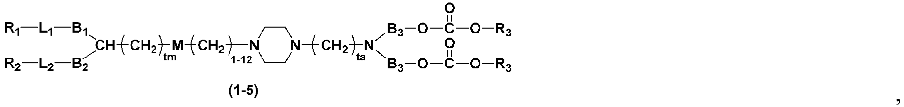 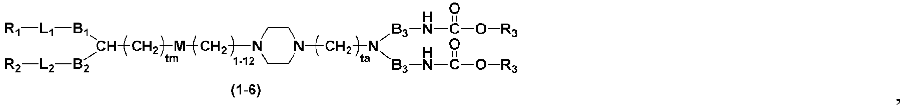 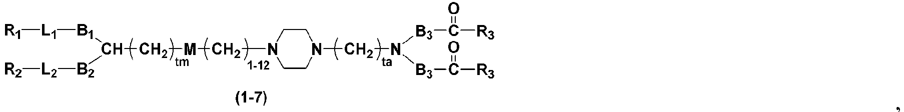 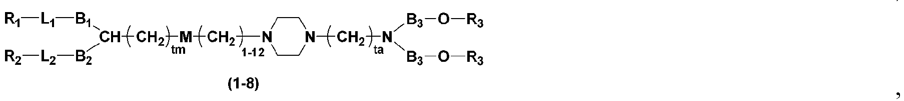 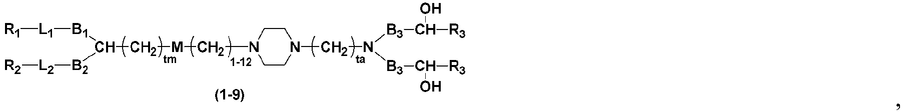 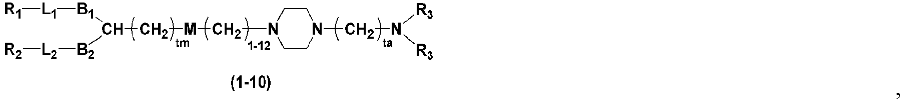 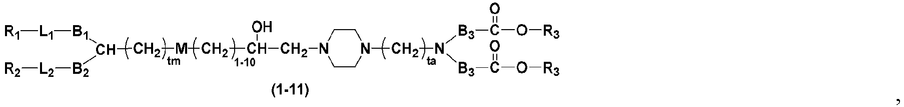 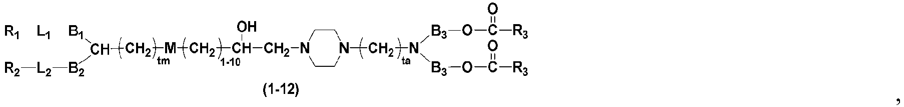 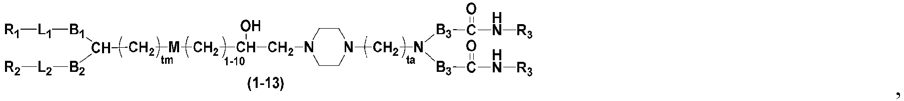 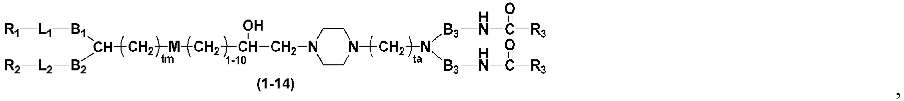 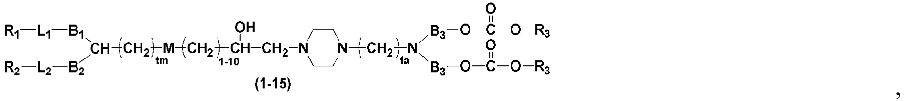 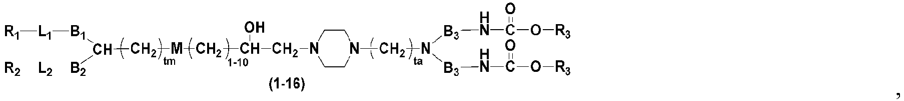 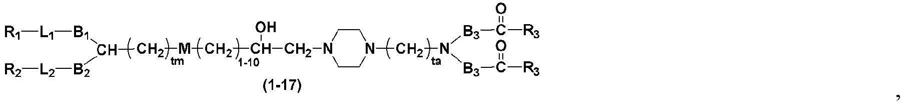 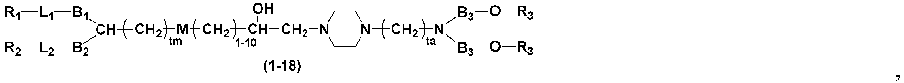 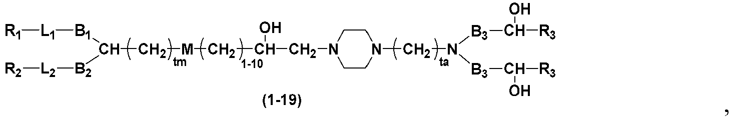 and 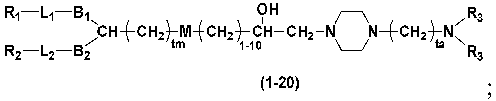
preferably, in the aforementioned formulas (1-1) to (1-20), B₁ and B₂ are the same and each a linking bond or a C₁₋₁₂ alkylene group; the L₁ and L₂ are the same and each selected from the group consisting of a linking bond, -O-, -OC(=O)-, -C(=O)O-, -NHC(=O)-, -C(=O)NH-, and -OC(=O)O-; R₁ and R₂ are the same and each a linear C₅₋₃₀ hydrocarbon group; R₃ is a linear C₅₋₃₀ hydrocarbon group.

9. The cationic lipid according to claim **1,** wherein the cationic lipid is selected from the group consisting of the following structures: 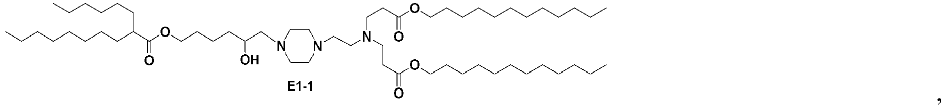 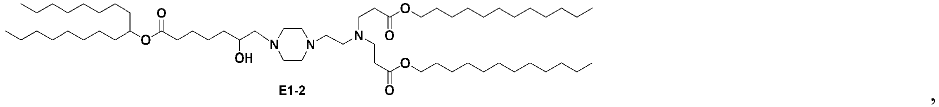 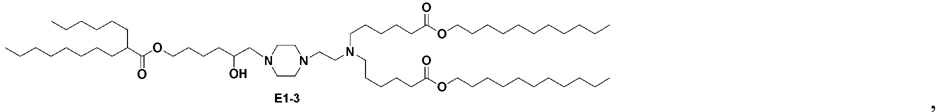 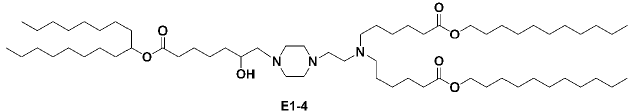 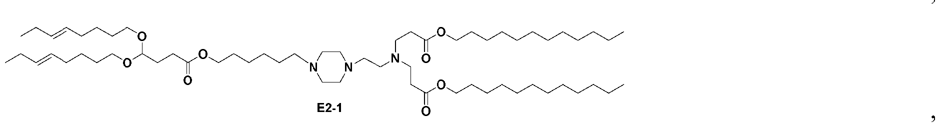 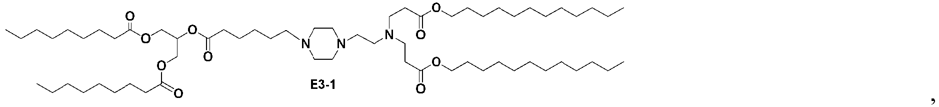 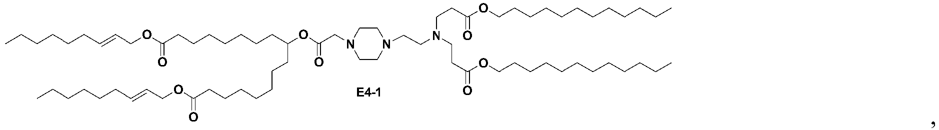 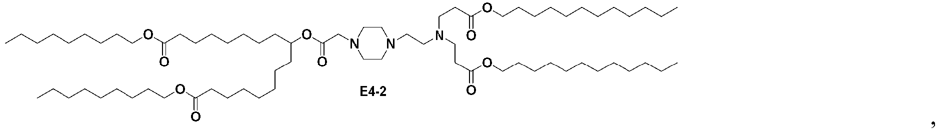 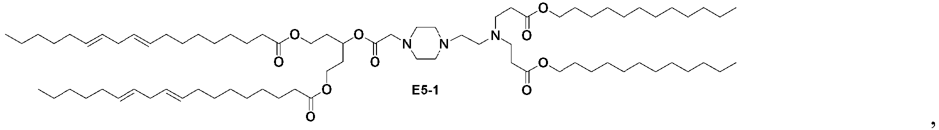 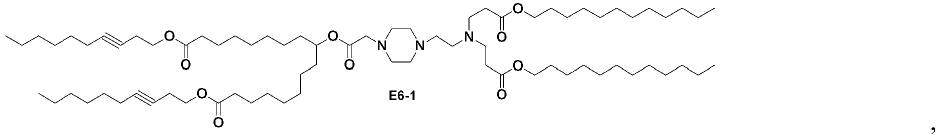 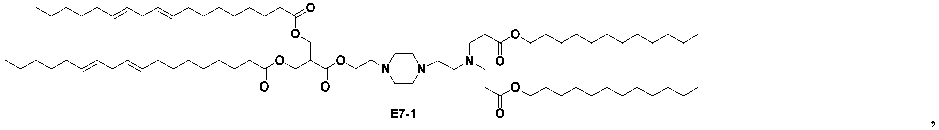 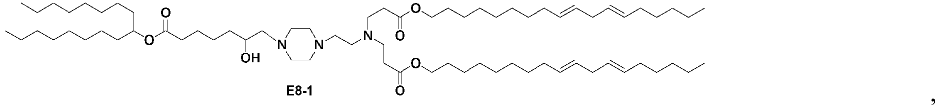 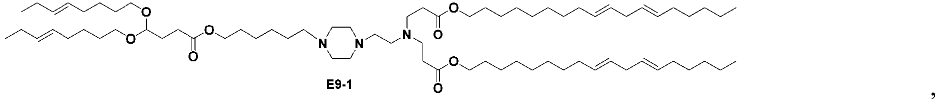 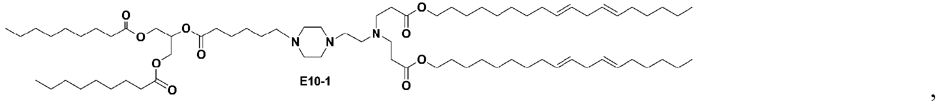 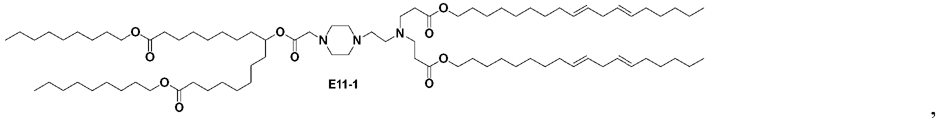 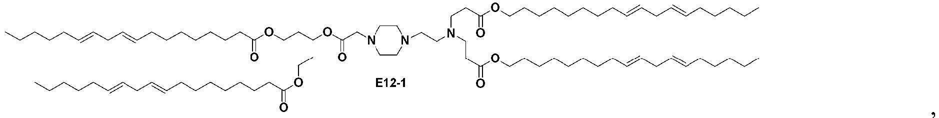 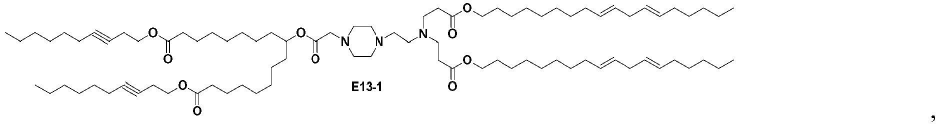 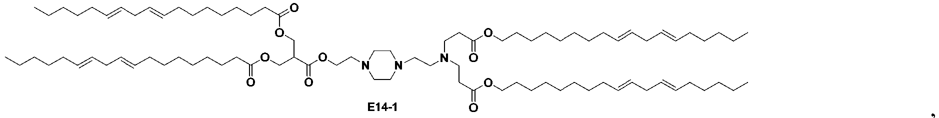 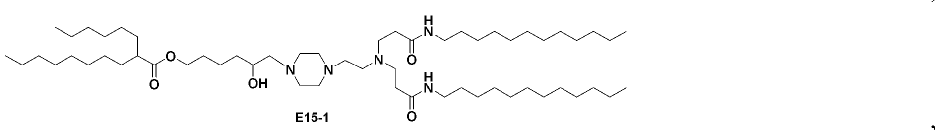 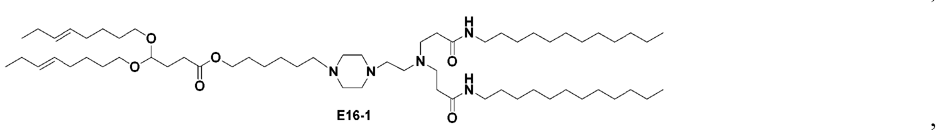 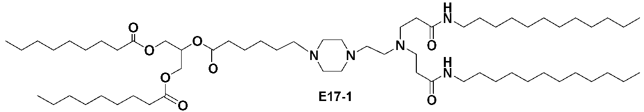 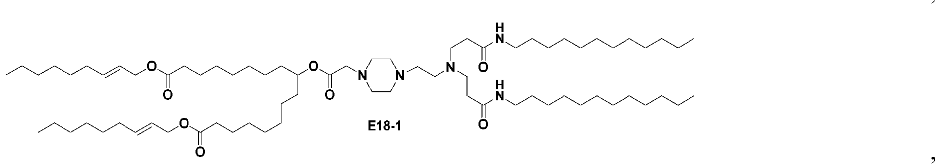 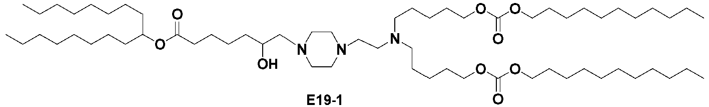 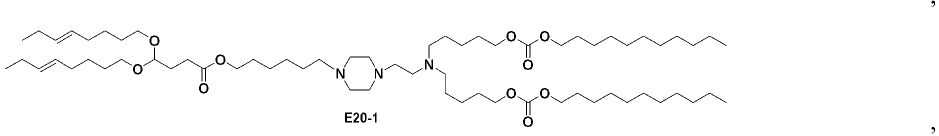 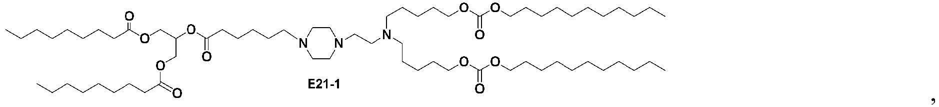 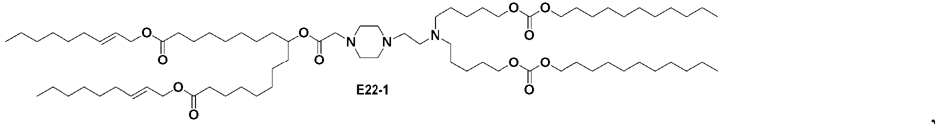 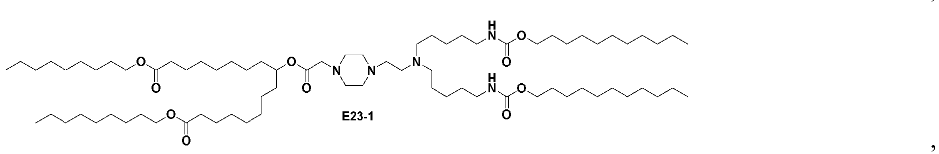 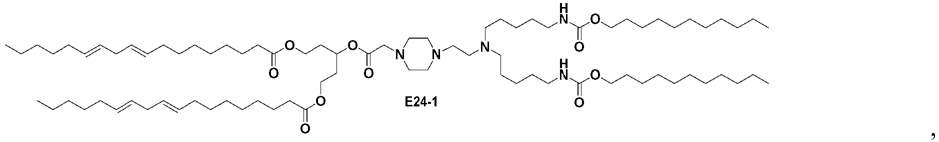 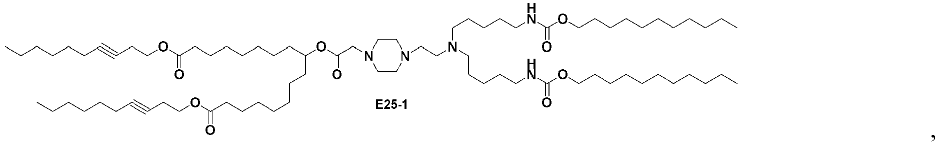 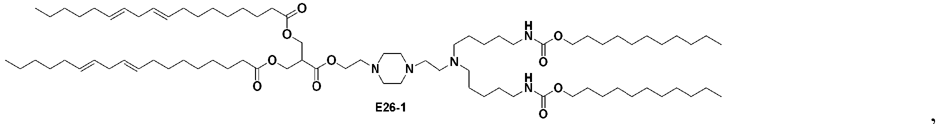 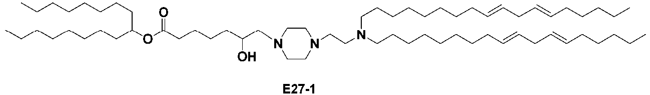 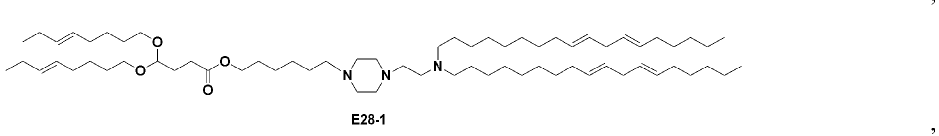 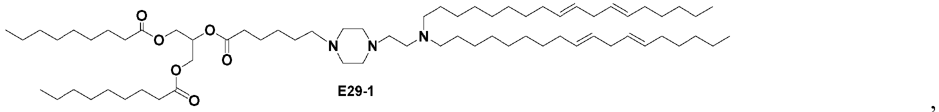 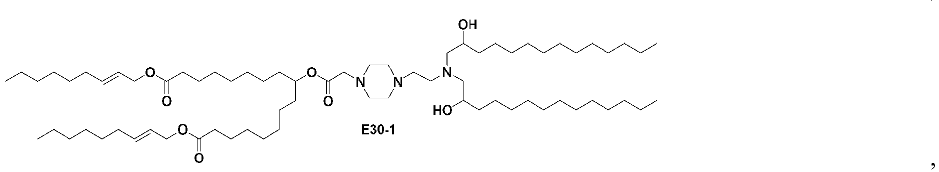 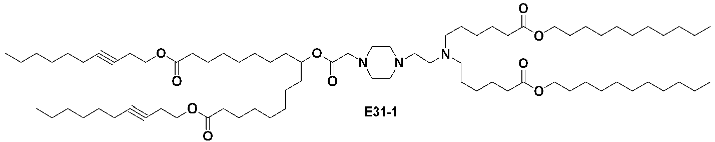 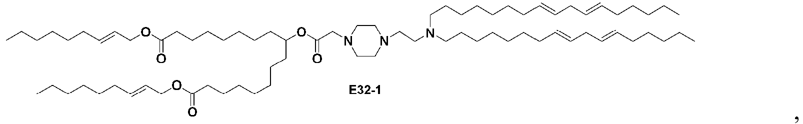 and

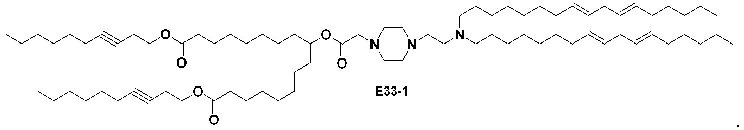

10. A lipid composition comprising a cationic lipid of any one of claims **1** to **9.**

11. The lipid composition according to claim **10,** wherein the lipid composition comprises one or more types of lipids selected from the group consisting of phospholipid, steroid lipid, and PEGylated lipid; the lipid composition is selected from any of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a phospholipid lipid, a steroid lipid, and a PEGylated lipid.

12. The lipid composition according to claim **11,** wherein the phospholipid in the lipid composition is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-O-octadecenyl-sn-glycero-3-phosphatidylcholine, 1-oleoyl-2-cholesterylhemisuccinyl-sn-glycero-3-phosphocholine, 1-O-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
or the steroid lipid in the lipid composition is selected from the group consisting of cholesterol, fecal sterol, sitosterol, ergosterol, campesterol, stigmasterol, rapeseed sterol, lycopene, ursolic acid, α-tocopherol, and combinations thereof;
or the PEGylated lipid in the lipid composition is selected from the group consisting of 1,2-dimyristoyl-sn-glycerol methoxypolyethylene glycol, 1,2-distearoyl-sn-glycero-3-phosphoethanolamine-N-[amino(polyethylene glycol)], PEG-cholesterol, PEG-diacylglycamide, and PEG-dialkyloxypropyl, specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol, and combinations thereof;
or the PEGylated lipid in the lipid composition is selected from the group consisting of the following structures and combinations thereof: 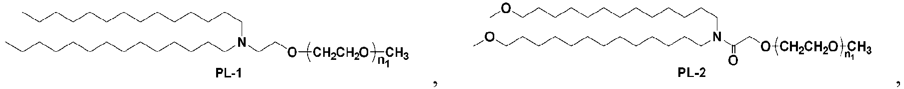 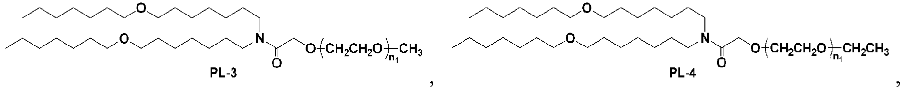 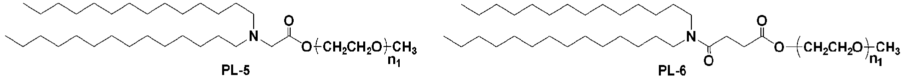 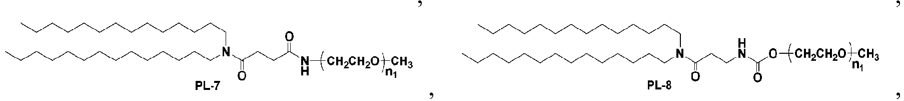 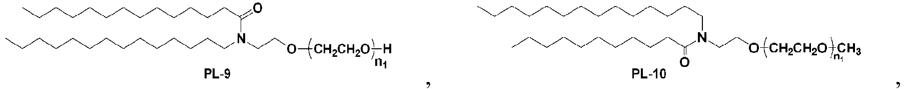 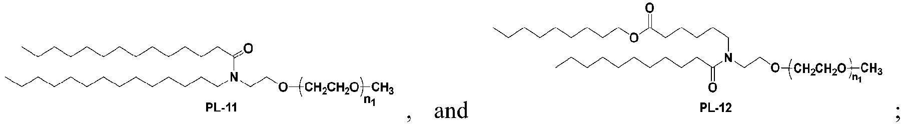 wherein, n₁ is an integer of 25 to 300, and is more preferably selected from the group consisting of 44, 45, 46, 47, and 48;
or the other cationic lipid in the lipid composition is selected from the group consisting of 1,2-dioleoyl-3-trimethylammonium-propane, 1,2-bisoctadecoxy-3-methylammonium propane chloride, 1-[2-(oleoyloxy)ethyl]-2-oleo-3-(2-hydroxyethyl)imidazoline chloride, 1,2-diolecanol-3-dimethylamino-propane, 2,3-bis(tetradecanoyloxy)propyltrimonium chloride, didecyldimonium chloride, bisdecyldimonium bromide, *N,N*-diole*-N,N*-dimethylammonium chloride, *N*-(4-carboxybenzyl)-*N*,*N*-dimethyl-2,3-bis(oleoyloxy)propyl-1-ammonium, 3,6-bis{4-[bis(2-hydroxydodecyl)amino]butyl}piperazine-2,5-dione, 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol), 4-(*N*,*N*-dimethylamino)butyric acid(dilinole)methyl ester, 1,2-dimyristoyl-sn-glycero-3-ethylphosphocholine, ((4-hydroxybutyl) azadiyl)bis(hexane-6,1-diyl)bis(2-hexyldecanoate), 8-[(2-hydroxyethyl)(6-oxo-6-(undecoxy)hexyl)amino]caprylic acid (hepta-9-yl) ester, ((2-(2-hydroxyethoxy)ethyl)azadialkyl)bis(hexane-6,1-diyl)bis(2-hexyldecanoate), and combinations thereof;
or the anionic lipid in the lipid composition is selected from the group consisting of 1,2-dioleoyl-sn-glycerol-3-phosphate sodium salt, 1,2-dimyristoyl-sn-glycerol-3-phosphate sodium salt, bis(monoolein) phosphatidylmonomethylethanolamine, cardiolipin, and combinations thereof.

13. The lipid composition according to any one of claims **11** to **12,** wherein the lipid composition comprises 20-80% cationic lipids, 5-16% phospholipids, 25-55% steroid lipids and 0.5-10% PEGylated lipids; the percentages are molar percentages of each lipid with respect to the total lipids in the solvent-comprising solution.

14. The lipid composition according to claim **13,** wherein the molar percentage of cationic lipid in the total lipids in the solvent-comprising solution is 30-65%, more preferably 35%, 40%, 45%, 46%, 47%, 48%, 49%, 50%, or 55%;
or the molar percentage of phospholipid in the total lipids in the solvent-comprising solution is 7.5-16%, more preferably 8%, 9%, 10%, 11%, 12%, or 16%;
or the molar percentage of steroid lipid in the total lipids in the solvent-comprising solution is 35-50%, more preferably 40%, 41%, 42%, 43%, 44%, 45%, 46%, 47%, 48%, 49%, or 50%;
or the molar percentage of PEGylated lipid in the total lipids in the solvent-comprising solution is 0.5-5%, preferably 1-3%, more preferably 1.5%, 1.6%, 1.7%, 1.8%, 1.9%, 2.0%, or 2.5%.

15. A lipid pharmaceutical composition comprising a lipid composition of any one of claims **10** to **14** and a drug, wherein the drug is selected from the group consisting of a nucleic acid drug, a gene vaccine, an antitumor drug, a small molecule drug, a peptide drug, and a protein drug.

16. The lipid pharmaceutical composition according to claim **15,** wherein the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

17. A lipid pharmaceutical composition according to any one of claims **15** to **16,** wherein the lipid pharmaceutical composition is used as a drug, selected from any of the following drugs: an antineoplastic agent, an antiviral agent, an antifungal agent, and a vaccine.

18. A formulation of lipid pharmaceutical composition comprising any lipid pharmaceutical composition of any one of claims **15** to **17** and a pharmaceutically acceptable diluent or excipient; the diluent or excipient is preferably deionized water, ultrapure water, phosphate buffer or normal saline, more preferably phosphate buffer or normal saline, and most preferably normal saline.

19. A liposome or lipid nanoparticle comprising a lipid composition of any one of claims **10** to **14.**

20. The liposome or lipid nanoparticle according to claim **19,** wherein the lipid nanoparticle is an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.
